# EUROPÄISCHE PATENTSCHRIFT

(11) **EP 1 761 516 B1**
(45) Veröffentlichungstag und Bekanntmachung des Hinweises auf die Patenterteilung: **28.11.2007**
(21) Anmeldenummer: 05755060.0
(22) Anmeldetag: 18.06.2005
(51) Int. Cl.: C07D 401/04, C07D 213/61, C07D 417/04, C07D 213/84, C07D 213/70, C07D 213/86, C07D 213/78, A01N 43/40

(54) **2, 4, 5, 6-SUBSTITUIERTE PYRIDINE, VERFAHREN ZU IHRER HERSTELLUNG UND IHRE VERWENDUNG ZUR BEKÄMPFUNG VON SCHADPILZEN**
2, 4, 5, 6-SUBSTITUTED PYRIMIDINES, METHOD FOR THE PRODUCTION AND USE THEREOF FOR COMBATING PARASITIC FUNGI
PYRIDINES SUBSTITUEES EN POSITION 2, 4, 5, 6, PROCEDES DE PRODUCTION DESDITES PYRIDINES ET LEUR UTILISATION POUR LUTTER CONTRE LES CHAMPIGNONS PARASITES

(30) Priorität: 25.06.2004 DE 102004030927
(43) Veröffentlichungstag der Anmeldung: 14.03.2007
(73) Patentinhaber: BASF Aktiengesellschaft, 67056 Ludwigshafen (DE)
(72) Erfinder: RHEINHEIMER, Joachim, 67063 Ludwigshafen (DE); SCHIEWECK, Frank, 67258 Hessheim (DE); GROTE, Thomas, 67157 Wachenheim (DE); BLETTNER, Carsten, 68165 Mannheim (DE); SCHWÖGLER, Anja, 68165 Mannheim (DE); GEWEHR, Markus, 56288 Kastellaun (DE); GRAMMENOS, Wassilios, 67071 Ludwigshafen (DE); HÜNGER, Udo, 55124 Mainz (DE); MÜLLER, Bernd, 67227 Frankenthal (DE); SCHÄFER, Peter, 67308 Ottersheim (DE); SPEAKMAN, John-Bryan, 67273 Bobenheim (DE); SCHERER, Maria, 76829 Landau-Godramstein (DE); STRATHMANN, Siegfried, 67117 Limburgerhof (DE); SCHÖFL, Ulrich, 68782 Brühl (DE); STIERL, Reinhard, 67251 Freinsheim (DE)
(86) Internationale Anmeldenummer: PCT/EP2005/006598
(87) Internationale Veröffentlichungsnummer: WO 2006/000358

(56) Entgegenhaltungen:
- EP-A- 0 407 899
- EP-A- 0 588 146

## Beschreibung

Die vorliegende Erfindung betrifft 2-substituierte Pyridine der Formel I 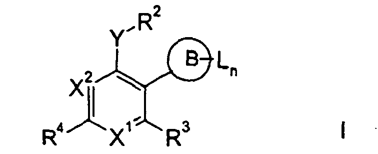 in der die Indices und die Substituenten die folgende Bedeutung haben:
- X¹, X²: jeweils einer der beiden Ringglieder bedeutet N, der andere C-H oder C-Halogen;
- Y: bedeutet eine Gruppe -CH-R¹-, -N-R¹-, -O- oder -S-;
- R¹, R²: bedeuten unabhängig voneinander C₁-C₈-Alkyl, C₂-C₈-Alkenyl, C₂-C₈-Alkinyl, C₃-C₆-Cycloalkyl, C₃-C₆-Halogencycloalkyl, wobei R¹ und R² ihrerseits partiell oder vollständig halogeniert sein oder eine bis vier Gruppen R^{v} tragen können:
R^{v} bedeutet Cyano, C₁-C₆-Alkyl, C₃-C₈-Cycloalkyl, C₄-C₆-Cycloalkenyl, Hydroxy, C₁-C₆-Alkoxy, C₂-C₈-Alkenyloxy, C₂-C₈-Alkinyloxy, C₃-C₆-Cycloalkyloxy, C₄-C₆-Cycloalkenyloxy, C₁-C₆-Alkylthio, -C(=O)-A, -C(=O)-O-A, -C(=O)-N(A')A, C(A')(=N-OA), N(A')A, N(A')-C(=O)-A, N(A")-C(=O)-N(A')A, S(=O)ₘ-A, S(=O)ₘ-O-A oder S(=O)ₘ N(A')A oder Phenyl, wobei der Phenylteil ein bis drei Reste ausgewählt aus der Gruppe: Halogen, C₁-C₆-Alkyl, C₂-C₆-Alkenyl, C₂-C₆-Alkinyl, C₃-C₆-Cycloalkyl, C₁-C₆-Halogenalkyl, C₁-C₆-Alkoxy, Cyano, Nitro, -C(=O)-A, -C(=O)-O-A, -C(=O)-N(A')A, C(A')(=N-OA), N(A')A tragen kann;
- R¹: kann zusätzlich Wasserstoff bedeuten;
- R¹ und R²: können auch zusammen mit dem Stickstoff- oder Kohlenstoffatom, an das sie gebunden sind, einen gesättigten oder ungesättigten fünf- oder sechsgliedrigen Ring bilden, der durch eine Ether -(-O-), Carbonyl -(C=O)-, Thio -(-S-), Sulfoxyl -(-S[=O]-) oder Sulfenyl -(-SO₂-) oder eine weitere Amino -(-N(R^{a})-Gruppe, wobei R^{a} Wasserstoff oder C₁-C₆-Alkyl bedeutet, unterbrochen sein und/oder einen oder mehrere Substituenten aus der Gruppe Halogen, C₁-C₆-Alkyl, C₁-C₆-Halogenalkyl und Oxy-C₁-C₃-alkylenoxy enthalten kann; wobei R¹, R² vorzugsweise bedeuten:
- R¹, R²: unabhängig voneinander C₁-C₆-Alkyl, C₂-C₆-Alkenyl, C₂-C₆-Alkinyl, C₃-C₆-Cycloalkyl, C₃-C₆-Halogencycloalkyl, wobei die aliphatischen Gruppen der Restedefinitionen von R¹ und R² ihrerseits partiell oder vollständig halogeniert sein oder eine bis vier Gruppen R^{v} tragen können:
R^{v} Cyano, C₃-C₆-Cycloalkyl, C₄-C₆-Cycloalkenyl, Hydroxy, C₁-C₆-Alkoxy, C₂-C₈-Alkenyloxy, C₂-C₈-Alkinyloxy, C₃-C₆-Cycloalkyloxy, C₄-C₆-Cycloalkenyloxy, C₁-C₆-Alkylthio, -C(=O)-A, -C(=O)-O-A, -C(=O)-N(A')A, C(A')(=N-OA), N(A')A, N(A')-C(=O)-A, N(A")-C(=O)-N(A')A, S(=O)ₘ-A, S(=O)ₘ-O-A oder S(=O)ₘ-N(A')A oder Phenyl, wobei der Phenylteil ein bis drei Reste ausgewählt aus der Gruppe: Halogen, C₁-C₆-Alkyl, C₂-C₆-Alkenyl, C₂-C₆-Alkinyl, C₃-C₆-Cycloalkyl, C₁-C₆-Halogenalkyl, C₁-C₆-Alkoxy, Cyano, Nitro, -C(=O)-A, -C(=O)-O-A, -C(=O)-N(A')A, C(A')(=N-OA), N(A')A tragen kann;
- R¹: kann zusätzlich Wasserstoff bedeuten;
- R¹ und R²: können auch zusammen mit dem Stickstoff- oder Kohlenstoffatom, an das sie gebunden sind, einen gesättigten oder ungesättigten fünf- oder sechsgliedrigen Ring bilden, der durch eine Ether -(-O-), Carbonyl -(C=O)-, Thio -(-S-), Sulfoxyl -(-S[=O]-) oder Sulfenyl -(-SO₂-) oder eine weitere Amino -(-N(R^{a})-Gruppe, wobei R^{a} Wasserstoff oder C₁-C₆-Alkyl bedeutet, unterbrochen sein und/oder einen oder mehrere Substituenten aus der Gruppe Halogen, C₁-C₆-Alkyl, C₁-C₆-Halogenalkyl und Oxy-C₁-C₃-alkylenoxy enthalten kann;
- R³: bedeutet Halogen, Cyano, C₁-C₄-Alkyl, C₂-C₄-Alkenyl, C₂-C₄-Alkinyl, C₃-C₆-Cycloalkyl, C₁-C₄-Alkoxy, C₃-C₄-Alkenyloxy, C₃-C₄-Alkinyloxy, C₁-C₆-Alkylthio, Di-(C₁-C₆-alkyl)amino oder C₁-C₆-Alkylamino, wobei die Alkyl, Alkenyl und Alkinylreste von R³ durch Halogen, Cyano, Nitro, C₁-C₂-Alkoxy oder C₁-C₄-Alkoxycarbonyl substituiert sein können;
- R⁴: bedeutet ein fünf- oder sechsgliedriger gesättigter, partiell ungesättigter oder aromatischer mono- oder bicyclischer Heterocyclus, enthaltend ein bis vier Heteroatome aus der Gruppe O, N oder S, der seinerseits partiell oder vollständig halogeniert sein oder eine bis vier Gruppen R^{u} tragen kann:
R^{u} bedeutet Cyano, C₁-C₈-Alkyl, C₂-C₈-Alkenyl, C₂-C₈-Alkinyl, C₁-C₆-Alkoxy, C₃-C₆-Cycloalkyl, C₂-C₈-Alkenyloxy, C₂-C₈-Alkinyloxy, C₄-C₆-Cycloalkenyl, C₃-C₆-Cycloalkyloxy, C₄-C₆-Cycloalkenyloxy, -C(=O)-A, -C(=O)-O-A, -C(=O)-N(A')A, C(A')(=N-OA), N(A')A, N(A')-C(=O)-A, N(A")-C(=O)-N(A')A, S(=O)ₘ-A, S(=O)ₘ-O-A oder S(=O)ₘ-N(A')A, wobei m, A, A', A" die vorgenannte Bedeutung haben;
weiterhin kann R⁴ bedeuten:
Cyano, C(=Z)OR^{a}, C(=Z)NR^{z}R^{b}, C(=Z)NR^{a}-NR^{z}R^{b}, C(=Z)R^{a}, CR^{a}R^{b}-OR^{z}, CR^{a}R^{b}-NR^{z}R^{c},
   ON(=CR^{a}R^{b}), O-C(=Z)R^{a},
   NR^{a}R^{b'}, NR^{a}(C(=Z)R^{b}), NR^{a}(C(=Z)OR^{b}), NR^{a}(C(=Z)-NR^{z}R^{b}), NR^{a}(N=CR^{c}R^{b}), NR^{a}-NR^{z}R^{b}, NR^{z}-OR^{a}, wobei
   - Z: O, S, NR^{a} , NOR^{a} oder N-NR^{z}R^{c} bedeutet;
   - R^{a},R^{b},R^{c}: unabhängig voneinander für Wasserstoff, C₁-C₆-Alkyl, C₂-C₆-Alkenyl, C₂-C₆-Alkinyl, C₃-C₆-Cycloalkyl oder C₄-C₆-Cycloalkenyl stehen;
   - R^{b'}: bis auf Wasserstoff die gleichen Bedeutungen wie R^{b} hat;
   - R^{z}: die gleichen Bedeutungen wie R^{a} hat und zusätzlich -CO-R^{a} bedeuten kann;
   wobei die aliphatischen oder alicyclischen Gruppen der Restedefinitionen von R^{a},R^{b},R^{c} oder R^{z} ihrerseits partiell oder vollständig halogeniert sein oder eine bis vier Gruppen R^{w} tragen können:
   - R^{w}: bedeutet Halogen, Cyano, C₁-C₈-Alkyl, C₂-C₁₀-Alkenyl, C₂-C₁₀-Alkinyl, C₁-C₆-Alkoxy, C₂-C₁₀-Alkenyloxy, C₂-C₁₀-Alkinyloxy, C₃-C₆-Cycloalkyl, C₃-C₆-Cycloalkenyl, C₃-C₆-Cycloalkoxy, C₃-C₆-Cycloalkenyloxy, und wobei zwei der Reste R^{a}, R^{b}, R^{c} oder R^{z} zusammen mit den Atomen an die sie gebunden sind einen fünf- bis sechsgliedrigen gesättigten, partiell ungesättigten oder aromatischen Heterocyclus enthaltend ein bis vier Heteroatome aus der Gruppe O, N oder S, bilden können.

- Ⓑ: steht für fünf- oder sechsgliedriges Hetaryl enthaltend 1 bis 3 Heteroatome ausgewählt aus der Gruppe O, N oder S oder Phenyl;
- n: ist eine ganze Zahl von 1 bis 5;
- L: bedeutet Halogen, Cyano, Cyanato (OCN), C₁-C₈-Alkyl, C₂-C₈-Alkenyl, C₂-C₈-Alkinyl. C₁-C₆-Alkoxy, C₂-C₈-Alkenyloxy, C₂-C₈-Alkinyloxy, C₃-C₆-Cycloalkyl, C₄-C₆-Cycloalkenyl, C₃-C₆-Cycloalkyloxy, C₄-C₆-Cycloalkenyloxy, Nitro, -C(=O)-A, -C(=O)-O-A, -C(=O)-N(A')A, -C(=S)-N(A')A, C(A')(=N-OA), N(A')A, N(A')-C(=O)-A, N(A")-C(=O)-N(A')A, S(=O)ₘ-A, S(=O)ₘ-O-A oder S(=O)ₘ-N(A')A,
m ist 0, 1 oder 2;
A, A', A" sind unabhängig voneinander Wasserstoff, C₁-C₆-Alkyl, C₂-C₆-Alkenyl, C₂-C₆-Alkinyl, C₃-C₈-Cycloalkyl, C₃-C₈-Cycloalkenyl, Phenyl, wobei die organischen Reste partiell oder vollständig halogeniert sein können oder durch Nitro, Cyanato, Cyano oder C₁-C₄-Alkoxy substituiert sein können; oder A und A' stehen zusammen mit den Atomen, an die sie gebunden sind, für einen fünf- bis sechsgliedrigen gesättigten, partiell ungesättigten oder aromatischen Heterocyclus, enthaltend ein bis vier Heteroatome aus der Gruppe O, N oder S; wobei die aliphatischen Gruppen der Restedefinitionen von L ihrerseits partiell oder vollständig halogeniert sein oder eine bis vier Gruppen R^{L} tragen können:
R^{L} bedeutet Cyano, C₁-C₆-Alkoxy, C₃-C₈-Cycloalkyl, C₂-C₈-Alkenyloxy, C₂-C₈-Alkinyloxy, C₄-C₆-Cycloalkenyl, C₃-C₆-Cycloalkyloxy, C₄-C₆-Cycloalkenyloxy, -C(=O)-A, -C(=O)-O-A, -C(=O)-N(A')A, C(A')(=N-OA), N(A')A, N(A')-C(=O)-A, N(A")-C(=O)-N(A')A, S(=O)ₘ-A, S(=O)ₘ-O-A oder S(=O)ₘ N(A')A.

Aus der Literatur sind bisher 2-substituierte Pyrimidine mit fungizider Wirkung bekannt (EP-A 407899, WO-A 02/074753 und WO-A 03/043993).

Die Wirkung der o.g. Pyrimidine (Pyridine) ist jedoch in vielen Fällen nicht vollauf zufriedenstellend. Daher lag als Aufgabe zugrunde, weitere Verbindungen mit fungizider Wirkung bereitzustellen.

Demgemäß wurden die eingangs definierten 2-substituierten Pyridine I gefunden. Außerdem wurden Verfahren zu ihrer Herstellung sowie sie enthaltende Mittel zur Bekämpfung von Schadpilzen und ihre Verwendung in diesem Sinne gefunden.

Die Verbindungen der Formel I können auf verschiedenen Wegen erhalten werden.

Die beschriebenen Verbindungen können beispielsweise ausgehend von entsprechend substituierten Phenylacetonitrilen II hergestellt werden. Diese sind bekannt oder analog zu den bekannten Substanzen zugänglich.

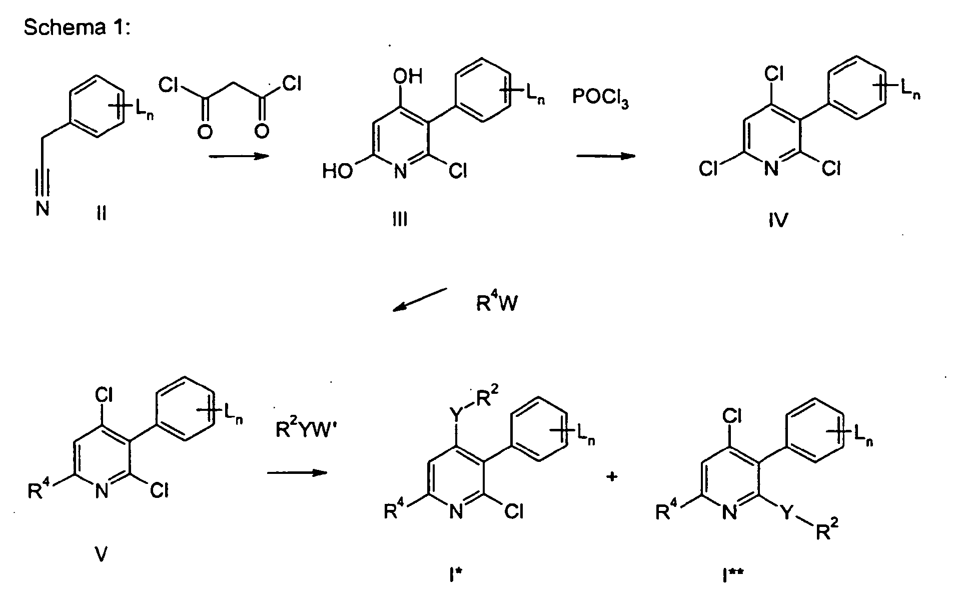

Die Phenylacetonitrile II können mit Malonylchlorid zu den Dihydroxypyridin-Derivaten III umgesetzt werden (s. Schema 1). Diese Reaktion kann ohne Lösungsmittel erfolgen; man kann aber auch ein Lösungsmittel verwenden, das unter den Reaktionsbedingungen inert ist und die Reaktanden ausreichend löst. Die Reaktionstemperatur kann zwischen -20°C und 150°C, bevorzugt zwischen 0°C und 100°C liegen.

Die so zugänglichen Dihydroxypyridine III können dann nach den üblichen Methoden zu den Trichlorpyridinen IV chloriert werden. Besonders bewährt hat sich dabei die Verwendung von Phosphoroxychlorid, ggf. unter Zugabe eines Amins wie Diethylanilin, eines Amin-Hydrochlorids oder von Dimethylformamid. Normalerweise ist es vorteilhaft, die Reaktion bei erhöhter Temperatur durchzuführen, um so den Umsatz zu steigern.

Trichlorpyridine IV können nun auf verschiedene Weise weiter substituiert werden. Dabei hat sich gezeigt, daß die Regioselektivität oft unerwartet stark von den gewählten Reaktionspartnern und -Bedingungen abhängt. Auf der in Schema 1 gezeigten Route wird zunächst der Substituent (R⁴) in die 6-Position eingeführt und dann das Amin nucleophil in 2- bzw. 4-Position verknüpft. Man kann die Reihenfolge dieser Umsetzungen aber auch verändern, wenn die relativen Reaktivitäten der Reaktionszentren dies erfordern.

Für die Einführung eines heterocyclischen Restes R⁴ in 6-Position können je nach Nukleophilie der Heterocyclus direkt eingesetzt werden (wie z.B. Pyrazol, Triazol). In diesen Fällen wird in der Regel eine Hilfsbase eingesetzt, W bedeutet hier Wasserstoff. Heterocyclische Substituenten können auch über Palladium- oder Nickel-katalysierte Reaktionen eingeführt werden. Dabei trägt der Heterocyclus eine geeignete metallorganische Abgangsgruppe. W steht hier für einen metallorganischen Bor-, Zinn (wie in den Synthesebeispielen A bis C), Zink, Magnesium oder Eisen-Rest.

Andere wichtige Zwischenprodukte der Formel V lassen sich wie in Schema 2 dargestellt herstellen. In 6-Position lässt sich via nukleophile Substitution eine Thiolat-Gruppe (C₁-C₆-Alkylthio) einführen, die zur C₁-C₆-Alkylsulfenyl (C₁-C₆-A)kylS[=O]₂-) oxidiert und so in eine Abgangsgruppe für weitere Austausch-Reaktionen verwendet werden kann. Als Oxidationsmittel haben sich insbesondere Wasserstoffperoxid oder Persäuren organischer Carbonsäuren bewährt. Die Oxidation kann jedoch auch beispielsweise mit Selendioxid durchgeführt werden. Beispielsweise lassen sich auf diese Weise in 6-Position Cyanide (Nitrile) einführen, die dann nach bekannten Methoden weiter z. B. zu Amiden, Amidoximen, Amidinen umgesetzt werden können. Beispielsweise lassen sich Amidoxime aus den Nitrilen und Hydroxylamin oder O-alkylierten Hydroxylaminen herstellen.

5-Brom-substituierte Pyridinderivate lassen sich wie in Schema 2 gezeigt beispielsweise durch Metallierung und Halogenierung aus dem Trichlorpyridin IV sowie analogen Substanzen herstellen. IV' kann dann über das Alkyllthiolat Va, Oxidation zu Vb zum Nitril Vc umgesetzt werden. Dieses kann nun als Zwischenprodukt z. B. zur Synthese von Amiden, Estern und Amidoximen (wie Vc) verwendet werden. Dabei kommen die üblichen Verfahren zur Umwandlung dieser funktionellen Gruppen zum Einsatz. Um O-alkylierte Amidoxime zu erhalten, kann man entweder Vd alkylieren (z. B. mit Methyliodid und einer Base wie Natriumhydrid oder Kalium-tert.-butanolat in Dimethylsulfoxid) oder ein Nitril wie Vc direkt mit einem O-alkylierten Hydroxylamin zur Reaktion bringen.

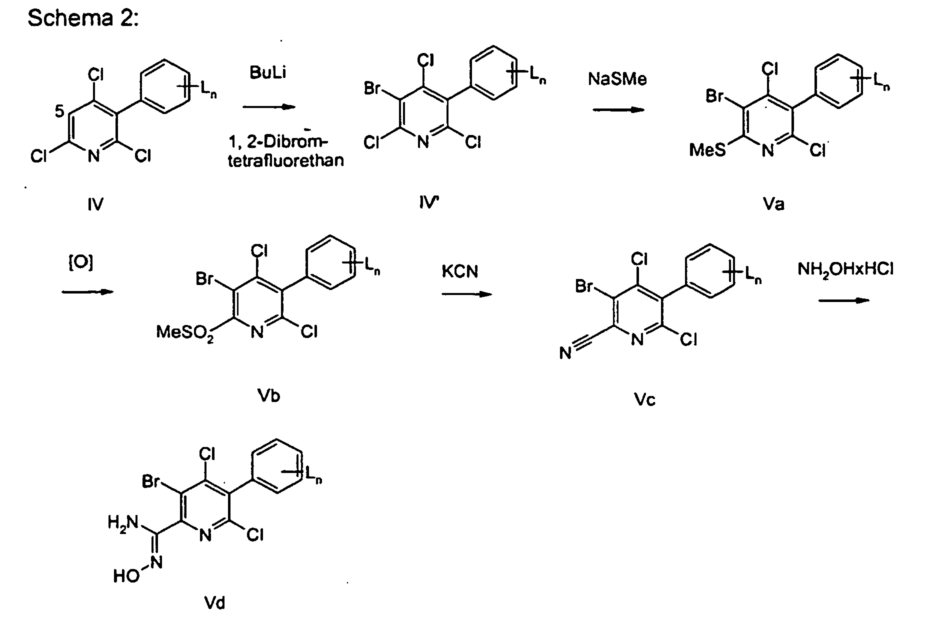

Vc und Vd sowie die aus diesen hervorgegangenen Zwischenprodukte (allgemein V) kann man dann wie oben beschrieben mit entsprechenden Nukleophilen (Aminen; Alkoholen , Merkaptanen) in die Wirkstoffe I überführen, wobei Y N-R¹-, -O- oder -S-bedeutet (s. Schema 3).
Die Reaktionstemperatur kann zwischen 0°C und 200°C liegen. Meist beschleunigt eine etwas erhöhte Temperatur die Reaktion. Falls erforderlich wird bei erhöhtem Druck gearbeitet, um diese Temperatur zu erreichen.

Sofern Y für einen Rest -CH-R¹ bzw. R³ für einen über Kohlenstoff gebundenen Rest stehen, werden diese Reste mittels metallorganischer Verbindungen unter Übergangsmetallkatalyse, wie Ni- oder Pd-Katalyse eingeführt. In manchen Fällen kann es ratsam sein, die Reihenfolge umzudrehen und den Substituenten R³ zuerst einzuführen.

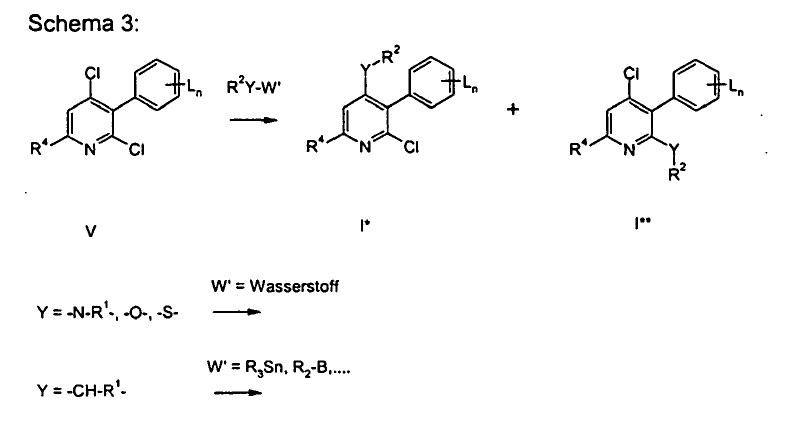

In diesem Fall steht die Formel R²Y-W' beispielsweise für eine Verbindung der Formel: W'= (R²CH(R¹))ₙ-Mⁿ. M für ein Metallion der Wertigkeit n, wie beispielsweise B, Zn, Mg oder Sn. Diese Reaktion kann beispielsweise analog folgender Methoden durchgeführt werden: J. Chem. Soc. Perkin Trans. 1, 1187 (1994), ebenda 1, 2345 (1996); WO-A 99/41255; Aust. J. Chem., Bd. 43, 733 (1990); J. Org. Chem., Bd. 43, 358 (1978); J. Chem. Soc. Chem. Commun. 866 (1979); Tetrahedron Lett., Bd. 34, 8267 (1993); ebenda, Bd. 33, 413 (1992).

Die obengenannten Angaben beziehen sich auch auf die Herstellung von Verbindungen, in denen R³ eine Alkylgruppe darstellt. Wie oben näher ausgeführt, kann eine derartige Akylgruppe (R³) mittels metallorganischen Verbindungen der Formel (R³)ₙ-Mⁿ, wobei M die obengenannte Bedeutung besitzt , hergestellt werden. Sofern R³ eine Cyangruppe oder einen Alkoxysubstituenten bedeutet, kann der Rest R³ durch Umsetzung mit Alkalimetallcyaniden bzw. Alkalimetallalkoholaten eingeführt werden.

Bei den in den vorstehenden Formeln angegebenen Definitionen der Symbole wurden Sammelbegriffe verwendet, die allgemein repräsentativ für die folgenden Substituenten stehen:
**Halogen:** Fluor, Chlor, Brom und Jod;
**Alkyl sowie die Alkylteile von beispielsweise Alkoxy, Alkylamino,Alkoxycarbonyl:** gesättigte, geradkettige oder verzweigte Kohlenwasserstoffreste mit 1 bis 4, 6 oder 8 Kohlenstoffatomen, z.B. C₁-C₆-Alkyl wie Methyl, Ethyl, Propyl, 1-Methylethyl, Butyl, 1-Methyl-propyl, 2-Methylpropyl, 1,1-Dimethylethyl, Pentyl, 1-Methylbutyl, 2-Methylbutyl, 3-Methylbutyl, 2,2-Di-methylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl;
**Halogenalkyl:** geradkettige oder verzweigte Alkylgruppen mit 1 bis 4, 6 oder 8 Kohlenstoffatomen (wie vorstehend genannt), wobei in diesen Gruppen teilweise oder vollständig die Wasserstoffatome durch Halogenatome wie vorstehend genannt ersetzt sein können, z.B. C₁-C₂-Halogenalkyl wie Chlormethyl, Brommethyl, Dichlormethyl, Trichlormethyl, Fluormethyl, Difluormethyl, Trifluormethyl, Chlorfluormethyl, Dichlorfluormethyl, Chlordifluormethyl, 1-Chlorethyl, 1-Bromethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl, Pentafluorethyl oder 1,1,1-Trifluorprop-2-yl;
**Alkenyl:** ungesättigte, geradkettige oder verzweigte Kohlenwasserstoffreste mit 2 bis 4, 6 oder 8 Kohlenstoffatomen und einer Doppelbindung in einer beliebigen Position, z.B. C₂-C₆-Alkenyl wie Ethenyl, 1-Propenyl, 2-Propenyl, 1-Methylethenyl, 1 -Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl,1-Ethyl-1-methyl-2-propenyl, 1-Ethyl-2-methyl-1 propenyl und 1-Ethyl-2-methyl-2-propenyl;
**Alkadienyl:** ungesättigte, geradkettige oder verzweigte Kohlenwasserstoffreste mit 4, bis 8 Kohlenstoffatomen und zwei Doppelbindungen in beliebiger Position;
**Halogenalkenyl:** ungesättigte, geradkettige oder verzweigte Kohlenwasserstoffreste mit 2 bis 8 Kohlenstoffatomen und einer Doppelbindung in einer beliebigen Position (wie vorstehend genannt), wobei in diesen Gruppen die Wasserstoffatome teilweise oder vollständig gegen Halogenatome wie vorstehend genannt, insbesondere Fluor, Chlor und Brom, ersetzt sein können;
**Alkinyl:** geradkettige oder verzweigte Kohlenwasserstoffgruppen mit 2 bis 8 Kohlenstoffatomen und einer Dreifachbindung in einer beliebigen Position, z.B. C₂-C₆-Alkinyl wie Ethinyl, 1-Propinyl, 2-Propinyl, 1-Butinyl, 2-Butinyl, 3-Butinyl, 1-Methyl-2-propinyl, 1-Pentinyl, 2-Pentinyl, 3-Pentinyl, 4-Pentinyl, 1-Methyl-2-butinyl, 1-Methyl-3-butinyl, 2-Methyl-3-butinyl, 3-Methyl-1-butinyl, 1,1-Dimethyl-2-propinyl, 1-Ethyl-2-propinyl, 1-Hexinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-1-pentinyl, 3-Methyl-4-pentinyl, 4-Methyl-1-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 3,3-Dimethyl-1-butinyl, 1-Ethyl-2-butinyl, 1-Ethyl-3-butinyl, 2-Ethyl-3-butinyl und 1-Ethyl-1-methyl-2-propinyl;
**Cycloalkyl:** mono- oder bicyclische, gesättigte Kohlenwasserstoffgruppen mit 3 bis 6 Kohlenstoffringgliedern, z.B. C₃-C₆-Cycloalkyl wie Cyclopropyl, Cyclobutyl, Cyclopentyl, Cyclohexyl;
   - **fünf- bis sechsgliedriger gesättigter, partiell ungesättigter oder aromatischer Heterocyclus, enthaltend ein bis vier Heteroatome aus der Gruppe O, N oder S:** z.B. 2-Tetrahydrofuranyl, 3-Tetrahydrofuranyl, 2-Tetrahydrothienyl, 3-Tetrahydrothienyl, 2-Pyrrolidinyl, 3-Pyrrolidinyl, 3-Isoxazolidinyl, 4-Isoxazolidinyl, 5-Isoxazolidinyl, 3-Isothiazolidinyl, 4-Isothiazolidinyl, 5-Isothiazolidinyl, 3-Pyrazolidinyl, 4-Pyrazolidinyl, 5-Pyrazolidinyl, 2-Oxazolidinyl, 4-Oxazolidinyl, 5-Oxazolidinyl, 2-Thiazolidinyl, 4-Thiazolidinyl, 5-Thiazolidinyl, 2-Imidazolidinyl, 4-Imidazolidinyl, 1,2,4-Oxadiazolidin-3-yl, 1,2,4-Oxadiazolidin-5-yl, 1,2,4-Thiadiazolidin-3-yl, 1,2,4-Thiadiazolidin-5-yl, 1,2,4-Triazolidin-3-yl, 1,3,4-Oxadiazolidin-2-yl, 1,3,4-Thiadiazolidin-2-yl, 1,3,4-Triazolidin-2-yl, 2,3-Dihydrofur-2-yl, 2,3-Dihydrofur-3-yl, 2,4-Dihydrofur-2-yl, 2,4-Dihydrofur-3-yl, 2,3-Dihydrothien-2-yl, 2,3-Dihydrothien-3-yl, 2,4-Dihydrothien-2-yl, 2,4-Dihydrothien-3-yl, 2-Pyrrolin-2-yl, 2-Pyrrolin-3-yl, 3-Pyrrolin-2-yl, 3-Pyrrolin-3-yl, 2-Isoxazolin-3-yl, 3-Isoxazolin-3-yl, 4-Isoxazolin-3-yl, 2-Isoxazolin-4-yl, 3-Isoxazolin-4-yl, 4-Isoxazolin-4-yl, 2-Isoxazolin-5-yl, 3-Isoxazolin-5-yl, 4-Isoxazolin-5-yl, 2-Isothiazolin-3-yl, 3-Isothiazolin-3-yl, 4-Isothiazolin-3-yl, 2-Isothiazolin-4-yl, 3-Isothiazolin-4-yl, 4-Isothiazolin-4-yl, 2-Isothiazolin-5-yl, 3-Isothiazolin-5-yl, 4-Isothiazolin-5-yl, 2,3-Dihydropyrazol-1-yl, 2,3-Dihydropyrazol-2-yl, 2,3-Dihydropyrazol-3-yl, 2,3-Dihydropyrazol-4-yl, 2,3-Dihydropyrazol-5-yl, 3,4-Dihydropyrazol-1-yl, 3,4-Dihydropyrazol-3-yl, 3,4-Dihydropyrazol-4-yl, 3,4-Dihydropyrazol-5-yl, 4,5-Dihydropyrazol-1-yl, 4,5-Dihydropyrazol-3-yl, 4,5-Dihydropyrazol-4-yl, 4,5-Dihydropyrazol-5-yl, 2,3-Dihydrooxazol-2-yl, 2,3-Dihydrooxazol-3-yl, 2,3-Dihydrooxazol-4-yl, 2,3-Dihydrooxazol-5-yl, 3,4-Dihydrooxazol-2-yl, 3,4-Dihydrooxazol-3-yl, 3,4-Dihydrooxazol-4-yl, 3,4-Dihydrooxazol-5-yl, 3,4-Dihydrooxazol-2-yl, 3,4-Dihydrooxazol-3-yl, 3,4-Dihydrooxazol-4-yl, 2-Piperidinyl, 3-Piperidinyl, 4-Piperidinyl, 1,3-Dioxan-5-yl, 2-Tetrahydropyranyl, 4-Tetrahydropyranyl, 2-Tetrahydrothienyl, 3-Hexahydropyridazinyl, 4-Hexahydropyridazinyl, 2-Hexahydropyrimidinyl, 4-Hexahydropyrimidinyl, 5-Hexahydropyrimidinyl, 2-Piperazinyl, 1,3,5-Hexahydrotriazin-2-yl und 1,2,4-Hexahydrotriazin-3-yl;
   - **Ringsystem, das gegebenenfalls von R¹ und R² bzw. von A und A' zusammen mit dem Stickstoff, an den sie gebunden sind, aufgespannt wird:** z.B. Pyrrolidin, Morpholin, Piperidin oder Tetrahydropyrazol.

In dem Umfang der vorliegenden Erfindung sind die (R)- und (S)-Isomere und die Racemate von Verbindungen der Formel I eingeschlossen, die chirale Zentren aufweisen.

Im folgenden werden die Ausführungsformen der Erfindung genauer beschrieben.

Im Hinblick auf die bestimmungsgemäße Verwendung der Pyridine der Formel I sind die folgenden Bedeutungen der Substituenten, und zwar jeweils für sich allein oder in Kombination, besonders bevorzugt:

Verbindungen I werden bevorzugt, in denen R¹ für C₁-C₆-Alkyl, C₁-C₆-Halogenalkyl, C₂-C₆-Alkenyl, C₂-C₆-Alkinyl oder C₃-C₆-Cycloalkyl und R² für Wasserstoff stehen.

Insbesondere werden Verbindungen I bevorzugt, in denen R¹ für in α-Stellung verzweigtes C₁-C₆-Alkyl, C₂-C₆-Alkenyl oder C₁-C₆-Halogenalkyl steht. Besonders bevorzugt sind diejenigen Verbindungen I, in denen R¹ die zuvor genannte Bedeutung und R² Wasserstoff bedeuten.

Daneben werden Verbindungen 1 bevorzugt, in denen R¹ für C₁-C₄-Halogenalkyl und R² für Wasserstoff stehen.

Außerdem werden Verbindungen I bevorzugt, in denen R¹ und R² zusammen mit dem Stickstoff, an das sie gebunden sind, einen fünf- oder sechsgliedrigen Ring bilden, der durch ein Sauerstoffatom unterbrochen sein kann und einen oder zwei C₁-C₆-Alkylsubstituenten tragen kann.

Insbesondere bevorzugt sind Gruppen NR¹R² (entspricht -NCH(R¹)-R²) wie - insbesondere in α-Stellung - methylierte Pyrrolidine oder Piperidine. Weiterhin ist 4-Methylpiperidin bevorzugt.

Weiterhin bevorzugt sind Pyridine, in denen der Substituent R²-Y- die folgenden Bedeutungen hat:
- Y: bedeutet eine Gruppe -CH-R¹- oder -N-R¹-, wobei R¹ Wasserstoff bedeutet, und
- R²: bedeutet in α-Stellung verzweigtes C₃-C₈-Alkyl, C₃-C₈-Alkenyl, C₃-C₈-Alkinyl oder C₃-C₆-Halogenalkyl.

Besonders bevorzugt werden auch Verbindungen I, in denen R³ C₁-C₄-Alkyl bedeutet, das durch Halogen substituiert sein kann.

Außerdem werden Verbindungen I besonders bevorzugt, in denen R³ für Halogen, Cyano, C₁-C₄-Alkyl oder C₁-C₄-Alkoxy steht.

Insbesondere werden Verbindungen I bevorzugt, in denen R³ Methyl, Cyano, Methoxy oder insbesondere Chlor bedeutet.

Ferner werden Verbindungen I bevorzugt, in denen R⁴ Pyrrol, Pyrazol, Imidazol, 1,2,3-Triazol, 1,2,4-Triazol, Tetrazol, Oxazol, Isoxazol, 1,3,4-Oxadiazol, Furan, Thiophen, Thiazol, Isothiazol, Pyridin, Pyrimidin, Pyrazin, Pyridazin, 1,2,3-Triazin, 1,2,4-Triazin, 1-Pyridin(1,2,-dihydro)-2-on oder 1-Pyrrolidon bedeutet, wobei der Heterocyclus über C oder N an den Pyridinring gebunden sein kann und bis zu drei Substituenten R^{u} tragen kann. Diese Bevorzugung ist sowohl in Kombination mit der in Anspruch 1 gegebenen, breiten Definition von R^{u} als auch mit der folgenden, engeren Definition von R^{u}: Halogen, Cyano, C₁-C₈-Alkyl, C₁-C₈-Halogenalkyl, C₁-C₆-Alkoxy, -C(=O)-A, -C(=O)-O-A, - C(=O)-N(A')A, C(A')(=N-OA), N(A')A, N(A')-C(=0)-A zu lesen.

Besonders bevorzugt sind Verbindungen I, in denen R⁴ 1-Pyrazolyl, 1-[1,2,4]Triazolyl, 2-Thiazolyl, 2-Pyridinyl, 2-Pyrimidinyl, 3-Pyridazinyl, 1-Pyridin(1,2-dihydro)-2-onyl oder 1-Pyrrolidonyl bedeutet. Diese Bevorzugung ist sowohl in Kombination mit der in Anspruch 1 gegebenen, breiten Definition von R^{u} als auch mit der folgenden, engeren Definition von R^{u} : Halogen, Cyano, C₁-C₈-Alkyl, C₁-C₈-Halogenalkyl, C₁-C₈-Alkoxy, -C(=O)-A, -C(=O)-O-A, -C(=O)-N(A')A, C(A')(=N-OA), N(A')A, N(A')-C(=O)-A zu lesen.

Weiterhin bevorzugt sind Verbindungen 1, in denen R⁴ Pyrazolyl oder [1,2,4]Triazolyl bedeutet.

Insbesondere bevorzugt sind Verbindungen I, in denen R⁴ 2-Pyrimidinyl bedeutet. Diese Bevorzugung ist sowohl in Kombination mit der in Anspruch 1 gegebenen, breiten Definition von R^{u} als auch mit der folgenden, engeren Definition von R^{u} : Halogen, Cyano, C₁-C₈-Alkyl, C₁-C₈-Halogenalkyl, C₁-C₆-Alkoxy, -C(=O)-A, -C(=O)-O-A, -C(=O)-N(A')A, C(A')(=N-OA), N(A')A, N(A')-C(=O)-A zu lesen.

Auch bevorzugt sind Verbindungen I, in denen R⁴ Cyano, C(=O)NR^{z}R^{b}, C(=NOR^{a})NR^{z}R^{b}, C(=NOR^{b})R^{a}, C(=N-NR^{z}R^{b})R^{a} oder CR^{a}R^{b}-NR^{z}R^{c}' ON(=CR^{a}R^{b}), NR^{a}(C(=O)R^{b}), NR^{a}(C(=O)OR^{b}), NR^{a}(N=CR^{c}R^{b}) oder NR^{z}-OR^{a} bedeutet.

Außerdem bevorzugt sind Verbindungen I, in denen R⁴ C(=Z)OR^{a}, C(=Z)NR^{z}R^{b} oder C(=Z)R^{a} und Z O, NR^{a} oder NOR^{a} bedeuten.

Insbesondere bevorzugt sind Verbindungen I, in denen R⁴ C(=O)NR^{z}R^{b} oder C(=NOCH₃)NR^{z}R^{b} und vorzugsweise C(=O)NH₂ oder C(=N-OCH₃)NH₂ bedeutet.

Weiterhin bevorzugt sind Verbindungen I, in denen R⁴ C(=NH)NR^{z}R^{b} und R^{z} einen A-cylsubstituenten: -CO-R^{a} bedeutet.
- Ⓑ: ist besonders bevorzugt fünfgliedriges Hetaryl enthaltend 1 bis 3 Heteroatome ausgewählt aus der Gruppe O, N oder S oder Pyridyl oder besonders bevorzugt Phenyl;
Insbesondere werden Pyridine I bevorzugt, wobei die Substituenten L (L¹ bis L⁵) die folgende Bedeutung haben:
- L: Halogen, Cyano, Methyl, Methoxy, -C(=O)-O-A, -C(=O)-N(A')A, -C(=S)-N(A')A, C(A')(=N-OA), N(A')A, N(A')-C(=O)-A,
A,A' unabhängig voneinander Wasserstoff, C₁-C₆-Alkyl, C₂-C₆-Alkenyl, C₂-C₆-Alkinyl, Phenyl, wobei die organischen Reste partiell oder vollständig halogeniert sein können oder durch C₁-C₄-Alkoxy substituiert sein können, oder A und A' zusammen mit den Atomen an die sie gebunden sind für einen fünf- bis sechsgliedrigen gesättigten Heterocyclus, enthaltend ein oder zwei Heteroatome aus der Gruppe O, N oder S stehen;

Außerdem werden Pyridine 1 bevorzugt, wobei die durch Lₙ substituierte Gruppe B 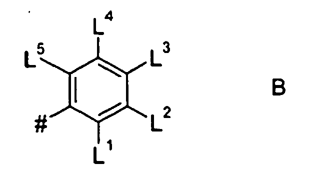 bedeutet, worin # die Verknüpfungsstelle mit dem Pyridin-Gerüst ist und
- L¹: Fluor, Chlor, CH₃ oder CF₃;
- L², L⁴: unabhängig voneinander Wasserstoff, CH₃ oder Fluor;
- L³: Wasserstoff, Fluor, Chlor, Brom, Cyano, CH₃, SCH₃, OCH₃, SO₂CH₃, CO-NH₂, CO-NHCH₃, CO-NHC₂H₅, CO-N(CH₃)₂, CS-NH₂, CS-NHCH₃, CS-N(CH₃)₂, NH-C(=O)CH₃, N(CH₃)-C(=O)CH₃ oder COOCH₃ und
- L⁵: Wasserstoff, Fluor, Chlor oder CH₃ bedeuten.

Weiterhin sind 2-substituierte Pyridine der Formel bevorzugt, 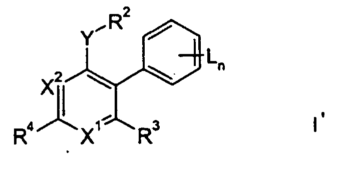 wobei
- Y: eine Gruppe -CH-R¹-, -N-R¹- oder -O- bedeutet;
- R¹,R²: unabhängig voneinander C₁-C₆-Alkyl, C₂-C₆-Alkenyl, C₂-C₆-Alkinyl, C₁-C₆-Halogenalkyl, C₂-C₆-Halogenalkenyl oder C₂-C₆-Halogenalkinyl bedeuten;
- R¹: zusätzlich Wasserstoff bedeuten kann;
- R¹ und R²: auch zusammen mit dem Stickstoffatom, an das sie gebunden sind, einen gesättigten oder ungesättigten fünf- oder sechsgliedrigen Ring bilden können, der durch eine Ether -(-O-) oder eine weitere Amino-(-N(R^{a})- Gruppe, wobei R^{a} Wasserstoff oder C₁-C₆-Alkyl bedeutet, unterbrochen sein und/oder einen oder mehrere Substituenten aus der Gruppe Halogen, C₁-C₆-Alkyl, C₁-C₆-Halogenalkyl und Oxy-C₁-C₃-alkylenoxy enthalten kann;
- R³: Halogen, Cyano, C₁-C₄-Alkyl, C₁-C₄-Alkoxy oder C₁-C₄-Haloalkyl bedeutet;
- R⁴: Pyrrol, Pyrazol, Imidazol, 1,2,3-Triazol, 1,2,4-Triazol, Tetrazol, Oxazol, Isoxazol, 1,3,4-Oxadiazol, Furan, Thiophen, Thiazol, Isothiazol, Pyridin, Pyrimidin, Pyrazin, Pyridazin, 1,2,3-Triazin, 1,2,4-Triazin, 1-Pyridin(1,2,-dihydro)-2-on oder 1-Pyrrolidon bedeutet, wobei der Heterocyclus über C oder N an den Pyridinring gebunden sein kann und bis zu drei Substituenten R^{u} tragen kann;
R^{u} Halogen, Cyano, C₁-C₈-Alkyl, C₁-C₆-Alkoxy, -C(=O)-A, -C(=O)-O-A, -C(=O)-N(A')A, C(A')(=N-OA), N(A')A, N(A')-C(=O)-A,
oder
Cyano, C(=O)NR^{z}R^{b}, C(=NOR^{a})NR^{z}R^{b}, C(=NOR^{b})R^{a}, C(=N-NR^{z}R^{b})R^{a} oder CR^{a}R^{b}-NR^{z}R^{c}, ON(=CR^{a}R^{b}), NR^{a}(C(=O)R^{b}), NR^{a}(C(=O)OR^{b}), N-R^{a}(N=CR^{c}R^{b}) oder NR^{z}-OR^{a} bedeutet;
- n: eine ganze Zahl von 1 bis 3 ist, wobei mindestens ein Substituent L in ortho-Stellung am Phenylring sitzt;
- L: Halogen, Cyano, Methyl, Methoxy, -C(=O)-O-A, -C(=O)-N(A')A, -C(=S)-N(A')A, C(A')(=N-OA), N(A')A, N(A')-C(=O)-A bedeutet, wobei
A,A' unabhängig voneinander Wasserstoff, C₁-C₆-Alkyl, C₂-C₆-Alkenyl, C₂-C₆-Alkinyl, Phenyl bedeuten, wobei die organischen Reste partiell oder vollständig halogeniert sein können oder durch C₁-C₄-Alkoxy substituiert sein können, oder A und A' zusammen mit den Atomen an die sie gebunden sind für einen fünf- bis sechsgliedrigen gesättigten Heterocyclus, enthaltend ein oder zwei Heteroatome aus der Gruppe O, N oder S stehen;
wobei die aliphatischen Gruppen der Restedefinitionen von L ihrerseits partiell oder vollständig halogeniert sein können.

Insbesondere sind im Hinblick auf ihre Verwendung die in den folgenden Tabellen zusammengestellten Verbindungen I bevorzugt. Die in den Tabellen für einen Substituenten genannten Gruppen stellen außerdem für sich betrachtet, unabhängig von der Kombination, in der sie genannt sind, eine besonders bevorzugte Ausgestaltung des betreffenden Substituenten dar.

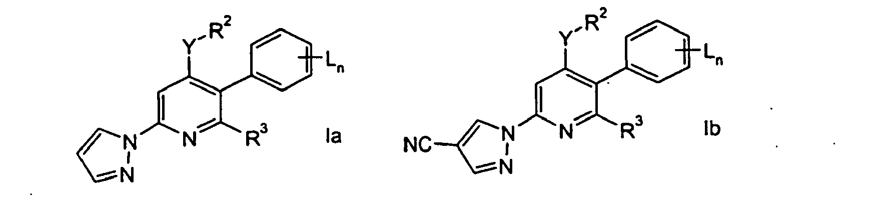

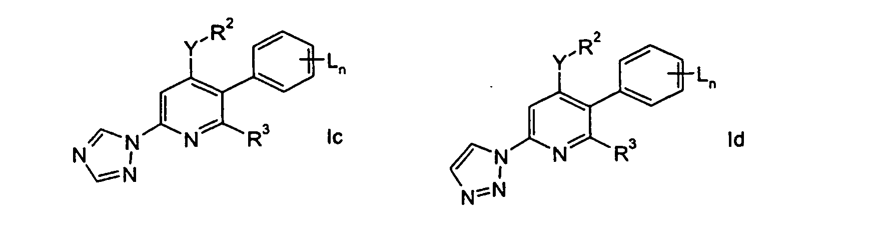

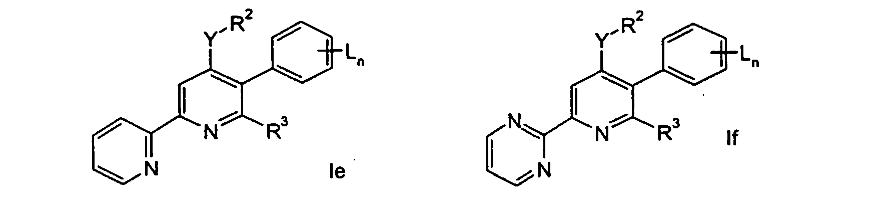

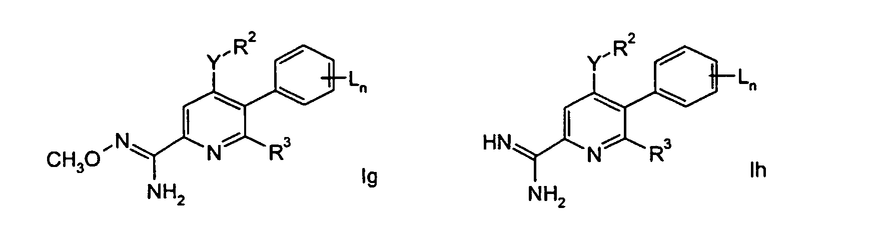

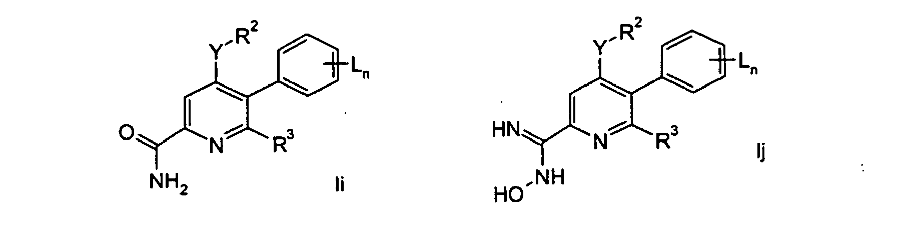

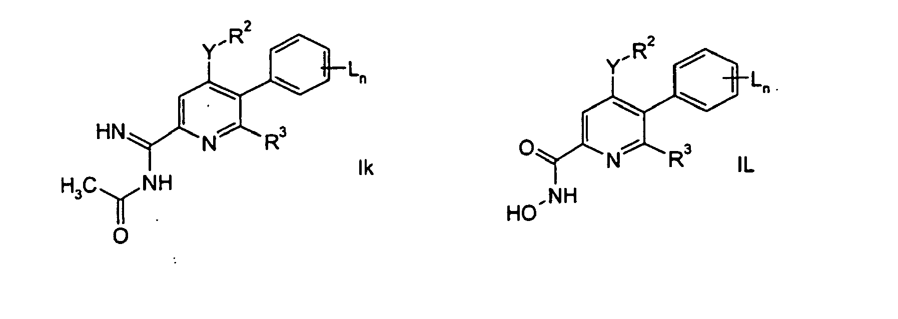

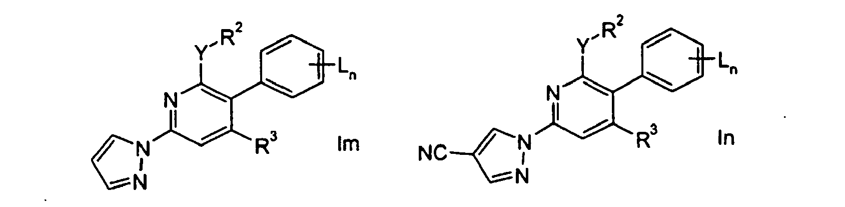

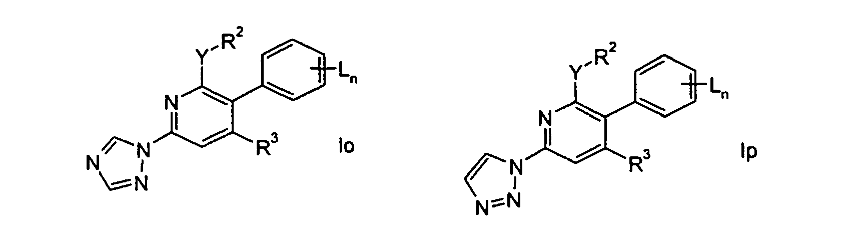

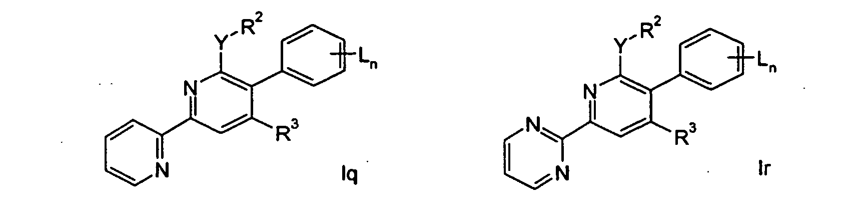

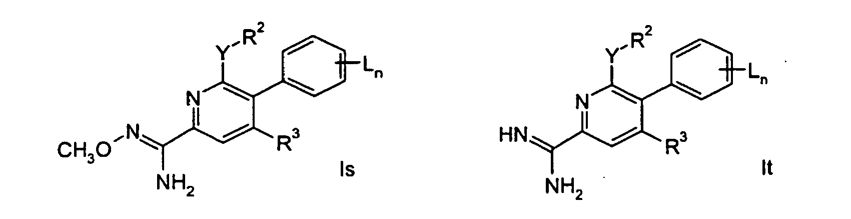

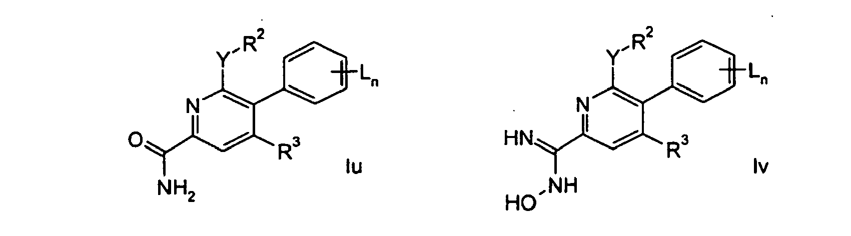

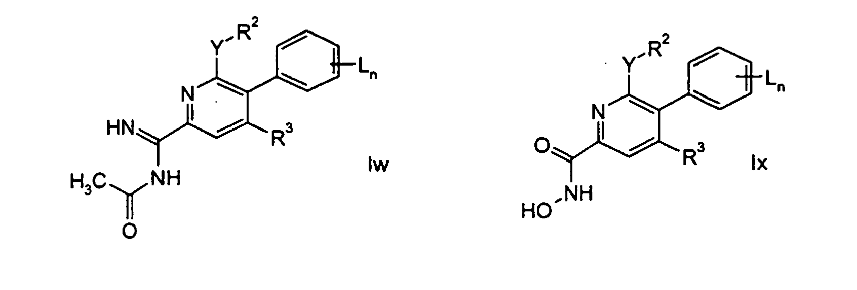

### Tabelle 1

Verbindungen der Formel Ia, Ib, Ic, Id, Ie, If, Ig, Ih, li, Ij, Ik, IL, Im, In, Io, Ip, Iq, Ir, Is, It, lu, Iv, Iw und Ix, in denen Lₙ 2-Fluor,6-chlor, R³ Methyl bedeuten und YR² für eine Verbindung jeweils einer Zeile der Tabelle A entspricht

### Tabelle 2

Verbindungen der Formel la, Ib, Ic, Id, le, If, Ig, Ih, Ii, Ij, Ik, IL, Im, In, Io, Ip, Iq, Ir, Is, lt, lu, Iv, Iw und Ix, in denen Lₙ 2,6-Difluor, R³ Methyl bedeuten und YR² für eine Verbindung jeweils einer Zeile der Tabelle A entspricht

### Tabelle 3

Verbindungen der Formel Ia, Ib, Ic, Id, Ie, If, Ig, Ih, li, Ij, Ik, IL, Im, In, Io, Ip, Iq, Ir, Is, It, lu, Iv, Iw und Ix, in denen Lₙ 2,6-Dichlor, R³ Methyl bedeuten und YR² für eine Verbindung jeweils einer Zeile der Tabelle A entspricht

### Tabelle 4

Verbindungen der Formel Ia, Ib, Ic, Id, le, If, Ig, Ih, li, Ij, Ik, IL, Im, In, Io, Ip, Iq, Ir, Is, It, lu, Iv, Iw und Ix, in denen Lₙ 2-Fluor,6-methyl, R³ Methyl bedeuten und YR² für eine Verbindung jeweils einer Zeile der Tabelle A entspricht

### Tabelle 5

Verbindungen der Formel Ia, Ib, Ic, Id, le, If, Ig, Ih, Ii, Ij, Ik, IL, Im, In, Io, Ip, Iq, Ir, Is, It, lu, Iv, Iw und Ix, in denen Lₙ 2,4,6-Trifluor, R³ Methyl bedeuten und YR² für eine Verbindung jeweils einer Zeile der Tabelle A entspricht

### Tabelle 6

Verbindungen der Formel Ia, Ib, Ic, Id, le, If, Ig, Ih, li, Ij, Ik, IL, Im, In, Io, Ip, Iq, Ir, Is, It, lu, Iv, Iw und Ix, in denen Lₙ 2-Methyl,4-fluor, R³ Methyl bedeuten und YR² für eine Verbindung jeweils einer Zeile der Tabelle A entspricht

### Tabelle 7

Verbindungen der Formel Ia, Ib, Ic, Id, le, If, Ig, Ih, li, Ij, Ik, IL, Im, In, Io, Ip, Iq, Ir, Is, It, lu, Iv, Iw und Ix, in denen Lₙ 2-Fluor,4-methoxycarbonyl, R³ Methyl bedeuten und YR² für eine Verbindung jeweils einer Zeile der Tabelle A entspricht

### Tabelle 8

Verbindungen der Formel Ia, Ib, Ic, Id, le, If, Ig, Ih, li, Ij, Ik, IL, Im, In, Io, Ip, Iq, Ir, Is, It, lu, Iv, Iw und Ix, in denen Lₙ 2-Fluor,4-CN, R³ Methyl bedeuten und YR² für eine Verbindung jeweils einer Zeile der Tabelle A entspricht

### Tabelle 9

Verbindungen der Formel Ia, Ib, Ic, Id, Ie, If, Ig, Ih, Ii, Ij, Ik, IL, Im, In, Io, Ip, Iq, Ir, Is, It, lu, Iv, Iw und Ix, in denen Lₙ 2,4,5-Trifluor, R³ Methyl bedeuten und YR² für eine Verbindung jeweils einer Zeile der Tabelle A entspricht

### Tabelle 10

Verbindungen der Formel Ia, Ib, Ic, Id, Ie, If, Ig, Ih, Ii, Ij, Ik, IL, Im, In, Io, Ip, Iq, Ir, Is, It, lu, Iv, Iw und Ix, in denen Lₙ 2,4-Dichlor, R³ Methyl bedeuten und YR² für eine Verbindung jeweils einer Zeile der Tabelle A entspricht

### Tabelle 11

Verbindungen der Formel Ia, Ib, Ic, Id, Ie, If, Ig, Ih, Ii, Ij, Ik, IL, Im, In, Io, Ip, Iq, Ir, Is, It, lu, Iv, Iw und Ix, in denen Lₙ 2-Chlor, R³ Methyl bedeuten und YR² für eine Verbindung jeweils einer Zeile der Tabelle A entspricht

### Tabelle 12

Verbindungen der Formel Ia, Ib, Ic, Id, Ie, If, Ig, Ih, Ii, Ij, Ik, IL, Im, In, Io, Ip, Iq, Ir, Is, It, Iu, Iv, Iw und Ix, in denen Lₙ 2-Fluor, R³ Methyl bedeuten und YR² für eine Verbindung jeweils einer Zeile der Tabelle A entspricht

### Tabelle 13

Verbindungen der Formel Ia, Ib, Ic, Id, Ie, If, Ig, Ih, Ii, Ij, Ik, IL, Im, In, Io, Ip, Iq, Ir, Is, lt, lu, Iv, Iw und Ix, in denen Lₙ 2,4-Difluor, R³ Methyl bedeuten und YR² für eine Verbindung jeweils einer Zeile der Tabelle A entspricht

### Tabelle 14

Verbindungen der Formel Ia, Ib, Ic, Id, Ie, If, Ig, Ih, Ii, Ij, Ik, IL, Im, In, Io, Ip, Iq, Ir, Is, lt, lu, Iv, Iw und Ix, in denen Lₙ 2-Fluor-4-chlor, R³ Methyl bedeuten und YR² für eine Verbindung jeweils einer Zeile der Tabelle A entspricht

### Tabelle 15

Verbindungen der Formel Ia, Ib, Ic, Id, Ie, If, Ig, Ih, Ii, Ij, Ik, IL, Im, In, Io, Ip, Iq, Ir, Is, lt, Iu, Iv, Iw und Ix, in denen Lₙ 2-Chlor-4-fluor, R³ Methyl bedeuten und YR² für eine Verbindung jeweils einer Zeile der Tabelle A entspricht

### Tabelle 16

Verbindungen der Formel Ia, Ib, Ic, Id, Ie, If, Ig, Ih, Ii, Ij, Ik, IL, Im, In, Io, Ip, Iq, Ir, Is, It, Iu, Iv, Iw und Ix, in denen Lₙ 2,3-Difluor, R³ Methyl bedeuten und YR² für eine Verbindung jeweils einer Zeile der Tabelle A entspricht

### Tabelle 17

Verbindungen der Formel Ia, Ib, Ic, Id, Ie, If, Ig, Ih, Ii, Ij, Ik, IL, Im, In, Io, Ip, Iq, Ir, Is, It, Iu, Iv, Iw und Ix, in denen Lₙ 2,5-Difluor, R³ Methyl bedeuten und YR² für eine Verbindung jeweils einer Zeile der Tabelle A entspricht

### Tabelle 18

Verbindungen der Formel Ia, Ib, Ic, Id, Ie, If, Ig, Ih, Ii, Ij, Ik, IL, Im, In, Io, Ip, Iq, Ir, Is, It, lu, Iv, Iw und Ix, in denen Lₙ 2,3,4-Trifluor, R³ Methyl bedeuten und YR² für eine Verbindung jeweils einer Zeile der Tabelle A entspricht

### Tabelle 19

Verbindungen der Formel Ia, Ib, Ic, Id, Ie, If, Ig, Ih, Ii, Ij, Ik, IL, Im, In, Io, Ip, Iq, Ir, Is, It, lu, Iv, Iw und Ix, in denen Lₙ 2-Methyl, R³ Methyl bedeuten und YR² für eine Verbindung jeweils einer Zeile der Tabelle A entspricht

### Tabelle 20

Verbindungen der Formel Ia, Ib, Ic, Id, Ie, If, Ig, Ih, Ii, Ij, Ik, IL, Im, In, Io, Ip, Iq, Ir, Is, It, Iu, Iv, Iw und Ix, in denen Lₙ 2,4-Dimethyl, R³ Methyl bedeuten und YR² für eine Verbindung jeweils einer Zeile der Tabelle A entspricht

### Tabelle 21

Verbindungen der Formel Ia, Ib, Ic, Id, Ie, If, Ig, Ih, Ii, Ij, Ik, IL, Im, In, Io, Ip, Iq, Ir, Is, It, Iu, Iv, Iw und Ix, in denen Lₙ 2-Methyl-4-chlor, R³ Methyl bedeuten und YR² für eine Verbindung jeweils einer Zeile der Tabelle A entspricht

### Tabelle 22

Verbindungen der Formel Ia, Ib, Ic, Id, Ie, If, Ig, Ih, Ii, Ij, Ik, IL, Im, In, Io, Ip, Iq, Ir, Is, lt, lu, Iv, Iw und Ix, in denen Lₙ 2-Fluor-4-methyl, R³ Methyl bedeuten und YR² für eine Verbindung jeweils einer Zeile der Tabelle A entspricht

### Tabelle 23

Verbindungen der Formel Ia, Ib, Ic, Id, Ie, If, Ig, Ih, Ii, Ij, Ik, IL, Im, In, Io, Ip, Iq, Ir, Is, lt, lu, Iv, Iw und Ix, in denen Lₙ 2,6-Dimethyl, R³ Methyl bedeuten und YR² für eine Verbindung jeweils einer Zeile der Tabelle A entspricht

### Tabelle 24

Verbindungen der Formel Ia, Ib, Ic, Id, Ie, If, Ig, Ih, Ii, Ij, Ik, IL, Im, In, Io, Ip, Iq, Ir, Is, It, lu, Iv, Iw und Ix, in denen Lₙ 2,4,6-Trimethyl, R³ Methyl bedeuten und YR² für eine Verbindung jeweils einer Zeile der Tabelle A entspricht

### Tabelle 25

Verbindungen der Formel Ia, Ib, Ic, Id, Ie, If, Ig, Ih, Ii, Ij, Ik, IL, Im, In, Io, Ip, Iq, Ir, Is, It, lu, Iv, Iw und Ix, in denen Lₙ 2,6-Difluor-4-cyano, R³ Methyl bedeuten und YR² für eine Verbindung jeweils einer Zeile der Tabelle A entspricht

### Tabelle 26

Verbindungen der Formel Ia, Ib, Ic, Id, Ie, If, Ig, Ih, Ii, Ij, Ik, IL, Im, In, Io, Ip, Iq, Ir, Is, lt, lu, Iv, Iw und Ix, in denen Lₙ 2,6-Difluor-4-methyl, R³ Methyl bedeuten und YR² für eine Verbindung jeweils einer Zeile der Tabelle A entspricht

### Tabelle 27

Verbindungen der Formel Ia, Ib, Ic, Id, Ie, If, Ig, Ih, Ii, Ij, Ik, IL, Im, In, Io, Ip, Iq, Ir, Is, It, lu, Iv, Iw und Ix, in denen Lₙ 2,6-Difluor-4-methoxycarbonyl, R³ Methyl bedeuten und YR² für eine Verbindung jeweils einer Zeile der Tabelle A entspricht

### Tabelle 28

Verbindungen der Formel Ia, Ib, Ic, Id, Ie, If, Ig, Ih, Ii, Ij, Ik, IL, Im, In, Io, Ip, Iq, Ir, Is, lt, lu, Iv, Iw und Ix, in denen Lₙ 2-Chlor,4-Methoxy, R³ Methyl bedeuten und YR² für eine Verbindung jeweils einer Zeile der Tabelle A entspricht

### Tabelle 29

Verbindungen der Formel Ia, Ib, Ic, Id, Ie, If, Ig, Ih, Ii, Ij, Ik, IL, Im, In, Io, Ip, Iq, Ir, Is, It, lu, Iv, Iw und Ix, in denen Lₙ 2-Chlor,4-Methyl, R³ Methyl bedeuten und YR² für eine Verbindung jeweils einer Zeile der Tabelle A entspricht

### Tabelle 30

Verbindungen der Formel Ia, Ib, Ic, Id, Ie, If, Ig, Ih, Ii, Ij, Ik, IL, Im, In, Io, Ip, Iq, Ir, Is, It, lu, Iv, Iw und Ix, in denen Lₙ 2-Chlor,4-methoxycarbonyl, R³ Methyl bedeuten und YR² für eine Verbindung jeweils einer Zeile der Tabelle A entspricht

### Tabelle 31

Verbindungen der Formel Ia, Ib, Ic, Id, Ie, If, Ig, Ih, Ii, Ij, Ik, IL, Im, In, Io, Ip, Iq, Ir, Is, lt, Iu, Iv, Iw und Ix, in denen Lₙ 2-Chlor,4-Brom, R³ Methyl bedeuten und YR² für eine Verbindung jeweils einer Zeile der Tabelle A entspricht

### Tabelle 32

Verbindungen der Formel Ia, Ib, Ic, Id, Ie, If, Ig, Ih, Ii, Ij, Ik, IL, Im, In, Io, Ip, Iq, Ir, Is, It, Iu, Iv, Iw und Ix, in denen Lₙ 2-Chlor,4-Cyan, R³ Methyl bedeuten und YR² für eine Verbindung jeweils einer Zeile der Tabelle A entspricht

### Tabelle 33

Verbindungen der Formel Ia, Ib, Ic, Id, Ie, If, Ig, Ih, Ii, Ij, Ik, IL, Im, In, Io, Ip, Iq, Ir, Is, It, Iu, Iv, Iw und Ix, in denen Lₙ 2,6-Difluor,4-methoxy, R³ Methyl bedeuten und YR² für eine Verbindung jeweils einer Zeile der Tabelle A entspricht

### Tabelle 34

Verbindungen der Formel Ia, Ib, Ic, Id, Ie, If, Ig, Ih, Ii, Ij, Ik, IL, Im, In, Io, Ip, Iq, Ir, Is, It, Iu, Iv, Iw und Ix, in denen Lₙ 2-Fluor,3-methyl, R³ Methyl bedeuten und YR² für eine Verbindung jeweils einer Zeile der Tabelle A entspricht

### Tabelle 35

Verbindungen der Formel Ia, Ib, Ic, Id, Ie, If, Ig, Ih, Ii, Ij, Ik, IL, Im, In, Io, Ip, Iq, Ir, Is, It, lu, Iv, Iw und Ix, in denen Lₙ 2,5-Dimethyl, R³ Methyl bedeuten und YR² für eine Verbindung jeweils einer Zeile der Tabelle A entspricht

### Tabelle 36

Verbindungen der Formel Ia, Ib, Ic, Id, Ie, If, Ig, Ih, Ii, Ij, Ik, IL, Im, In, Io, Ip, Iq, Ir, Is, It, Iu, Iv, Iw und Ix, in denen Lₙ 2-Methyl,4-Cyan, R³ Methyl bedeuten und YR² für eine Verbindung jeweils einer Zeile der Tabelle A entspricht

### Tabelle 37

Verbindungen der Formel Ia, Ib, Ic, Id, Ie, If, Ig, Ih, Ii, Ij, Ik, IL, Im, In, Io, Ip, Iq, Ir, Is, It, Iu, Iv, Iw und Ix, in denen Lₙ 2-Methyl,4-brom, R³ Methyl bedeuten und YR² für eine Verbindung jeweils einer Zeile der Tabelle A entspricht

### Tabelle 38

Verbindungen der Formel Ia, Ib, Ic, Id, Ie, If, Ig, Ih, Ii, Ij, Ik, IL, Im, In, Io, Ip, Iq, Ir, Is, lt, Iu, Iv, Iw und Ix, in denen Lₙ 2-Methyl,5-fluor, R³ Methyl bedeuten und YR² für eine Verbindung jeweils einer Zeile der Tabelle A entspricht

### Tabelle 39

Verbindungen der Formel Ia, Ib, Ic, Id, Ie, If, Ig, Ih, Ii, Ij, Ik, IL, Im, In, Io, Ip, Iq, Ir, Is, lt, Iu, Iv, Iw und Ix, in denen Lₙ 2-Methyl,4-methoxy, R³ Methyl bedeuten und YR² für eine Verbindung jeweils einer Zeile der Tabelle A entspricht

### Tabelle 40

Verbindungen der Formel Ia, Ib, Ic, Id, Ie, If, Ig, Ih, Ii, Ij, Ik, IL, Im, In, Io, Ip, Iq, Ir, Is, It, lu, Iv, Iw und Ix, in denen Lₙ 2-Methyl,4-methoxycarbonyl, R³ Methyl bedeuten und YR² für eine Verbindung jeweils einer Zeile der Tabelle A entspricht

### Tabelle 41

Verbindungen der Formel Ia, Ib, Ic, Id, Ie, If, Ig, Ih, Ii, Ij, Ik, IL, Im, In, Io, Ip, Iq, Ir, Is, It, Iu, Iv, Iw und Ix, in denen Lₙ 2,5-Dimethyl,4-brom, R³ Methyl bedeuten und YR² für eine Verbindung jeweils einer Zeile der Tabelle A entspricht

### Tabelle 42

Verbindungen der Formel Ia, Ib, Ic, Id, Ie, If, Ig, Ih, Ii, Ij, Ik, IL, Im, In, Io, Ip, Iq, Ir, Is, It, lu, Iv, Iw und Ix, in denen Lₙ 2-Fluor, 4-brom, R³ Methyl bedeuten und YR² für eine Verbindung jeweils einer Zeile der Tabelle A entspricht

### Tabelle 43

Verbindungen der Formel Ia, Ib, Ic, Id, Ie, If, Ig, Ih, Ii, Ij, Ik, IL, Im, In, Io, Ip, Iq, Ir, Is, It, lu, Iv, Iw und Ix, in denen Lₙ 2-Fluor,4-methoxy, R³ Methyl bedeuten und YR² für eine Verbindung jeweils einer Zeile der Tabelle A entspricht

### Tabelle 44

Verbindungen der Formel Ia, Ib, Ic, Id, Ie, If, Ig, Ih, Ii, Ij, Ik, IL, Im, In, Io, Ip, Iq, Ir, Is, It, Iu, Iv, Iw und Ix, in denen Lₙ 2-Fluor,5-methyl, R³ Methyl bedeuten und YR² für eine Verbindung jeweils einer Zeile der Tabelle A entspricht

### Tabelle 45

Verbindungen der Formel Ia, Ib, Ic, Id, Ie, If, Ig, Ih, Ii, Ij, Ik, IL, Im, In, Io, Ip, Iq, Ir, Is, lt, Iu, Iv, Iw und Ix, in denen Lₙ Pentafluor, R³ Methyl bedeuten und YR² für eine Verbindung jeweils einer Zeile der Tabelle A entspricht

### Tabelle 46

Verbindungen der Formel Ia, Ib, Ic, Id, Ie, If, Ig, Ih, Ii, Ij, Ik, IL, Im, In, Io, Ip, Iq, Ir, Is, lt, lu, Iv, Iw und Ix, in denen Lₙ 2-Chlor,5-fluor,4-methoxy, R³ Methyl bedeuten und YR² für eine Verbindung jeweils einer Zeile der Tabelle A entspricht

### Tabelle 47

Verbindungen der Formel Ia, Ib, Ic, Id, Ie, If, Ig, Ih, Ii, Ij, Ik, IL, Im, In, Io, Ip, Iq, Ir, Is, It, lu, Iv, Iw und Ix, in denen Lₙ 2-Chlor,5-fluor, R³ Methyl bedeuten und YR² für eine Verbindung jeweils einer Zeile der Tabelle A entspricht

### Tabelle 48

Verbindungen der Formel Ia, Ib, Ic, Id, Ie, If, Ig, Ih, Ii, Ij, Ik, IL, Im, In, Io, Ip, Iq, Ir, Is, It, Iu, Iv, Iw und Ix, in denen Lₙ 2-Fluor,6-chlor, R³ Chlor bedeuten und YR² für eine Verbindung jeweils einer Zeile der Tabelle A entspricht

### Tabelle 49

Verbindungen der Formel Ia, Ib, Ic, Id, Ie, If, Ig, Ih, Ii, Ij, Ik, IL, Im, In, Io, Ip, Iq, Ir, Is, It, Iu, Iv, Iw und Ix, in denen Lₙ 2,6-Difluor, R³ Chlor bedeuten und YR² für eine Verbindung jeweils einer Zeile der Tabelle A entspricht

### Tabelle 50

Verbindungen der Formel Ia, Ib, Ic, Id, Ie, If, Ig, Ih, Ii, Ij, Ik, IL, Im, In, Io, Ip, Iq, Ir, Is, It, Iu, Iv, Iw und Ix, in denen Lₙ 2,6-Dichlor, R³ Chlor bedeuten und YR² für eine Verbindung jeweils einer Zeile der Tabelle A entspricht

### Tabelle 51

Verbindungen der Formel Ia, Ib, Ic, Id, Ie, If, Ig, Ih, Ii, Ij, Ik, IL, Im, In, Io, Ip, Iq, Ir, Is, lt, Iu, Iv, Iw und Ix, in denen Lₙ 2-Fluor,6-methyl, R³ Chlor bedeuten und YR² für eine Verbindung jeweils einer Zeile der Tabelle A entspricht

### Tabelle 52

Verbindungen der Formel Ia, Ib, Ic, Id, Ie, If, Ig, Ih, Ii, Ij, Ik, IL, Im, In, Io, Ip, Iq, Ir, Is, lt, lu, Iv, Iw und Ix, in denen Lₙ 2,4,6-Trifluor, R³ Chlor bedeuten und YR² für eine Verbindung jeweils einer Zeile der Tabelle A entspricht

### Tabelle 53

Verbindungen der Formel Ia, Ib, Ic, Id, Ie, If, Ig, Ih, Ii, Ij, Ik, IL, Im, In, Io, Ip, Iq, Ir, Is, It, lu, Iv, Iw und Ix, in denen Lₙ 2-Methyl,4-fluor, R³ Chlor bedeuten und YR² für eine Verbindung jeweils einer Zeile der Tabelle A entspricht

### Tabelle 54

Verbindungen der Formel Ia, Ib, Ic, Id, Ie, If, Ig, Ih, Ii, Ij, Ik, IL, Im, In, Io, Ip, Iq, Ir, Is, It, lu, Iv, Iw und Ix, in denen Lₙ 2-Fluor,4-methoxycarbonyl, R³ Chlor bedeuten und YR² für eine Verbindung jeweils einer Zeile der Tabelle A entspricht

### Tabelle 55

Verbindungen der Formel Ia, Ib, Ic, Id, Ie, If, Ig, Ih, Ii, Ij, Ik, IL, Im, In, Io, Ip, Iq, Ir, Is, It, lu, Iv, Iw und Ix, in denen Lₙ 2-Fluor,4-CN, R³ Chlor bedeuten und YR² für eine Verbindung jeweils einer Zeile der Tabelle A entspricht

### Tabelle 56

Verbindungen der Formel Ia, Ib, Ic, Id, Ie, If, Ig, Ih, Ii, Ij, Ik, IL, Im, In, Io, Ip, Iq, Ir, Is, lt, Iu, Iv, Iw und Ix, in denen Lₙ 2,4,5-Trifluor, R³ Chlor bedeuten und YR² für eine Verbindung jeweils einer Zeile der Tabelle A entspricht

### Tabelle 57

Verbindungen der Formel Ia, Ib, Ic, Id, le, If, Ig, Ih, li, Ij, Ik, IL, Im, In, Io, Ip, Iq, Ir, Is, It, lu, Iv, Iw und Ix, in denen Lₙ 2,4-Dichlor, R³ Chlor bedeuten und YR² für eine Verbindung jeweils einer Zeile der Tabelle A entspricht

### Tabelle 58

Verbindungen der Formel Ia, Ib, Ic, Id, le, If, Ig, Ih, li, Ij, Ik, IL, Im, In, Io, Ip, Iq, Ir, Is, It, lu, Iv, Iw und Ix, in denen Lₙ 2-Chlor, R³ Chlor bedeuten und YR² für eine Verbindung jeweils einer Zeile der Tabelle A entspricht

### Tabelle 59

Verbindungen der Formel Ia, Ib, Ic, Id, le, If, Ig, Ih, li, Ij, Ik, IL, Im, In, Io, Ip, Iq, Ir, Is, It, lu, Iv, Iw und Ix, in denen Lₙ 2-Fluor, R³ Chlor bedeuten und YR² für eine Verbindung jeweils einer Zeile der Tabelle A entspricht

### Tabelle 60

Verbindungen der Formel Ia, Ib, Ic, Id, le, If, Ig, Ih, Ii, Ij, Ik, IL, Im, In, Io, Ip, Iq, Ir, Is, It, Iu, Iv. Iw und Ix, in denen Lₙ 2,4-Difluor, R³ Chlor bedeuten und YR² für eine Verbindung jeweils einer Zeile der Tabelle A entspricht

### Tabelle 61

Verbindungen der Formel Ia, Ib, Ic, Id, le, If, Ig, Ih, li, Ij, Ik, IL, Im, In, Io, Ip, Iq, Ir, Is, It, Iu, Iv, Iw und Ix, in denen Lₙ 2-Fluor-4-chlor, R³ Chlor bedeuten und YR² für eine Verbindung jeweils einer Zeile der Tabelle A entspricht

### Tabelle 62

Verbindungen der Formel Ia, Ib, Ic, Id, le, If, Ig, Ih, Ii, Ij, Ik, IL, Im, In, Io, Ip, Iq, Ir, Is, It, lu, Iv, Iw und Ix, in denen Lₙ 2-Chlor-4-fluor, R³ Chlor bedeuten und YR² für eine Verbindung jeweils einer Zeile der Tabelle A entspricht

### Tabelle 63

Verbindungen der Formel Ia, Ib, Ic, Id, Ie, If, Ig, Ih, Ii, Ij, Ik, IL, Im, In, Io, Ip, Iq, Ir, Is, It, lu, Iv, Iw und Ix, in denen Lₙ 2,3-Difluor, R³ Chlor bedeuten und YR² für eine Verbindung jeweils einer Zeile der Tabelle A entspricht

### Tabelle 64

Verbindungen der Formel Ia, Ib, Ic, Id, le, If, Ig, Ih, li, Ij, Ik, IL, Im, In, Io, Ip, Iq, Ir, Is, It, lu, Iv, Iw und Ix, in denen Lₙ 2,5-Difluor, R³ Chlor bedeuten und YR² für eine Verbindung jeweils einer Zeile der Tabelle A entspricht

### Tabelle 65

Verbindungen der Formel Ia, Ib, Ic, Id, le, If, Ig, Ih, li, Ij, Ik, IL, Im, In, Io, Ip, Iq, Ir, Is, It, Iu, Iv, Iw und Ix, in denen Lₙ 2,3,4-Trifluor, R³ Chlor bedeuten und YR² für eine Verbindung jeweils einer Zeile der Tabelle A entspricht

### Tabelle 66

Verbindungen der Formel Ia, Ib, Ic, Id, le, If, Ig, Ih, Ii, Ij, Ik, IL, Im, In, Io, Ip, Iq, Ir, Is, It, lu, Iv, Iw und Ix, in denen Lₙ 2-Methyl, R³ Chlor bedeuten und YR² für eine Verbindung jeweils einer Zeile der Tabelle A entspricht

### Tabelle 67

Verbindungen der Formel Ia, Ib, Ic, Id, le, If, Ig, Ih, Ii, Ij, Ik, IL, Im, In, Io, Ip, Iq, Ir, Is, It, lu, Iv, Iw und Ix, in denen Lₙ 2,4-Dimethyl, R³ Chlor bedeuten und YR² für eine Verbindung jeweils einer Zeile der Tabelle A entspricht

### Tabelle 68

Verbindungen der Formel Ia, Ib, Ic, Id, le, If, Ig, Ih, Ii, Ij, Ik, IL, Im, In, Io, Ip, Iq, Ir, Is, It, lu, Iv, Iw und Ix, in denen Lₙ 2-Methyl-4-chlor, R³ Chlor bedeuten und YR² für eine Verbindung jeweils einer Zeile der Tabelle A entspricht

### Tabelle 69

Verbindungen der Formel Ia, Ib, Ic, Id, Ie, If, Ig, Ih, Ii, Ij, Ik, IL, Im, In, Io, Ip, Iq, Ir, Is, It, lu, Iv, Iw und Ix, in denen Lₙ 2-Fluor-4-methyl, R² Chlor bedeuten und YR² für eine Verbindung jeweils einer Zeile der Tabelle A entspricht

### Tabelle 70

Verbindungen der Formel Ia, Ib, Ic, Id, le, If, Ig, Ih, li, Ij, Ik, IL, Im, In, Io, Ip, Iq, Ir, Is, It, lu, Iv, Iw und Ix, in denen Lₙ 2,6-Dimethyl, R³ Chlor bedeuten und YR² für eine Verbindung jeweils einer Zeile der Tabelle A entspricht

### Tabelle 71

Verbindungen der Formel Ia, Ib, Ic, Id, le, If, Ig, Ih, Ii, Ij, Ik, IL, Im, In, Io, Ip, Iq, Ir, Is, It, lu, Iv, Iw und Ix, in denen Lₙ 2,4,6-Trimethyl, R³ Chlor bedeuten und YR² für eine Verbindung jeweils einer Zeile der Tabelle A entspricht

### Tabelle 72

Verbindungen der Formel Ia, Ib, Ic, Id, le, If, Ig, Ih, Ii, Ij, Ik, IL, Im, In, Io, Ip, Iq, Ir, Is, lt, lu, Iv, Iw und Ix, in denen Lₙ 2,6-Difluor-4-cyano, R³ Chlor bedeuten und YR² für eine Verbindung jeweils einer Zeile der Tabelle A entspricht

### Tabelle 73

Verbindungen der Formel Ia, Ib, Ic, Id, Ie, If, Ig, Ih, Ii, Ij, Ik, IL, Im, In, Io, Ip, Iq, Ir, Is, lt, lu, Iv, Iw und Ix, in denen Lₙ 2,6-Difluor-4-methyl, R³ Chlor bedeuten und YR² für eine Verbindung jeweils einer Zeile der Tabelle A entspricht

### Tabelle 74

Verbindungen der Formel Ia, Ib, Ic, Id, Ie, If, Ig, Ih, Ii, Ij, Ik, IL, Im, In, Io, Ip, Iq, Ir, Is, It, lu, Iv, Iw und Ix, in denen Lₙ 2,6-Difluor-4-methoxycarbonyl, R³ Chlor bedeuten und YR² für eine Verbindung jeweils einer Zeile der Tabelle A entspricht

### Tabelle 75

Verbindungen der Formel Ia, Ib, Ic, Id, Ie, If, Ig, Ih, Ii, Ij, Ik, IL, Im, In, Io, Ip, Iq, Ir, Is, It, Iu, Iv, Iw und Ix, in denen Lₙ 2-Chlor,4-Methoxy, R³ Chlor bedeuten und YR² für eine Verbindung jeweils einer Zeile der Tabelle A entspricht

### Tabelle 76

Verbindungen der Formel Ia, Ib, Ic, Id, Ie, If, Ig, Ih, Ii, Ij, Ik, IL, Im, In, Io, Ip, Iq, Ir, Is, lt, Iu, Iv, Iw und Ix, in denen Lₙ 2-Chlor,4-Methyl, R³ Chlor bedeuten und YR² für eine Verbindung jeweils einer Zeile der Tabelle A entspricht

### Tabelle 77

Verbindungen der Formel Ia, Ib, Ic, Id, Ie, If, Ig, Ih, Ii, Ij, Ik, IL, Im, In, Io, Ip, Iq, Ir, Is, lt, lu, Iv, Iw und Ix, in denen Lₙ 2-Chlor,4-methoxycarbonyl, R³ Chlor bedeuten und YR² für eine Verbindung jeweils einer Zeile der Tabelle A entspricht

### Tabelle 78

Verbindungen der Formel Ia, Ib, Ic, Id, Ie, If, Ig, Ih, Ii, Ij, Ik, IL, Im, In, Io, Ip, Iq, Ir, Is, lt, lu, Iv, Iw und Ix, in denen Lₙ 2-Chlor,4-Brom, R³ Chlor bedeuten und YR² für eine Verbindung jeweils einer Zeile der Tabelle A entspricht

### Tabelle 79

Verbindungen der Formel Ia, Ib, Ic, Id, Ie, If, Ig, Ih, Ii, Ij, Ik, IL, Im, In, Io, Ip, Iq, Ir, Is, It, Iu, Iv, Iw und Ix, in denen Lₙ 2-Chlor,4-Cyan, R³ Chlor bedeuten und YR² für eine Verbindung jeweils einer Zeile der Tabelle A entspricht

### Tabelle 80

Verbindungen der Formel Ia, Ib, Ic, Id, Ie, If, Ig, Ih, Ii, Ij, Ik, IL, Im, In, Io, Ip, Iq, Ir, Is, It, Iu, Iv, Iw und Ix, in denen Lₙ 2,6-Difluor,4-methoxy, R³ Chlor bedeuten und YR² für eine Verbindung jeweils einer Zeile der Tabelle A entspricht

### Tabelle 81

Verbindungen der Formel Ia, Ib, Ic, Id, le, If, Ig, Ih, li, Ij, Ik, IL, Im, In, Io, Ip, Iq, Ir, Is, It, lu, Iv, Iw und Ix, in denen Lₙ 2-Fluor,3-methyl, R³ Chlor bedeuten und YR² für eine Verbindung jeweils einer Zeile der Tabelle A entspricht

### Tabelle 82

Verbindungen der Formel Ia, Ib, Ic, Id, le, If, Ig, Ih, li, Ij, Ik, IL, Im, In, Io, Ip, Iq, Ir, Is, lt, lu, Iv, Iw und Ix, in denen Lₙ 2,5-Dimethyl, R³ Chlor bedeuten und YR² für eine Verbindung jeweils einer Zeile der Tabelle A entspricht

### Tabelle 83

Verbindungen der Formel Ia, Ib, Ic, Id, le, If, Ig, Ih, Ii, Ij, Ik, IL, Im, In, Io, Ip, Iq, Ir, Is, It, lu, Iv, Iw und Ix, in denen Lₙ 2-Methyl,4-cyan, R³ Chlor bedeuten und YR² für eine Verbindung jeweils einer Zeile der Tabelle A entspricht

### Tabelle 84

Verbindungen der Formel Ia, Ib, Ic, Id, Ie, If, Ig, Ih, Ii, Ij, Ik, IL, Im, In, Io, Ip, Iq, Ir, Is, It, lu, Iv, Iw und Ix, in denen Lₙ 2-Methyl,4-brom, R³ Chlor bedeuten und YR² für eine Verbindung jeweils einer Zeile der Tabelle A entspricht

### Tabelle 85

Verbindungen der Formel Ia, Ib, Ic, Id, le, If, Ig, Ih, li, Ij, Ik, IL, Im, In, Io, Ip, Iq, Ir, Is, It, lu, Iv, Iw und Ix, in denen Lₙ 2-Methyl,5-fluor, R³ Chlor bedeuten und YR² für eine Verbindung jeweils einer Zeile der Tabelle A entspricht

### Tabelle 86

Verbindungen der Formel Ia, Ib, Ic, Id, Ie, If, Ig, Ih, li, Ij, Ik, IL, Im, In, Io, Ip, Iq, Ir, Is, It, lu, Iv, Iw und Ix, in denen Lₙ 2-Methyl,4-methoxy, R³ Chlor bedeuten und YR² für eine Verbindung jeweils einer Zeile der Tabelle A entspricht

### Tabelle 87

Verbindungen der Formel Ia, Ib, Ic, Id, Ie, If, Ig, Ih, li, Ij, Ik, IL, Im, In, Io, Ip, Iq, Ir, Is, lt, lu, Iv, Iw und Ix, in denen Lₙ 2-Methyl,4-methoxycarbonyl, R³ Chlor bedeuten und YR² für eine Verbindung jeweils einer Zeile der Tabelle A entspricht

### Tabelle 88

Verbindungen der Formel Ia, Ib, Ic, Id, le, If, Ig, Ih, Ii, Ij, Ik, IL, Im, In, Io, Ip, Iq, Ir, Is, It, lu, Iv, Iw und Ix, in denen Lₙ 2,5-Dimethyl,4-brom, R³ Chlor bedeuten und YR² für eine Verbindung jeweils einer Zeile der Tabelle A entspricht

### Tabelle 89

Verbindungen der Formel Ia, Ib, Ic, Id, le, If, Ig, Ih, li, Ij, Ik, IL, Im, In, Io, Ip, Iq, Ir, Is, It, lu, Iv, Iw und Ix, in denen Lₙ 2-Fluor,4-brom, R³ Chlor bedeuten und YR² für eine Verbindung jeweils einer Zeile der Tabelle A entspricht

### Tabelle 90

Verbindungen der Formel Ia, Ib, Ic, Id, le, If, Ig, Ih, Ii, Ij, Ik, IL, Im, In, Io, Ip, Iq, Ir, Is, It, Iu, Iv, Iw und Ix, in denen Lₙ 2-Fluor,4-methoxy, R³ Chlor bedeuten und YR² für eine Verbindung jeweils einer Zeile der Tabelle A entspricht

### Tabelle 91

Verbindungen der Formel Ia, Ib, Ic, Id, le, If, Ig, Ih, li, Ij, Ik, IL, Im, In, Io, Ip, Iq, Ir, Is, It, Iu, Iv, Iw und Ix, in denen Lₙ 2-Fluor,5-methyl, R³ Chlor bedeuten und YR² für eine Verbindung jeweils einer Zeile der Tabelle A entspricht

### Tabelle 92

Verbindungen der Formel Ia, Ib, Ic, Id, Ie, If, Ig, Ih, Ii, Ij, Ik, IL, Im, In, Io, Ip, Iq, Ir, Is, It, Iu, Iv, Iw und Ix, in denen Lₙ Pentafluor, R³ Chlor bedeuten und YR² für eine Verbindung jeweils einer Zeile der Tabelle A entspricht

### Tabelle 93

Verbindungen der Formel Ia, Ib, Ic, Id, le, If, Ig, Ih, li, Ij, Ik, IL, Im, In, Io, Ip, Iq, Ir, Is, It, Iu, Iv, Iw und Ix, in denen Lₙ 2-Chlor,5-fluor,4-methoxy, R³ Chlor bedeuten und YR² für eine Verbindung jeweils einer Zeile der Tabelle A entspricht

### Tabelle 94

Verbindungen der Formel Ia, Ib, Ic, Id, le, If, Ig, Ih, li, Ij, Ik, IL, Im, In, Io, Ip, Iq, Ir, Is, It, lu, Iv, Iw und Ix, in denen Lₙ 2-Chlor,5-fluor, R³ Chlor bedeuten und YR² für eine Verbindung jeweils einer Zeile der Tabelle A entspricht

### Tabelle 95

Verbindungen der Formel Ia, Ib, Ic, Id, le, If, Ig, Ih, li, Ij, Ik, IL, Im, In, Io, Ip, Iq, Ir, Is, It, Iu, Iv, Iw und Ix, in denen Lₙ 2-Fluor,6-chlor, R³ Methoxy bedeuten und YR² für eine Verbindung jeweils einer Zeile der Tabelle A entspricht

### Tabelle 96

Verbindungen der Formel Ia, Ib, Ic, Id, le, If, Ig, Ih, li, Ij, Ik, IL, Im, In, Io, Ip, Iq, Ir, Is, It, lu, Iv, Iw und Ix, in denen Lₙ 2,6-Difluor, R³ Methoxy bedeuten und YR² für eine Verbindung jeweils einer Zeile der Tabelle A entspricht

### Tabelle 97

Verbindungen der Formel Ia, Ib, Ic, Id, Ie, If, Ig, Ih, Ii, Ij, Ik, IL, Im, In, Io, Ip, Iq, Ir, Is, It, lu, Iv, Iw und Ix, in denen Lₙ 2,6-Dichlor, R³ Methoxy bedeuten und YR² für eine Verbindung jeweils einer Zeile der Tabelle A entspricht

### Tabelle 98

Verbindungen der Formel Ia, Ib, Ic, Id, Ie, If, Ig, Ih, Ii, Ij, Ik, IL, Im, In, Io, Ip, Iq, Ir, Is, It, lu, Iv, Iw und Ix, in denen Lₙ 2-Fluor,6-methyl, R³ Methoxy bedeuten und YR² für eine Verbindung jeweils einer Zeile der Tabelle A entspricht

### Tabelle 99

Verbindungen der Formel Ia, Ib, Ic, Id, Ie, If, Ig, Ih, Ii, Ij, Ik, IL, Im, In, Io, Ip, Iq, Ir, Is, It, Iu, Iv, Iw und Ix, in denen Lₙ 2,4,6-Trifluor, R³ Methoxy bedeuten und YR² für eine Verbindung jeweils einer Zeile der Tabelle A entspricht

### Tabelle 100

Verbindungen der Formel Ia, Ib, Ic, Id, Ie, If, Ig, Ih, Ii, Ij, Ik, IL, Im, In, Io, Ip, Iq, Ir, Is, lt, Iu, Iv, Iw und Ix, in denen Lₙ 2-Methyl,4-fluor, R³ Methoxy bedeuten und YR² für eine Verbindung jeweils einer Zeile der Tabelle A entspricht

### Tabelle 101

Verbindungen der Formel Ia, Ib, Ic, Id, Ie, If, Ig, Ih, Ii, Ij, Ik, IL, Im, In, Io, Ip, Iq, Ir, Is, It, Iu, Iv, Iw und Ix, in denen Lₙ 2-Fluor,4-methoxycarbonyl, R³ Methoxy bedeuten und YR² für eine Verbindung jeweils einer Zeile der Tabelle A entspricht

### Tabelle 102

Verbindungen der Formel Ia, Ib, Ic, Id, Ie, If, Ig, Ih, Ii, Ij, Ik, IL, Im, In, Io, Ip, Iq, Ir, Is, It, lu, Iv, Iw und Ix, in denen Lₙ 2-Fluor,4-CN, R³ Methoxy bedeuten und YR² für eine Verbindung jeweils einer Zeile der Tabelle A entspricht

### Tabelle 103

Verbindungen der Formel Ia, Ib, Ic, Id, Ie, If, Ig, Ih, Ii, Ij, Ik, IL, Im, In, Io, Ip, Iq, Ir, Is, It, lu, Iv, Iw und Ix, in denen Lₙ 2,4,5-Trifluor, R³ Methoxy bedeuten und YR² für eine Verbindung jeweils einer Zeile der Tabelle A entspricht

### Tabelle 104

Verbindungen der Formel Ia, Ib, Ic, Id, Ie, If, Ig, Ih, Ii, Ij, Ik, IL, Im, In, Io, Ip, Iq, Ir, Is, lt, lu, Iv, Iw und Ix, in denen Lₙ 2,4-Dichlor, R³ Methoxy bedeuten und YR² für eine Verbindung jeweils einer Zeile der Tabelle A entspricht

### Tabelle 105

Verbindungen der Formel Ia, Ib, Ic, Id, Ie, If, Ig, Ih, Ii, Ij, Ik, IL, Im, In, Io, Ip, Iq, Ir, Is, It, Iu, Iv, Iw und Ix, in denen Lₙ 2-Chlor, R³ Methoxy bedeuten und YR² für eine Verbindung jeweils einer Zeile der Tabelle A entspricht

### Tabelle 106

Verbindungen der Formel Ia, Ib, Ic, Id, Ie, If, Ig, Ih, Ii, Ij, Ik, IL, Im, In, Io, Ip, Iq, Ir, Is, It, lu, Iv, Iw und Ix, in denen Lₙ 2-Fluor, R³ Methoxy bedeuten und YR² für eine Verbindung jeweils einer Zeile der Tabelle A entspricht

### Tabelle 107

Verbindungen der Formel Ia, Ib, Ic, Id, Ie, If, Ig, Ih, Ii, Ij, Ik, IL, Im, In, Io, Ip, Iq, Ir, Is, lt, lu, Iv, Iw und Ix, in denen Lₙ 2,4-Difluor, R³ Methoxy bedeuten und YR² für eine Verbindung jeweils einer Zeile der Tabelle A entspricht

### Tabelle 108

Verbindungen der Formel Ia, Ib, Ic, Id, Ie, If, Ig, Ih, Ii, Ij, Ik, IL, Im, In, Io, Ip, Iq, Ir, Is, It, Iu, Iv, Iw und Ix, in denen Lₙ 2-Fluor-4-chlor, R³ Methoxy bedeuten und YR² für eine Verbindung jeweils einer Zeile der Tabelle A entspricht

### Tabelle 109

Verbindungen der Formel Ia, Ib, Ic, Id, Ie, If, Ig, Ih, Ii, Ij, Ik, IL, Im, In, Io, Ip, Iq, Ir, Is, It, Iu, Iv, Iw und Ix, in denen Lₙ 2-Chlor-4-fluor, R³ Methoxy bedeuten und YR² für eine Verbindung jeweils einer Zeile der Tabelle A entspricht

### Tabelle 110

Verbindungen der Formel Ia, Ib, Ic, Id, Ie, If, Ig, Ih, Ii, Ij, Ik, IL, Im, In, Io, Ip, Iq, Ir, Is, It, Iu, Iv, Iw und Ix, in denen Lₙ 2,3-Difluor, R³ Methoxy bedeuten und YR² für eine Verbindung jeweils einer Zeile der Tabelle A entspricht

### Tabelle 111

Verbindungen der Formel Ia, Ib, Ic, Id, Ie, If, Ig, Ih, Ii, Ij, Ik, IL, Im, In, Io, Ip, Iq, Ir, Is, It, lu, Iv, Iw und Ix, in denen Lₙ 2,5-Difluor, R³ Methoxy bedeuten und YR² für eine Verbindung jeweils einer Zeile der Tabelle A entspricht

### Tabelle 112

Verbindungen der Formel Ia, Ib, Ic, Id, Ie, If, Ig, Ih, Ii, Ij, Ik, IL, Im, In, Io, Ip, Iq, Ir, Is, It, lu, Iv, Iw und Ix, in denen Lₙ 2,3,4-Trifluor, R³ Methoxy bedeuten und YR² für eine Verbindung jeweils einer Zeile der Tabelle A entspricht

### Tabelle 113

Verbindungen der Formel Ia, Ib, Ic, Id, Ie, If, Ig, Ih, Ii, Ij, Ik, IL, Im, In, Io, Ip, Iq, Ir, Is, lt, Iu, Iv, Iw und Ix, in denen Lₙ 2-Methyl, R³ Methoxy bedeuten und YR² für eine Verbindung jeweils einer Zeile der Tabelle A entspricht

### Tabelle 114

Verbindungen der Formel Ia, Ib, Ic, Id, Ie, If, Ig, Ih, Ii, Ij, Ik, IL, Im, In, Io, Ip, Iq, Ir, Is, It, lu, Iv, Iw und Ix, in denen Lₙ 2,4-Dimethyl, R³ Methoxy bedeuten und YR² für eine Verbindung jeweils einer Zeile der Tabelle A entspricht

### Tabelle 115

Verbindungen der Formel Ia, Ib, Ic, Id, Ie, If, Ig, Ih, Ii, Ij, Ik, IL, Im, In, Io, Ip, Iq, Ir, Is, It, Iu, Iv, Iw und Ix, in denen Lₙ 2-Methyl-4-chlor, R³ Methoxy bedeuten und YR² für eine Verbindung jeweils einer Zeile der Tabelle A entspricht

### Tabelle 116

Verbindungen der Formel Ia, Ib, Ic, Id, Ie, If, Ig, Ih, Ii, Ij, Ik, IL, Im, In, Io, Ip, Iq, Ir, Is, lt, Iu, Iv, Iw und Ix, in denen Lₙ 2-Fluor-4-methyl, R³ Methoxy bedeuten und YR² für eine Verbindung jeweils einer Zeile der Tabelle A entspricht

### Tabelle 117

Verbindungen der Formel Ia, Ib, Ic, Id, Ie, If, Ig, Ih, Ii, Ij, Ik, IL, Im, In, Io, Ip, Iq, Ir, Is, It, Iu, Iv, Iw und Ix, in denen Lₙ 2,6-Dimethyl, R³ Methoxy bedeuten und YR² für eine Verbindung jeweils einer Zeile der Tabelle A entspricht

### Tabelle 118

Verbindungen der Formel Ia, Ib, Ic, Id, Ie, If, Ig, Ih, Ii, Ij, Ik, IL, Im, In, Io, Ip, Iq, Ir, Is, It, lu, Iv, Iw und Ix, in denen Lₙ 2,4,6-Trimethyl, R³ Methoxy bedeuten und YR² für eine Verbindung jeweils einer Zeile der Tabelle A entspricht

### Tabelle 119

Verbindungen der Formel Ia, Ib, Ic, Id, Ie, If, Ig, Ih, Ii, Ij, Ik, IL, Im, In, Io, Ip, Iq, Ir, Is, It, Iu, Iv, Iw und Ix, in denen Lₙ 2,6-Difluor-4-cyano, R³ Methoxy bedeuten und YR² für eine Verbindung jeweils einer Zeile der Tabelle A entspricht

### Tabelle 120

Verbindungen der Formel Ia, Ib, Ic, Id, Ie, If, Ig, Ih, Ii, Ij, Ik, IL, Im, In, Io, Ip, Iq, Ir, Is, It, lu, Iv, Iw und Ix, in denen Lₙ 2,6-Difluor-4-methyl, R³ Methoxy bedeuten und YR² für eine Verbindung jeweils einer Zeile der Tabelle A entspricht

### Tabelle 121

Verbindungen der Formel Ia, Ib, Ic, Id, Ie, If, Ig, Ih, Ii, Ij, Ik, IL, Im, In, Io, Ip, Iq, Ir, Is, It, lu, Iv, Iw und Ix, in denen Lₙ 2,6-Difluor-4-Methoxycarbonyl, R³ Methoxy bedeuten und YR² für eine Verbindung jeweils einer Zeile der Tabelle A entspricht

### Tabelle 122

Verbindungen der Formel Ia, Ib, Ic, Id, Ie, If, Ig, Ih, Ii, Ij, Ik, IL, Im, In, Io, Ip, Iq, Ir, Is, It, lu, Iv, Iw und Ix, in denen Lₙ 2-Chlor,4-Methoxy, R³ Methoxy bedeuten und YR² für eine Verbindung jeweils einer Zeile der Tabelle A entspricht

### Tabelle 123

Verbindungen der Formel Ia, Ib, Ic, Id, Ie, If, Ig, Ih, Ii, Ij, Ik, IL, Im, In, Io, Ip, Iq, Ir, Is, It, lu, Iv, Iw und Ix, in denen Lₙ 2-Chlor,4-Methyl, R³ Methoxy bedeuten und YR² für eine Verbindung jeweils einer Zeile der Tabelle A entspricht

### Tabelle 124

Verbindungen der Formel Ia, Ib, Ic, Id, Ie, If, Ig, Ih, Ii, Ij, Ik, IL, Im, In, Io, Ip, Iq, Ir, Is, It, lu, Iv, Iw und Ix, in denen Lₙ 2-Chlor, 4-methoxycarbonyl, R³ Methoxy bedeuten und YR² für eine Verbindung jeweils einer Zeile der Tabelle A entspricht

### Tabelle 125

Verbindungen der Formel Ia, Ib, Ic, Id, Ie, If, Ig, Ih, Ii, Ij, Ik, IL, Im, In, Io, Ip, Iq, Ir, Is, It, lu, Iv, Iw und Ix, in denen Lₙ 2-Chlor,4-Methoxy, R³ Methoxy bedeuten und YR² für eine Verbindung jeweils einer Zeile der Tabelle A entspricht

### Tabelle 126

Verbindungen der Formel Ia, Ib, Ic, Id, Ie, If, Ig, Ih, Ii, Ij, Ik, IL, Im, In, lo, Ip, Iq, Ir, Is, lt, lu, Iv, Iw und Ix, in denen Lₙ 2-Chlor,4-Cyan, R³ Methoxy bedeuten und YR² für eine Verbindung jeweils einer Zeile der Tabelle A entspricht

### Tabelle 127

Verbindungen der Formel Ia, Ib, Ic, Id, Ie, If, Ig, Ih, Ii, Ij, Ik, IL, Im, In, Io, Ip, Iq, Ir, Is, It, lu, Iv, Iw und Ix, in denen Lₙ 2,6-Difluor, 4-methoxy, R³ Methoxy bedeuten und YR² für eine Verbindung jeweils einer Zeile der Tabelle A entspricht

### Tabelle 128

Verbindungen der Formel Ia, Ib, Ic, Id, Ie, If, Ig, Ih, Ii, Ij, Ik, IL, Im, In, Io, Ip, Iq, Ir, Is, It, lu, Iv, Iw und Ix, in denen Lₙ 2-Fluor,3-methyl, R³ Methoxy bedeuten und YR² für eine Verbindung jeweils einer Zeile der Tabelle A entspricht

### Tabelle 129

Verbindungen der Formel Ia, Ib, Ic, Id, Ie, If, Ig, Ih, Ii, Ij, Ik, IL, Im, In, Io, Ip, Iq, Ir, Is, It, Iu, Iv, Iw und Ix, in denen Lₙ 2,5-Dimethyl, R³ Methoxy bedeuten und YR² für eine Verbindung jeweils einer Zeile der Tabelle A entspricht

### Tabelle 130

Verbindungen der Formel Ia, Ib, Ic, Id, Ie, If, Ig, Ih, Ii, Ij, Ik, IL, Im, In, Io, Ip, Iq, Ir, Is, It, lu, Iv, Iw und Ix, in denen Lₙ 2-Methyl,4-Cyan, R³ Methoxy bedeuten und YR² für eine Verbindung jeweils einer Zeile der Tabelle A entspricht

### Tabelle 131

Verbindungen der Formel Ia, Ib, Ic, Id, Ie, If, Ig, Ih, Ii, Ij, Ik, IL, Im, In, Io, Ip, Iq, Ir, Is, lt, lu, Iv, Iw und Ix, in denen Lₙ 2-Methyl,4-Brom, R³ Methoxy bedeuten und YR² für eine Verbindung jeweils einer Zeile der Tabelle A entspricht

### Tabelle 132

Verbindungen der Formel Ia, Ib, Ic, Id, Ie, If, Ig, Ih, Ii, Ij, Ik, IL, Im, In, Io, Ip, Iq, Ir, Is, It, Iu, Iv, Iw und Ix, in denen Lₙ 2-Methyl,5-fluor, R³ Methoxy bedeuten und YR² für eine Verbindung jeweils einer Zeile der Tabelle A entspricht

### Tabelle 133

Verbindungen der Formel Ia, Ib, Ic, Id, Ie, If, Ig, Ih, Ii, Ij, Ik, IL, Im, In, Io, Ip, Iq, Ir, Is, It, Iu, Iv, Iw und Ix, in denen Lₙ 2-Methyl,4-methoxy, R³ Methoxy bedeuten und YR² für eine Verbindung jeweils einer Zeile der Tabelle A entspricht

### Tabelle 134

Verbindungen der Formel Ia, Ib, Ic, Id, Ie, If, Ig, Ih, Ii, Ij, Ik, IL, Im, In, Io, Ip, Iq, Ir, Is, lt, Iu, Iv, Iw und Ix, in denen Lₙ 2-Methyl,4-methoxycarbonyl, R³ Methoxy bedeuten und YR² für eine Verbindung jeweils einer Zeile der Tabelle A entspricht

### Tabelle 135

Verbindungen der Formel Ia, Ib, Ic, Id, Ie, If, Ig, Ih, Ii, Ij, Ik, IL, Im, In, Io, Ip, Iq, Ir, Is, It, Iu, Iv, Iw und Ix, in denen Lₙ 2,5-Dimethyl,4-brom, R³ Methoxy bedeuten und YR² für eine Verbindung jeweils einer Zeile der Tabelle A entspricht

### Tabelle 136

Verbindungen der Formel Ia, Ib, Ic, Id, Ie, If, Ig, Ih, Ii, Ij, Ik, IL, Im, In, Io, Ip, Iq, Ir, Is, lt, lu, Iv, Iw und Ix, in denen Lₙ 2-Fluor,4-brom, R³ Methoxy bedeuten und YR² für eine Verbindung jeweils einer Zeile der Tabelle A entspricht

### Tabelle 137

Verbindungen der Formel Ia, Ib, Ic, Id, le, If, Ig, Ih, Ii, Ij, Ik, IL, Im, In, Io, Ip, Iq, Ir, Is, lt, lu, Iv, Iw und Ix, in denen Lₙ 2-Fluor,4-methoxy, R³ Methoxy bedeuten und YR² für eine Verbindung jeweils einer Zeile der Tabelle A entspricht

### Tabelle 138

Verbindungen der Formel Ia, Ib, Ic, Id, le, If, Ig, Ih, li, Ij, Ik, IL, Im, In, Io, Ip, Iq, Ir, Is, It, lu, Iv, Iw und Ix, in denen Lₙ 2-Fluor,5-methyl, R³ Methoxy bedeuten und YR² für eine Verbindung jeweils einer Zeile der Tabelle A entspricht

### Tabelle 139

Verbindungen der Formel Ia, Ib, Ic, Id, le, If, Ig, Ih, Ii, Ij, Ik, IL, Im, In, Io, Ip, Iq, Ir, Is, lt, lu, Iv, Iw und Ix, in denen Lₙ Pentafluor, R³ Methoxy bedeuten und YR² für eine Verbindung jeweils einer Zeile der Tabelle A entspricht

### Tabelle 140

Verbindungen der Formel la, Ib, Ic, Id, le, If, Ig, Ih, li, Ij, Ik, IL, Im, In, Io, Ip, Iq, Ir, Is, It, lu, Iv, Iw und Ix, in denen Lₙ 2-Chlor,5-fluor,4-methoxy, R³ Methoxy bedeuten und YR² für eine Verbindung jeweils einer Zeile der Tabelle A entspricht

### Tabelle 141

Verbindungen der Formel Ia, Ib, Ic, Id, le, If, Ig, Ih, li, Ij, Ik, IL, Im, In, Io, Ip, Iq, Ir, Is, lt, Iu, Iv, Iw und Ix, in denen Lₙ 2-Chlor,5-fluor, R³ Methoxy bedeuten und YR² für eine Verbindung jeweils einer Zeile der Tabelle A entspricht

### Tabelle 142

Verbindungen der Formel la, Ib, Ic, Id, Ie, If, Ig, Ih, Ii, Ij, Ik, IL, Im, In, Io, Ip, Iq, Ir, Is, It, lu, Iv, Iw und Ix, in denen Lₙ 2-Fluor,6-chlor, R³ Cyano bedeuten und YR² für eine Verbindung jeweils einer Zeile der Tabelle A entspricht

### Tabelle 143

Verbindungen der Formel Ia, Ib, Ic, Id, le, If, Ig, Ih, li, Ij, Ik, IL, Im, In, Io, Ip, Iq, Ir, Is, It, lu, Iv, Iw und Ix, in denen Lₙ 2,6-Difluor, R³ Cyano bedeuten und YR² für eine Verbindung jeweils einer Zeile der Tabelle A entspricht

### Tabelle 144

Verbindungen der Formel Ia, Ib, Ic, Id, le, If, Ig, Ih, Ii, Ij, Ik, IL, Im, In, Io, Ip, Iq, Ir, Is, lt, lu, Iv, Iw und Ix, in denen Lₙ 2,6-Dichlor, R³ Cyano bedeuten und YR² für eine Verbindung jeweils einer Zeile der Tabelle A entspricht

### Tabelle 145

Verbindungen der Formel Ia, Ib, Ic, Id, Ie, If, Ig, Ih, Ii, Ij, Ik, IL, Im, In, Io, Ip, Iq, Ir, Is, It, Iu, Iv, Iw und Ix, in denen Lₙ 2-Fluor,6-methyl, R³ Cyano bedeuten und YR² für eine Verbindung jeweils einer Zeile der Tabelle A entspricht

### Tabelle 146

Verbindungen der Formel Ia, Ib, Ic, Id, Ie, If, Ig, Ih, Ii, Ij, Ik, IL, Im, In, Io, Ip, Iq, Ir, Is, It, Iu, Iv, Iw und Ix, in denen Lₙ 2,4,6-Trifluor, R³ Cyano bedeuten und YR² für eine Verbindung jeweils einer Zeile der Tabelle A entspricht

### Tabelle 147

Verbindungen der Formel Ia, Ib, Ic, Id, Ie, If, Ig, Ih, Ii, Ij, Ik, IL, Im, In, Io, Ip, Iq, Ir, Is, It, lu, Iv, Iw und Ix, in denen Lₙ 2-Methyl,4-fluor, R³ Cyano bedeuten und YR² für eine Verbindung jeweils einer Zeile der Tabelle A entspricht

### Tabelle 148

Verbindungen der Formel Ia, Ib, Ic, Id, Ie, If, Ig, Ih, Ii, Ij, Ik, IL, Im, In, Io, Ip, Iq, Ir, Is, It, lu, Iv, Iw und Ix, in denen Lₙ 2-Fluor,4-methoxycarbonyl, R³ Cyano bedeuten und YR² für eine Verbindung jeweils einer Zeile der Tabelle A entspricht

### Tabelle 149

Verbindungen der Formel Ia, Ib, Ic, Id, Ie, If, Ig, Ih, Ii, Ij, Ik, IL, Im, In, Io, Ip, Iq, Ir, Is, It, Iu, Iv, Iw und Ix, in denen Lₙ 2-Fluor,4-CN, R³ Cyano bedeuten und YR² für eine Verbindung jeweils einer Zeile der Tabelle A entspricht

### Tabelle 150

Verbindungen der Formel Ia, Ib, Ic, Id, Ie, If, Ig, Ih, Ii, Ij, Ik, IL, Im, In, Io, Ip, Iq, Ir, Is, lt, lu, Iv, Iw und Ix, in denen Lₙ 2,4,5-Trifluor, R³ Cyano bedeuten und YR² für eine Verbindung jeweils einer Zeile der Tabelle A entspricht

### Tabelle 151

Verbindungen der Formel Ia, Ib, Ic, Id, Ie, If, Ig, Ih, Ii, Ij, Ik, IL, Im, In, Io, Ip, Iq, Ir, Is, It, lu, Iv, Iw und Ix, in denen Lₙ 2,4-Dichlor, R³ Cyano bedeuten und YR² für eine Verbindung jeweils einer Zeile der Tabelle A entspricht

### Tabelle 152

Verbindungen der Formel Ia, Ib, Ic, Id, Ie, If, Ig, Ih, Ii, Ij, Ik, IL, Im, In, Io, Ip, Iq, Ir, Is, It, lu, Iv, Iw und Ix, in denen Lₙ 2-Chlor, R³ Cyano bedeuten und YR² für eine Verbindung jeweils einer Zeile der Tabelle A entspricht

### Tabelle 153

Verbindungen der Formel Ia, Ib, Ic, Id, Ie, If, Ig, Ih, Ii, Ij, Ik, IL, Im, In, Io, Ip, Iq, Ir, Is, It, Iu, Iv, Iw und Ix, in denen Lₙ 2-Fluor, R³ Cyano bedeuten und YR² für eine Verbindung jeweils einer Zeile der Tabelle A entspricht

### Tabelle 154

Verbindungen der Formel Ia, Ib, Ic, Id, Ie, If, Ig, Ih, Ii, Ij, Ik, IL, Im, In, Io, Ip, Iq, Ir, Is, lt, Iu, Iv, Iw und Ix, in denen Lₙ 2,4-Difluor, R³ Cyano bedeuten und YR² für eine Verbindung jeweils einer Zeile der Tabelle A entspricht

### Tabelle 155

Verbindungen der Formel Ia, Ib, Ic, Id, Ie, If, Ig, Ih, Ii, Ij, Ik, IL, Im, In, Io, Ip, Iq, Ir, Is, It, lu, Iv, Iw und Ix, in denen Lₙ 2-Fluor-4-chlor, R³ Cyano bedeuten und YR² für eine Verbindung jeweils einer Zeile der Tabelle A entspricht

### Tabelle 156

Verbindungen der Formel Ia, Ib, Ic, Id, Ie, If, Ig, Ih, Ii, Ij, Ik, IL, Im, In, Io, Ip, Iq, Ir, Is, It, Iu, Iv, Iw und Ix, in denen Lₙ 2-Chlor-4-fluor, R³ Cyano bedeuten und YR² für eine Verbindung jeweils einer Zeile der Tabelle A entspricht

### Tabelle 157 .

Verbindungen der Formel Ia, Ib, Ic, Id, Ie, If, Ig, Ih, Ii, Ij, Ik, IL, Im, In, Io, Ip, Iq, Ir, Is, lt, Iu, Iv, Iw und Ix, in denen Lₙ 2,3-Difluor, R³ Cyano bedeuten und YR² für eine Verbindung jeweils einer Zeile der Tabelle A entspricht

### Tabelle 158

Verbindungen der Formel Ia, Ib, Ic, Id, Ie, If, Ig, Ih, Ii, Ij, Ik, IL, Im, In, Io, Ip, Iq, Ir, Is, It, lu, Iv, Iw und Ix, in denen Lₙ 2,5-Difluor, R³ Cyano bedeuten und YR² für eine Verbindung jeweils einer Zeile der Tabelle A entspricht

### Tabelle 159

Verbindungen der Formel Ia, Ib, Ic, Id, Ie, If, Ig, Ih, Ii, Ij, Ik, IL, Im, In, Io, Ip, Iq, Ir, Is, lt, Iu, Iv, Iw und Ix, in denen Lₙ 2,3,4-Trifluor, R³ Cyano bedeuten und YR² für eine Verbindung jeweils einer Zeile der Tabelle A entspricht

### Tabelle 160

Verbindungen der Formel Ia, Ib, Ic, Id, Ie, If, Ig, Ih, Ii, Ij, Ik, IL, Im, In, Io, Ip, Iq, Ir, Is, It, Iu, Iv, Iw und Ix, in denen Lₙ 2-Methyl, R³ Cyano bedeuten und YR² für eine Verbindung jeweils einer Zeile der Tabelle A entspricht

### Tabelle 161

Verbindungen der Formel Ia, Ib, Ic, Id, Ie, If, Ig, Ih, Ii, Ij, Ik, IL, Im, In, Io, Ip, Iq, Ir, Is, It, Iu, Iv, Iw und Ix, in denen Lₙ 2,4-Dimethyl, R³ Cyano bedeuten und YR² für eine Verbindung jeweils einer Zeile der Tabelle A entspricht

### Tabelle 162

Verbindungen der Formel Ia, Ib, Ic, Id, Ie, If, Ig, Ih, Ii, Ij, Ik, IL, Im, In, Io, Ip, Iq, Ir, Is, It, Iu, Iv, Iw und Ix, in denen Lₙ 2-Methyl-4-chlor, R³ Cyano bedeuten und YR² für eine Verbindung jeweils einer Zeile der Tabelle A entspricht

### Tabelle 163

Verbindungen der Formel Ia, Ib, Ic, Id, Ie, If, Ig, Ih, Ii, Ij, Ik, IL, Im, In, Io, Ip, Iq, Ir, Is, It, Iu, Iv, Iw und Ix, in denen Lₙ 2-Fluor-4-methyl, R³ Cyano bedeuten und YR² für eine Verbindung jeweils einer Zeile der Tabelle A entspricht

### Tabelle 164

Verbindungen der Formel Ia, Ib, Ic, Id, Ie, If, Ig, Ih, Ii, Ij, Ik, IL, Im, In, Io, Ip, Iq, Ir, Is, lt, Iu, Iv, Iw und Ix, in denen Lₙ 2,6-Dimethyl, R³ Cyano bedeuten und YR² für eine Verbindung jeweils einer Zeile der Tabelle A entspricht

### Tabelle 165

Verbindungen der Formel Ia, Ib, Ic, Id, Ie, If, Ig, Ih, Ii, Ij, Ik, IL, Im, In, Io, Ip, Iq, Ir, Is, lt, Iu, Iv, Iw und Ix, in denen Lₙ 2,4,6-Trimethyl, R³ Cyano bedeuten und YR² für eine Verbindung jeweils einer Zeile der Tabelle A entspricht

### Tabelle 166

Verbindungen der Formel Ia, Ib, Ic, Id, Ie, If, Ig, Ih, Ii, Ij, Ik, IL, Im, In, Io, Ip, Iq, Ir, Is, It, Iu, Iv, Iw und Ix, in denen Lₙ 2,6-Difluor-4-cyano, R³ Cyano bedeuten und YR² für eine Verbindung jeweils einer Zeile der Tabelle A entspricht

### Tabelle 167

Verbindungen der Formel Ia, Ib, Ic, Id, Ie, If, Ig, Ih, Ii, Ij, Ik, IL, Im, In, Io, Ip, Iq, Ir, Is, It, Iu, Iv, Iw und Ix, in denen Lₙ 2,6-Difluor-4-methyl, R³ Cyano bedeuten und YR² für eine Verbindung jeweils einer Zeile der Tabelle A entspricht

### Tabelle 168

Verbindungen der Formel Ia, Ib, Ic, Id, Ie, If, Ig, Ih, Ii, Ij, Ik, IL, Im, In, Io, Ip, Iq, Ir, Is, lt, lu, Iv, Iw und Ix, in denen Lₙ 2,6-Difluor-4-methoxycarbonyl, R³ Cyano bedeuten und YR² für eine Verbindung jeweils einer Zeile der Tabelle A entspricht

### Tabelle 169

Verbindungen der Formel Ia, Ib, Ic, Id, Ie, If, Ig, Ih, Ii, Ij, Ik, IL, Im, In, Io, Ip, Iq, Ir, Is, It, lu, Iv, Iw und Ix, in denen Lₙ 2-Chlor,4-Methoxy, R³ Cyano bedeuten und YR² für eine Verbindung jeweils einer Zeile der Tabelle A entspricht

### Tabelle 170

Verbindungen der Formel Ia, Ib, Ic, Id, Ie, If, Ig, Ih, Ii, Ij, Ik, IL, Im, In, Io, Ip, Iq, Ir, Is, It, lu, Iv, Iw und Ix, in denen Lₙ 2-Chlor,4-Methyl, R³ Cyano bedeuten und YR² für eine Verbindung jeweils einer Zeile der Tabelle A entspricht

### Tabelle 171

Verbindungen der Formel Ia, Ib, Ic, Id, Ie, If, Ig, Ih, Ii, Ij, Ik, IL, Im, In, Io, Ip, Iq, Ir, Is, It, lu, Iv, Iw und Ix, in denen Lₙ 2-Chlor,4-methoxycarbonyl, R³ Cyano bedeuten und YR² für eine Verbindung jeweils einer Zeile der Tabelle A entspricht

### Tabelle 172

Verbindungen der Formel Ia, Ib, Ic, Id, Ie, If, Ig, Ih, Ii, Ij, Ik, IL, Im, In, Io, Ip, Iq, Ir, Is, lt, lu, Iv, Iw und Ix, in denen Lₙ 2-Chlor,4-Brom, R³ Cyano bedeuten und YR² für eine Verbindung jeweils einer Zeile der Tabelle A entspricht

### Tabelle 173

Verbindungen der Formel Ia, Ib, Ic, Id, Ie, If, Ig, Ih, Ii, Ij, Ik, IL, Im, In, Io, Ip, Iq, Ir, Is, It, Iu, Iv, Iw und Ix, in denen Lₙ 2-Chlor,4-Cyan, R³ Cyano bedeuten und YR² für eine Verbindung jeweils einer Zeile der Tabelle A entspricht

### Tabelle 174

Verbindungen der Formel Ia, Ib, Ic, Id, Ie, If, Ig, Ih, Ii, Ij, Ik, IL, Im, In, Io, Ip, Iq, Ir, Is, It, Iu, Iv, Iw und Ix, in denen Lₙ 2,6-Difluor,4-methoxy, R³ Cyano bedeuten und YR² für eine Verbindung jeweils einer Zeile der Tabelle A entspricht

### Tabelle 175

Verbindungen der Formel Ia, Ib, Ic, Id, Ie, If, Ig, Ih, Ii, Ij, Ik, IL, Im, In, Io, Ip, Iq, Ir, Is, lt, Iu, Iv, Iw und Ix, in denen Lₙ 2-Fluor,3-methyl, R³ Cyano bedeuten und YR² für eine Verbindung jeweils einer Zeile der Tabelle A entspricht

### Tabelle 176

Verbindungen der Formel la, Ib, Ic, Id, le, If, Ig, Ih, Ii, Ij, Ik, IL, Im, In, Io, Ip, Iq, Ir, Is, It, lu, Iv, Iw und Ix, in denen Lₙ 2,5-Dimethyl, R³ Cyano bedeuten und YR² für eine Verbindung jeweils einer Zeile der Tabelle A entspricht

### Tabelle 177

Verbindungen der Formel Ia, Ib, Ic, ld, Ie, If, Ig, Ih, Ii, Ij, Ik, IL, Im, In, Io, Ip, Iq, Ir, Is, lt, lu, Iv, Iw und Ix, in denen Lₙ 2-Methyl,4-cyan, R³ Cyano bedeuten und YR² für eine Verbindung jeweils einer Zeile der Tabelle A entspricht

### Tabelle 178

Verbindungen der Formel Ia, Ib, Ic, Id, Ie, If, Ig, Ih, Ii, Ij, Ik, IL, Im, In, Io, Ip, Iq, Ir, Is, It, Iu, Iv, Iw und Ix, in denen Lₙ 2-Methyl,4-brom, R³ Cyano bedeuten und YR² für eine Verbindung jeweils einer Zeile der Tabelle A entspricht

### Tabelle 179

Verbindungen der Formel Ia, Ib, Ic, Id, Ie, If, Ig, Ih, Ii, Ij, Ik, IL, Im, In, Io, Ip, Iq, Ir, Is, It, Iu, Iv, Iw und Ix, in denen Lₙ 2-Methyl,5-fluor, R³ Cyano bedeuten und YR² für eine Verbindung jeweils einer Zeile der Tabelle A entspricht

### Tabelle 180

Verbindungen der Formel Ia, Ib, Ic, Id, Ie, If, Ig, Ih, Ii, Ij, Ik, IL, Im, In, Io, Ip, Iq, Ir, Is, It, Iu, Iv, Iw und Ix, in denen Lₙ 2-Methyl,4-methoxy, R³ Cyano bedeuten und YR² für eine Verbindung jeweils einer Zeile der Tabelle A entspricht

### Tabelle 181

Verbindungen der Formel Ia, Ib, Ic, Id, Ie, If, Ig, Ih, Ii, Ij, Ik, IL, Im, In, Io, Ip, Iq, Ir, Is, It, Iu, Iv, Iw und Ix, in denen Lₙ 2-Methyl,4-methoxycarbonyl, R³ Cyano bedeuten und YR² für eine Verbindung jeweils einer Zeile der Tabelle A entspricht

### Tabelle 182

Verbindungen der Formel Ia, Ib, Ic, Id, Ie, If, Ig, Ih, Ii, Ij, Ik, IL, Im, In, Io, Ip, Iq, Ir, Is, It, Iu, Iv, Iw und Ix, in denen Lₙ 2,5-Dimethyl,4-brom, R³ Cyano bedeuten und YR² für eine Verbindung jeweils einer Zeile der Tabelle A entspricht

### Tabelle 183

Verbindungen der Formel Ia, Ib, Ic, Id, Ie, If, Ig, Ih, Ii, Ij, Ik, IL, Im, In, Io, Ip, Iq, Ir, Is, It, lu, Iv, Iw und Ix, in denen Lₙ 2-Fluor,4-brom, R³ Cyano bedeuten und YR² für eine Verbindung jeweils einer Zeile der Tabelle A entspricht

### Tabelle 184

Verbindungen der Formel Ia, Ib, Ic, Id, Ie, If, Ig, Ih, Ii, Ij, Ik, IL, Im, In, Io, Ip, Iq, It, Is, It, lu, Iv, Iw und Ix, in denen Lₙ 2-Fluor,4-methoxy, R³ Cyano bedeuten und YR² für eine Verbindung jeweils einer Zeile der Tabelle A entspricht

### Tabelle 185

Verbindungen der Formel la, Ib, Ic, Id, le, If, Ig, Ih, li, Ij, Ik, IL, Im, In, Io, Ip, Iq, Ir, Is, lt, lu, Iv, Iw und Ix, in denen Lₙ 2-Fluor,5-methyl, R³ Cyano bedeuten und YR² für eine Verbindung jeweils einer Zeile der Tabelle A entspricht

### Tabelle 186

Verbindungen der Formel Ia, Ib, Ic, Id, Ie, If, Ig, Ih, li, Ij, Ik, IL, Im, In, Io, Ip, Iq, Ir, Is, It, Iu, Iv, Iw und Ix, in denen Lₙ Pentafluor, R³ Cyano bedeuten und YR² für eine Verbindung jeweils einer Zeile der Tabelle A entspricht

### Tabelle 187

Verbindungen der Formel la, Ib, Ic, Id, Ie, If, Ig, Ih, li, Ij, Ik, IL, Im, In, Io, Ip, Iq, Ir, Is, It, lu, Iv, Iw und Ix, in denen Lₙ 2-Chlor,5-fluor,4-methoxy, R³ Cyano bedeuten und YR² für eine Verbindung jeweils einer Zeile der Tabelle A entspricht

### Tabelle 188

Verbindungen der Formel Ia, Ib, Ic, Id, le, If, Ig, Ih, li, Ij, Ik, IL, Im, In, Io, Ip, Iq, Ir, Is, It, lu, Iv, Iw und Ix, in denen Lₙ 2-Chlor,5-fluor, R³ Cyano bedeuten und YR² für eine Verbindung jeweils einer Zeile der Tabelle A entspricht

**Tabelle A**

| **Nr.** | **Y-R²** | |
|---|---|---|
| | **R²** | **Y [N-R¹, CH-R¹]** |
| A-1 | CH₂CH₃ | NH |
| A-2 | CH₂CH₃ | NCH₃ |
| A-3 | CH₂CH₃ | NCH₂CH₃ |
| A-4 | CH₂CH₂CH₃ | NH |
| A-5 | CH₂CH₂CH₃ | NCH₃ |
| A-6 | CH₂CH₂CH₃ | NCH₂CH₃ |
| A-7 | CH₂CH₂CH₃ | NCH₂CH₂CH₃ |
| A-8 | CH₂CH₂F | NH |
| A-9 | CH₂CH₂F | NCH₃ |
| A-10 | CH₂CH₂F | NCH₂CH₃ |
| A-11 | CH₂CF₃ | NH |
| A-12 | CH₂CF₃ | NCH₃ |
| A-13 | CH₂CF₃ | NCH₂CH₃ |
| A-14 | CH₂CF₃ | NCH₂CH₂CH₃ |
| A-15 | CH₂CCl₃ | NH |
| A-16 | CH₂CCl₃ | NCH₃ |
| A-17 | CH₂CCl₃ | NCH₂CH₃ |
| A-18 | CH₂CCl₃ | NCH₂CH₂CH₃ |
| A-19 | CH(CH₃)₂ | NH |
| A-20 | CH(CH₃)₂ | NCH₃ |
| A-21 | CH(CH₃)₂ | NCH₂CH₃ |
| A-22 | CH(CH₃)₂ | NCH₂CH₂CH₃ |
| A-23 | CH₂C(CH₃)₃ | NH |
| A-24 | CH₂C(CH₃)₃ | NCH₃ |
| A-25 | CH₂C(CH₃)₃ | NCH₂CH₃ |
| A-26 | CH₂CH(CH₃)₂ | NH |
| A-27 | CH₂CH(CH₃)₂ | NCH₃ |
| A-28 | CH₂CH(CH₃)₂ | NCH₂CH₃ |
| A-29 | (±) CH(CH₂CH₃)CH₃ | NH |
| A-30 | (±) CH(CH₂CH₃)CH₃ | NCH₃ |
| A-31 | (±) CH(CH₂CH₃)CH₃ | NCH₂CH₃ |
| A-32 | (R) CH(CH₂CH₃)CH₃ | NH |
| A-33 | (R) CH(CH₂CH₃)CH₃ | NCH₃ |
| A-34 | (R) CH(CH₂CH₃)CH₃ | NCH₂CH₃ |
| A-35 | (S) CH(CH₂CH₃)CH₃ | NH |
| A-36 | (S) CH(CH₂CH₃)CH₃ | NCH₃ |
| A-37 | (S) CH(CH₂CH₃)CH₃ | NCH₂CH₃ |
| A-38 | (±) CH(CH₃)-CH(CH₃)₂ | NH |
| A-39 | (±) CH(CH₃)-CH(CH₃)₂ | NCH₃ |
| A-40 | (±) CH(CH₃)-CH(CH₃)₂ | NCH₂CH₃ |
| A-41 | (R) CH(CH₃)-CH(CH₃)₂ | NH |
| A-42 | (R) CH(CH₃)-CH(CH₃)₂ | NCH₃ |
| A-43 | (R) CH(CH₃)-CH(CH₃)₂ | NCH₂CH₃ |
| A-44 | (S) CH(CH₃)-CH(CH₃)₂ | NH |
| A-45 | (S) CH(CH₃)-CH(CH₃)₂ | NCH₃ |
| A-46 | (S) CH(CH₃)-CH(CH₃)₂ | NCH₂CH₃ |
| A-47 | (±) CH(CH₃)-C(CH₃)₃ | NH |
| A-48 | (±) CH(CH₃)-C(CH₃)₃ | NCH₃ |
| A-49 | (±) CH(CH₃)-C(CH₃)₃ | NCH₂CH₃ |
| A-50 | (R) CH(CH₃)-C(CH₃)₃ | NH |
| A-51 | (R) CH(CH₃)-C(CH₃)₃ | NCH₃ |
| A-52 | (R) CH(CH₃)-C(CH₃)₃ | NCH₂CH₃ |
| A-53 | (S) CH(CH₃)-C(CH₃)₃ | NH |
| A-54 | (S) CH(CH₃)-C(CH₃)₃ | NCH₃ |
| A-55 | (S) CH(CH₃)-C(CH₃)₃ | NCH₂CH₃ |
| A-56 | (±) CH(CH₃)-CF₃ | NH |
| A-57 | (±) CH(CH₃)-CF₃ | NCH₃ |
| A-58 | (±) CH(CH₃)-CF₃ | NCH₂CH₃ |
| A-59 | (R) CH(CH₃)-CF₃ | NH |
| A-60 | (R) CH(CH₃)-CF₃ | NCH₃ |
| A-61 | (R) CH(CH₃)-CF₃ | NCH₂CH₃ |
| A-62 | (S) CH(CH₃)-CF₃ | NH |
| A-63 | (S) CH(CH₃)-CF₃ | NCH₃ |
| A-64 | (S) CH(CH₃)-CF₃ | NCH₂CH₃ |
| A-65 | (±) CH(CH₃)-CCl₃ | NH |
| A-66 | (±) CH(CH₃)-CCl₃ | CH₃ |
| A-67 | (±) CH(CH₃)-CCl₃ | NCH₂CH₃ |
| A-68 | (R) CH(CH₃)-CCl₃ | NH |
| A-69 | (R) CH(CH₃)-CCl₃ | NCH₃ |
| A-70 | (R) CH(CH₃)-CCl₃ | NCH₂CH₃ |
| A-71 | (S) CH(CH₃)-CCl₃ | NH |
| A-72 | (S) CH(CH₃)-CCl₃ | NCH₃ |
| A-73 | (S) CH(CH₃)-CCl₃ | NCH₂CH₃ |
| A-74 | CH₂C(CH₃)=CH₂ | NH |
| A-75 | CH₂C(CH₃)=CH₂ | NCH₃ |
| A-76 | CH₂C(CH₃)=CH₂ | NCH₂CH₃ |
| A-77 | CH(CH₃)₂₃ | O |
| A-78 | CH(CH₃)₂₃ | S |
| A-79 | CH₂CH(CH₃)₂ | O |
| A-80 | CH₂CH(CH₃)₂ | S |
| A-81 | CH₂C(CH₃)₃ | O |
| A-82 | CH₂C(CH₃)₃ | S |
| A-83 | CH(CH₂CH₃)CH₃ | O |
| A-84 | CH(CH₂CH₃)CH₃ | S |
| A-85 | CH(CH₃)-CH(CH₃)₂ | O |
| A-86 | CH(CH₃)-CH(CH₃)₂ | S |
| A-87 | CH(CH₃)-C(CH₃)₃ | O |
| A-88 | CH(CH₃)-C(CH₃)₃ | S |
| A-89 | CH(CH₃)-CF₃ | O |
| A-90 | CH(CH₃)-CF₃ | S |
| A-91 | CH(CH₃)-CCl₃ | O |
| A-92 | CH(CH₃)-CCl₃ | S |
| A-93 | CH₂C(CH₃)=CH₂ | O |
| A-94 | CH₂C(CH₃)=CH₂ | S |
| A-95 | Cyclopentyl | NH |
| A-96 | Cyclopentyl | NCH₃ |
| A-97 | Cyclopentyl | NCH₂CH₃ |
| A-98 | Cyclohexyl | NH |
| A-99 | Cyclohexyl | NCH₃ |
| A-100 | Cyclohexyl | NCH₂CH₃ |
| A-101 | 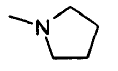 | |
| A-102 | 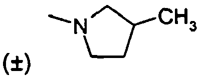 | |
| A-103 | 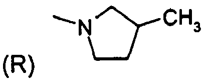 | |
| A-104 | 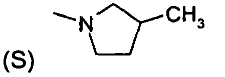 | |
| A-105 | 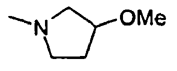 | |
| A-106 | 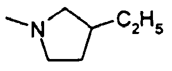 | |
| A-107 | 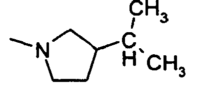 | |
| A-108 | 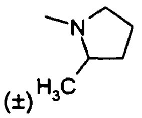 | |
| A-109 | 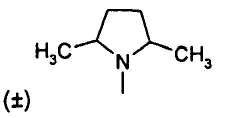 | |
| A-110 | 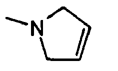 | |
| A-111 | 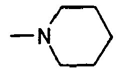 | |
| A-112 | 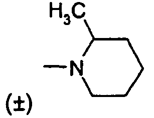 | |
| A-113 | 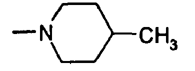 | |
| A-114 | 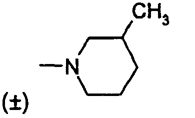 | |
| A-115 | 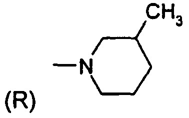 | |
| A-116 | 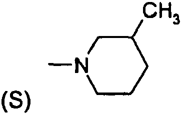 | |
| A-117 | 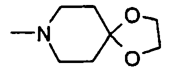 | |
| A-118 | 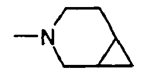 | |
| A-119 | 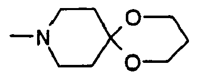 | |
| A-120 | 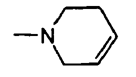 | |
| A-121 | CH₃ | |
| A-122 | CH₂CH₃ | |
| A-123 | CH₂CH₂CH₃ | |
| A-124 | CH(CH₃)₂ | |
| A-125 | CH₂CH(CH₃)₂ | |
| A-126 | (±) CH(CH₃)CH₂CH₃ | |
| A-127 | (R) CH(CH₃)CH₂CH₃ | |
| A-128 | (S) CH(CH₃)CH₂CH₃ | |
| A-129 | (CH₂)₃CH₃ | |
| A-130 | C(CH₃)₃ | |
| A-131 | (CH₂)₄CH₃ | |
| A-132 | CH(CH₂CH₃)₂ | |
| A-133 | CH₂CH₂CH(CH₃)₂ | |
| A-134 | (±) CH(CH₃)(CH₂)₂CH₃ | |
| A-135 | (R) CH(CH₃)(CH₂)₂CH₃ | |
| A-136 | (S) CH(CH₃)(CH₂)₂CH₃ | |
| A-137 | (±) CH₂CH(CH₃)CH₂CH₃ | |
| A-138 | (R) CH₂CH(CH₃)CH₂CH₃ | |
| A-139 | (S) CH₂CH(CH₃)CH₂CH₃ | |
| A-140 | (±) CH(CH₃)CH(CH₃)₂ | |
| A-141 | (R) CH(CH₃)CH(CH₃)₂ | |
| A-142 | (S) CH(CH₃)CH(CH₃)₂ | |
| A-143 | (CH₂)₅CH₃ | |
| A-144 | (±,±) CH(CH₃)CH(CH₃)CH₂CH₃ | |
| A-145 | (±,R) CH(CH₃)CH(CH₃)CH₂CH₃ | |
| A-146 | (±,S) CH(CH₃)CH(CH₃)CH₂CH₃ | |
| A-147 | (±) CH₂CH(CH₃)CF₃ | |
| A-148 | (R) CH₂CH(CH₃)CF₃ | |
| A-149 | (S) CH₂CH(CH₃)CF₃ | |
| A-150 | (±) CH₂CH(CF₃)CH₂CH₃ | |
| A-151 | (R) CH₂CH(CF₃)CH₂CH₃ | |
| A-152 | (S) CH₂CH(CF₃)CH₂CH₃ | |
| A-153 | (±,±) CH(CH₃)CH(CH₃)CF₃ | |
| A-154 | (±,R) CH(CH₃)CH(CH₃)CF₃ | |
| A-155 | (±,S) CH(CH₃)CH(CH₃)CF₃ | |
| A-156 | (±,±) CH(CH₃)CH(CF₃)CH₂CH₃ | |
| A-157 | (±,R) CH(CH₃)CH(CF₃)CH₂CH₃ | |
| A-158 | (±,S) CH(CH₃)CH(CF₃)CH₂CH₃ | |
| A-159 | CF₃ | |
| A-160 | CF₂CF₃ | |
| A-161 | CF₂CF₂CF₃ | |
| A-162 | c-C₃H₅ | |
| A-163 | (1-CH₃)-c-C₃H₄ | |
| A-164 | c-C₅H₉ | |
| A-165 | c-C₆H₁₁ | |
| A-166 | (4-CH₃)-c-C₆H₁₀ | |
| A-167 | CH₂C(CH₃)=CH₂ | |
| A-168 | CH₂CH₂C(CH₃)=CH₂ | |
| A-169 | CH₂-C(CH₃)₃ | |
| A-170 | CH₂-Si(CH₃)₃ | |
| A-171 | n-C₆H₁₃ | |
| A-172 | (CH₂)₃-CH(CH₃)₂ | |
| A-173 | (CH₂)₂-CH(CH₃)-C₂H₅ | |
| A-174 | CH₂-CH(CH₃)-n-C₃H₇ | |
| A-175 | CH(CH₃)-n-C₄H₉ | |
| A-176 | CH₂-CH(C₂H₅)₂ | |
| A-177 | CH(C₂H₅)-n-C₃H₇ | |
| A-178 | CH₂-c-C₅H₉ | |
| A-179 | CH₂-CH(CH₃)-CH(CH₃)₂ | |
| A-180 | CH(CH₃)-CH₂CH(CH₃)₂ | |
| A-181 | CH(CH₃)-CH(CH₃)-C₂H₅ | |
| A-182 | CH(CH₃)-C(CH₃)₃ | |
| A-183 | (CH₂)₂-C(CH₃)₃ | |
| A-184 | CH₂-C(CH₃)₂-C₂H₅ | |
| A-185 | 2-CH₃-c-C₅H₈ | |
| A-186 | 3-CH₃-c-C₅H₈ | |
| A-187 | C(CH₃)₂-n-C₃H₇ | |
| A-188 | (CH₂)₆-CH₃ | |
| A-189 | (CH₂)₄-CH(CH₃)₂ | |
| A-190 | (CH₂)₃-CH(CH₃)-C₂H₅ | |
| A-191 | (CH₂)₂-CH(CH₃)-n-C₃H₇ | |
| A-192 | CH₂-CH(CH₃)-n-C₄H₉ | |
| A-193 | CH(CH₃)-n-C₅H₁₁ | |
| A-194 | (CH₂)₃C(CH₃)₃ | |
| A-195 | (CH₂)₂CH(CH₃)-CH(CH₃)₂ | |
| A-196 | (CH₂)CH(CH₃)-CH₂CH(CH₃)₂ | |
| A-197 | CH(CH₃)(CH₂)₂-CH(CH₃)₂ | |
| A-198 | (CH₂)₂C(CH₃)₂C₂H₅ | |
| A-199 | CH₂CH(CH₃)CH(CH₃)C₂H₅ | |
| A-200 | CH(CH₃)CH₂CH(CH₃)C₂H₅ | |
| A-201 | CH₂C(CH₃)₂-n-C₃H₇ | |
| A-202 | CH(CH₃)CH(CH₃)-n-C₃H₇ | |
| A-203 | C(CH₃)₂-n-C₄H₉ | |
| A-204 | (CH₂)₂CH(C₂H₅)₂ | |
| A-205 | CH₂CH(C₂H₅)-n-C₃H₇ | |
| A-206 | CH(C₂H₅)-n-C₄H₉ | |
| A-207 | CH₂CH(CH₃)C(CH₃)₃ | |
| A-208 | CH(CH₃)CH₂C(CH₃)₃ | |
| A-209 | CH₂C(CH₃)₂CH(CH₃)₂ | |
| A-210 | CH₂CH(C₂H₅)CH(CH₃)₂ | |
| A-211 | CH(CH₃)CH(CH₃)CH(CH₃)₂ | |
| A-212 | C(CH₃)₂CH₂CH(CH₃)₂ | |
| A-213 | CH(C₂H₅)CH₂CH(CH₃)₂ | |
| A-214 | CH(CH₃)C(CH₃)₂C₂H₅ | |
| A-215 | CH(CH₃)CH(C₂H₅)₂ | |
| A-216 | C(CH₃)₂CH(CH₃)C₂H₅ | |
| A-217 | CH(C₂H₅)CH(CH₃)C₂H₅ | |
| A-218 | C(CH₃)(C₂H₅)-n-C₃H₇ | |
| A-219 | CH(n-C₃H₇)₂ | |
| A-220 | CH(n-C₃H₇)CH(CH₃)₂ | |
| A-221 | C(CH₃)₂C(CH₃)₃ | |
| A-222 | C(CH₃)(C₂H₅)-CH(CH₃)₂ | |
| A-223 | C(C₂H₅)₃ | |
| A-224 | (3-CH₃)-c-C₆H₁₀ | |
| A-225 | (2-CH₃)-c-C₆H₁₀ | |
| A-226 | n-C₈H₁₇ | |
| A-227 | CH₂C(=NO-CH₃)CH₃ | |
| A-228 | CH₂C(=NO-C₂H₅)CH₃ | |
| A-229 | CH₂C(=NO-n-C₃H₇)CH₃ | |
| A-230 | CH₂C(=NO-i-C₃H₇)CH₃ | |
| A-231 | CH(CH₃)C(=NOCH₃)CH₃ | |
| A-232 | CH(CH₃)C(=NOC₂H₅)CH₃ | |
| A-233 | CH(CH₃)C(=NO-n-C₃H₇)CH₃ | |
| A-234 | CH(CH₃)C(=NO-i-C₃H₇)CH₃ | |
| A-235 | C(=NOCH₃)C(=NOCH₃)CH₃ | |
| A-236 | C(=NOCH₃)C(=NOC₂H₅)CH₃ | |
| A-237 | C(=NOCH₃)C(=NO-n-C₃H₇)CH₃ | |
| A-238 | C(=NOCH₃)C(=NO-i-C₃H,)CH₃ | |
| A-239 | C(=NOC₂H₅)C(=NOCH₃)CH₃ | |
| A-240 | C(=NOC₂H₅)C(=NOC₂H₅)CH₃ | |
| A-241 | C(=NOC₂H₅)C(=NO-n-C₃H₇)CH₃ | |
| A-242 | C(=NOC₂H₅)C(=NO-i-C₃H₇)CH₃ | |
| A-243 | CH₂C(=NO-CH₃)C₂H₅ | |
| A-244 | CH₂C(=NO-C₂H₅)C₂H₅ | |
| A-245 | CH₂C(=NO-n-C₃H₇)C₂H₅ | |
| A-246 | CH₂C(=NO-i-C₃H₇)C₂H₅ | |
| A-247 | CH(CH₃)C(=NOCH₃)C₂H₅ | |
| A-248 | CH(CH₃)C(=NOC₂H₅)C₂H₅ | |
| A-249 | CH(CH₃)C(=NO-n-C₃H₇)C₂H₅ | |
| A-250 | CH(CH₃)C(=NO-n-C₃H₇)C₂H₅ | |
| A-251 | C(=NOCH₃)C(=NOCH₃)C₂H₅ | |
| A-252 | C(=NOCH₃)C(=NOC₂H₅)C₂H₅ | |
| A-253 | C(=NOCH₃)C(=NO-n-C₃H₇)C₂H₅ | |
| A-254 | C(=NOCH₃)C(=NO-i-C₃H₇)C₂H₅ | |
| A-255 | C(=NOC₂H₅)C(=NOCH₃)C₂H₅ | |
| A-256 | C(=NOC₂H₅)C(=NOC₂H₅)C₂H₅ | |
| A-257 | C(=NOC₂H₅)C(=NO-n-C₃H₇)C₂H₅ | |
| A-258 | C(=NOC₂H₅)C(=NO-i-C₃H₇)C₂H₅ | |
| A-259 | CH=CH-CH₂CH₃ | |
| A-260 | CH₂-CH=CH-CH₃ | |
| A-261 | CH₂-CH₂-CH=CH₂ | |
| A-262 | C(CH₃)₂CH₂CH₃ | |
| A-263 | CH=C(CH₃)₂ | |
| A-264 | C(=CH₂)-CH₂CH₃ | |
| A-265 | C(CH₃)=CH-CH₃ | |
| A-266 | CH(CH₃)CH=CH₂ | |
| A-267 | CH=CH-n-C₃H₇ | |
| A-268 | CH₂-CH=CH-C₂H₅ | |
| A-269 | (CH₂)₂-CH=CH-CH₃ | |
| A-270 | (CH₂)₃-CH=CH₂ | |
| A-271 | CH=CH-CH(CH₃)₂ | |
| A-272 | CH₂-CH=C(CH₃)₂ | |
| A-273 | (CH₂)₂-C(CH₃)=CH₂ | |
| A-274 | CH=C(CH₃)-C₂H₅ | |
| A-275 | CH₂-C(=CH₂)-C₂H₅ | |
| A-276 | CH₂-C(CH₃)=CH-CH₃ | |
| A-277 | CH₂-CH(CH₃)-CH=CH₂ | |
| A-278 | C(=CH₂)-CH₂-CH₂-CH₃ | |
| A-279 | C(CH₃)=CH-CH₂-CH₃ | |
| A-280 | CH(CH₃)-CH=CH-CH₃ | |
| A-281 | CH(CH₃)-CH₂-CH=CH₂ | |
| A-282 | C(=CH₂)CH(CH₃)₂ | |
| A-283 | C(CH₃)=C(CH₃)₂ | |
| A-284 | CH(CH₃)-C(=CH₂)-CH₃ | |
| A-285 | C(CH₃)₂-CH=CH₂ | |
| A-286 | C(C₂H₅)=CH-CH₃ | |
| A-287 | CH(C₂H₅)-CH=CH₂ | |
| A-288 | CH=CH-CH₂CH₂-CH₂-CH₃ | |
| A-289 | CH₂-CH=CH-CH₂-CH₂-CH₃ | |
| A-290 | CH₂-CH₂-CH=CH-CH₂-CH₃ | |
| A-291 | CH₂-CH₂-CH₂-CH=CH-CH₃ | |
| A-292 | CH₂-CH₂-CH₂-CH₂-CH=CH₂ | |
| A-293 | CH=CH-CH₂-CH(CH₃)CH₃ | |
| A-294 | CH₂-CH=CH-CH(CH₃)CH₃ | |
| A-295 | CH₂-CHᵣCH₌C(CH₃)CH₃ | |
| A-296 | CH₂-CH₂-CH_{Z}-C(CH₃)=CH₂ | |
| A-297 | CH=CH-CH(CH₃)-CH₂-CH₃ | |
| A-298 | CH₂-CH=C(CH₃)-CH₂-CH₃ | |
| A-299 | CH₂-CH₂-C(=CH₂)-CH₂-CH₃ | |
| A-300 | CH₂-CH₂-C(CH₃)=CH-CH₃ | |
| A-301 | CH₂-CH₂-CH(CH₃)-CH=CH₂ | |
| A-302 | CH=C(CH₃)-CH₂-CH₂-CH₃ | |
| A-303 | CH₂-C(=CH₂)-CH₂-CH₂-CH₃ | |
| A-304 | CH₂-C(CH₃)=CH-CH₂-CH₃ | |
| A-305 | CH₂-CH(CH₃)-CH=CH-CH₃ | |
| A-306 | CH₂-CH(CH₃)-CH₂-CH=CH₂ | |
| A-307 | C(=CH₂)-CH₂-CH₂-CH₂-CH₃ | |
| A-308 | C(CH₃)=CH-CH_{Z}-CH₂-CH₃ | |
| A-309 | CH(CH₃)-CH=CH-CH₂-CH₃ | |
| A-310 | CH(CH₃)-CH₂-CH=CH-CH₃ | |
| A-311 | CH(CH₃)-CH₂-CH₂-CH=CH₂ | |
| A-312 | CH=CH-C(CH₃)₃ | |
| A-313 | CH=C(CH₃)-CH(CH₃)-CH₃ | |
| A-314 | CH₂-C(=CH₂)-CH(CH₃)-CH₃ | |
| A-315 | CH₂-C(CH₃)=C(CH₃)-CH₃ | |
| A-316 | CH₂-CH(CH₃)-C(=CH₂)-CH₃ | |
| A-317 | C(=CH₂)=CH₂-CH(CH₃)-CH₃ | |
| A-318 | C(CH₃)=CH-CH(CH₃)-CH₃ | |
| A-319 | CH(CH₃)-CH=C(CH₃)-CH₃ | |
| A-320 | CH(CH₃)-CH₂-C(=CH₂)-CH₃ | |
| A-321 | CH=C(CH₂-CH₃)-CH₂-CH₃ | |
| A-322 | CH₂-C(=CH-CH₃)-CH₂-CH₃ | |
| A-323 | CH₂-CH(CH=CH₂)-CH₂-CH₃ | |
| A-324 | C(=CH-CH₃)-CH₂-CH₂-CH₃ | |
| A-325 | CH(CH=CH₂)-CH₂-CH₂-CH₃ | |
| A-326 | C(CH₂-CH₃)=CH-CH₂-CH₃ | |
| A-327 | CH(CH₂-CH₃)-CH=CH-CH₃ | |
| A-328 | CH(CH₂-CH₃)-CH₂-CH=CH₂ | |
| A-329 | CH₂-C(CH₃)₂-CH=CH₂ | |
| A-330 | C(=CH₂)-CH(CH₃)-CH₂-CH₃ | |
| A-331 | C(CH₃)=C(CH₃)-CH₂-CH₃ | |
| A-332 | CH(CH₃)-C(=CH₂)-CH₂-CH₃ | |
| A-333 | CH(CH₃)-C(CH₃)=CH-CH₃ | |
| A-334 | CH(CH₃)-CH(CH₃)-CH=CH₂ | |
| A-335 | C(CH₃)₂-CH=CH-CH₃ | |
| A-336 | C(CH₃)₂-CH₂-CH=CH₂ | |
| A-337 | C(=CH₂)-C(CH₃)₃ | |
| A-338 | C(=CH-CH₃)-CH(CH₃)-CH₃ | |
| A-339 | CH(CH=CH₂)-CH(CH₃)-CH₃ | |
| A-340 | C(CH₂-CH₃)=C(CH₃)-CH₃ | |
| A-341 | CH(CH₂-CH₃)-C(=CH₂)-CH₃ | |
| A-342 | C(CH₃)₂-C(=CH₂)-CH₃ | |
| A-343 | C(CH₃)(CH=CH₂)-CH₂-CH₃ | |
| A-344 | C(CH₃)(CH₂CH₃)-CH₂-CH₂-CH₃ | |
| A-345 | CH(CH₂CH₃)-CH(CH₃)-CH₂-CH₃ | |
| A-346 | CH(CH₂CH₃)-CH₂-CH(CH₃)-CH₃ | |
| A-347 | C(CH₃)₂-C(CH₃)₃ | |
| A-348 | C(CH₂-CH₃)-C(CH₃)₃ | |
| A-349 | C(CH₃)(CH₂-CH₃)-CH(CH₃)₂ | |
| A-350 | CH(CH(CH₃)₂)-CH(CH₃)₂ | |
| A-351 | CH=CH-CH₂-CH₂-CH₂-CH₂-CH₃ | |
| A-352 | CH₂-CH=CH-CH₂-CH₂-CH₂-CH₃ | |
| A-353 | CH₂-CH₂-CH=CH-CH₂-CH₂-CH₃ | |
| A-354 | CH₂-CH₂-CH₂-CH=CH-CH₂-CH₃ | |
| A-355 | CH₂-CH₂-CH₂-CH₂-CH=CH-CH₃ | |
| A-356 | CH₂-CH₂-CH₂-CH₂-CH₂-CH=CH₂ | |
| A-357 | CH=CH-CH₂-CH₂-CH(CH₃)-CH₃ | |
| A-358 | CH₂-CH=CH-CH₂-CH(CH₃)-CH₃ | |
| A-359 | CH₂-CH₂-CH=CH-CH(CH₃)-CH₃ | |
| A-360 | CH₂-CH₂-CH₂-CH=C(CH₃)-CH₃ | |
| A-361 | CH₂-CH₂-CH₂-CH₂-C(=CH₂)-CH₃ | |
| A-362 | CH=CH-CH₂-CH(CH₃)-CH₂-CH₃ | |
| A-363 | CH₂-CH=CH-CH(CH₃)-CH₂-CH₃ | |
| A-364 | CH₂-CH₂-CH=C(CH₃)-CH₂-CH₃ | |
| A-365 | CH₂-CH₂-CH₂-C(=CH₂)-CH₂-CH₃ | |
| A-366 | CH₂-CH₂-CH₂-C(CH₃)=CH-CH₃ | |
| A-367 | CH₂-CH₂-CH₂-CH(CH₃)-CH=CH₂ | |
| A-368 | CH=CH-CH(CH₃)-CH₂-CH₂-CH₃ | |
| A-369 | CH₂-CH=C(CH₃)-CH₂-CH₂-CH₃ | |
| A-370 | CH₂-CH₂-C(=CH₂)-CH₂-CH₂-CH₃ | |
| A-371 | CH₂-CH₂-C(CH₃)=CH-CH₂-CH₃ | |
| A-372 | CH₂-CH₂-CH(CH₃)-CH=CH-CH₃ | |
| A-373 | CH₂-CH₂-CH(CH₃)-CH₂-CH=CH₂ | |
| A-374 | CH=C(CH₃)-CH₂-CH₂-CH₂-CH₃ | |
| A-375 | CH₂-C(=CH₂)-CH₂-CH₂-CH₂-CH₃ | |
| A-376 | CH₂-C(CH₃)=CH-CH₂-CH₂-CH₃ | |
| A-377 | CH₂-CH(CH₃)-CH=CH-CH₂-CH₃ | |
| A-378 | CH₂-CH(CH₃)-CH₂-CH=CH-CH₃ | |
| A-379 | CH₂-CH(CH₃)-CH₂-CH₂-CH=CH₂ | |
| A-380 | C(=CH₂)-CH₂-CH₂-CH₂-CH₂-CH₃ | |
| A-381 | C(CH₃)=CH-CH₂-CH₂-CH₂-CH₃ | |
| A-382 | CH(CH₃)-CH=CH-CH₂-CH₂-CH₃ | |
| A-383 | CH(CH₃)-CH₂-CH=CH-CH₂-CH₃ | |
| A-384 | CH(CH₃)-CH₂-CH₂-CH=CH-CH₃ | |
| A-385 | CH(CH₃)-CH₂-CH₂-CH₂-CH=CH₂ | |
| A-386 | CH=CH-CH₂-C(CH₃)₃ | |
| A-387 | CH₂-CH=CH-C(CH₃)₃ | |
| A-388 | CH=CH-CH(CH₃)-CH(CH₃)₂ | |
| A-389 | CH₂-CH=C(CH₃)-CH(CH₃)₂ | |
| A-390 | CH₂-CH₂-C(=CH₂)-CH(CH₃)₂ | |
| A-391 | CH₂-CH₂-C(CH₃)=C(CH₃)₂ | |
| A-392 | CH₂-CH₂-CH(CH₃)-C(=CH₂)-CH₃ | |
| A-393 | CH=C(CH₃)-CH₂-CH(CH₃)₂ | |
| A-394 | CH₂-C(=CH₂)-CH₂-CH(CH₃)₂ | |
| A-395 | CH₂-C(CH₃)=CH-CH(CH₃)₂ | |
| A-396 | CH₂-CH(CH₃)-CH=C(CH₃)₂ | |
| A-397 | CH₂-CH(CH₃)-CH₂-C(=CH₂)-CH₃ | |
| A-398 | C(=CH₂)-CH₂-CH₂-CH(CH₃)₂ | |
| A-399 | C(CH₃)=CH-CH₂-CH(CH₃)₂ | |
| A-400 | CH(CH₃)-CH=CH-CH(CH₃)₂ | |
| A-401 | CH(CH₃)-CH₂-CH=C(CH₃)₂ | |
| A-402 | CH(CH₃)-CH₂-CH₂-C(=CH₂)-CH₃ | |
| A-403 | CH=CH-C(CH₃)₂-CH₂-CH₃ | |
| A-404 | CH₂-CH₂-C(CH₃)₂-CH=CH₂ | |
| A-405 | CH=C(CH₃)-CH(CH₃)-CH₂-CH₃ | |
| A-406 | CH₂-C(=CH₂)-CH(CH₃)-CH₂-CH₃ | |
| A-407 | CH₂-C(CH₃)=C(CH₃)-CH₂-CH₃ | |
| A-408 | CH₂-CH(CH₃)-C(=CH₂)-CH₂-CH₃ | |
| A-409 | CH₂-CH(CH₃)-C(CH₃)=CH-CH₃ | |
| A-410 | CH₂-CH(CH₃)-CH(CH₃)-CH=CH₂ | |
| A-411 | C(=CH₂)-CH₂-CH(CH₃)-CH₂-CH₃ | |
| A-412 | C(CH₃)=CH-CH(CH₃)-CH₂-CH₃ | |
| A-413 | CH(CH₃)-CH=C(CH₃)-CH₂-CH₃ | |
| A-414 | CH(CH₃)-CH₂-C(=CH₂)-CH₂-CH₃ | |
| A-415 | CH(CH₃)-CH₂-C(CH₃)=CH-CH₃ | |
| A-416 | CH(CH₃)-CH₂-CH(CH₃)-CH=CH₂ | |
| A-417 | CH₂-C(CH₃)₂-CH=CH-CH₃ | |
| A-418 | CH₂-C(CH₃)₂-CH₂-CH=CH₂ | |
| A-419 | C(=CH₂)-CH(CH₃)-CH₂-CH₂-CH₃ | |
| A-420 | C(CH₃)=C(CH₃)-CH₂-CH₂-CH₃ | |
| A-421 | CH(CH₃)-C(=CH₂)-CH₂-CH₂-CH₃ | |
| A-422 | CH(CH₃)-C(CH₃)=CH-CH₂-CH₃ | |
| A-423 | CH(CH₃)-CH(CH₃)-CH=CH-CH₃ | |
| A-424 | CH(CH₃)-CH(CH₃)-CH₂-CH=CH₂ | |
| A-425 | C(CH₃)₂-CH=CH-CH₂-CH₃ | |
| A-426 | C(CH₃)₂-CH₂-CH=CH-CH₃ | |
| A-427 | C(CH₃)₂-CH₂-CH₂-CH=CH₂ | |
| A-428 | CH=CH-CH(CH₂-CH₃)-CH₂-CH₃ | |
| A-429 | CH₂-CH=C(CH₂-CH₃)-CH₂-CH₃ | |
| A-430 | CH₂-CH₂-C(=CH-CH₃)-CH₂-CH₃ | |
| A-431 | CH₂-CH₂-CH(CH=CH₂)-CH₂-CH₃ | |
| A-432 | CH=C(CH₂-CH₃)-CH₂-CH₂-CH₃ | |
| A-433 | CH₂-C(=CH-CH₃)-CH₂-CH₂-CH₃ | |
| A-434 | CH₂-CH(CH=CH₂)-CH₂-CH₂-CH₃ | |
| A-435 | CH₂-C(CH₂-CH₃)=CH-CH₂-CH₃ | |
| A-436 | CH₂-CH(CH₂-CH₃)-CH=CH-CH₃ | |
| A-437 | CH₂-CH(CH₂-CH₃)-CH-CH=CH₂ | |
| A-438 | C(=CH-CH₃)-CH₂-CH₂-CH₂-CH₃ | |
| A-439 | CH(CH=CH₂)-CH₂-CH₂-CH₂-CH₃ | |
| A-440 | C(CH₂-CH₃)=CH-CH₂-CH₂-CH₃ | |
| A-441 | CH(CH₂-CH₃)-CH=CH-CH₂-CH₃ | |
| A-442 | CH(CH₂-CH₃)-CH₂-CH=CH-CH₃ | |
| A-443 | CH(CH₂-CH₃)-CH₂-CH₂-CH=CH₂ | |
| A-444 | C(=CH-CH₂-CH₃)-CH₂-CH₂-CH₃ | |
| A-445 | C(CH=CH-CH₃)-CH₂-CH₂-CH₃ | |
| A-446 | C(CH₂-CH=CH₂)-CH₂-CH₂-CH₃ | |
| A-447 | CH=C(CH₃)-C(CH₃)₃ | |
| A-448 | CH₂-C(=CH₂)-C(CH₃)₃ | |
| A-449 | CH₂-C(CH₃)₂-CH(=CH₂)-CH₃ | |
| A-450 | C(=CH₂)-CH(CH₃)-CH(CH₃)-CH₃ | |
| A-451 | C(CH₃)=C(CH₃)-CH(CH₃)-CH₃ | |
| A-452 | CH(CH₃)-C(=CH₂)-CH(CH₃)-CH₃ | |
| A-453 | CH(CH₃)-C(CH₃)=C(CH₃)-CH₃ | |
| A-454 | CH(CH₃)-CH(CH₃)-C(=CH₂)-CH₃ | |
| A-455 | C(CH₃)₂-CH=C(CH₃)-CH₃ | |
| A-456 | C(CH₃)₂-CH₂-C(=CH₂)-CH₃ | |
| A-457 | C(CH₃)₂-C(=CH₂)-CH₂-CH₃ | |
| A-458 | C(CH₃)₂-C(CH₃)=CH-CH₃ | |
| A-459 | C(CH₃)₂-CH(CH₃)CH=CH₂ | |
| A-460 | CH(CH₂-CH₃)-CH₂-CH(CH₃)-CH₃ | |
| A-461 | CH(CH₂-CH₃)-CH(CH₃)-CH₂-CH₃ | |
| A-462 | C(CH₃)(CH₂-CH₃)-CH₂-CH₂-CH₃ | |
| A-463 | CH(i-C₃H₇)-CH₂-CH₂-CH₃ | |
| A-464 | CH=C(CH₂-CH₃)-CH(CH₃)-CH₃ | |
| A-465 | CH₂-C(=CH-CH₃)-CH(CH₃)-CH₃ | |
| A-466 | CH₂-CH(CH=CH₂)-CH(CH₃)-CH₃ | |
| A-467 | CH₂-C(CH₂-CH₃)=C(CH₃)-CH₃ | |
| A-468 | CH₂-CH(CH₂-CH₃)-C(=CH₂)-CH₃ | |
| A-469 | CH₂-C(CH₃)(CH=CH₂)-CH₂-CH₃ | |
| A-470 | C(=CH₂)-CH(CH₂-CH₃)-CH₂-CH₃ | |
| A-471 | C(CH₃)=C(CH₂-CH₃)-CH₂-CH₃ | |
| A-472 | CH(CH₃)-C(=CH-CH₃)-CH₂-CH₃ | |
| A-473 | CH(CH₃)-CH(CH=CH₂)-CH2-CH₃ | |
| A-474 | CH=C(CH₂-CH₃)-CH(CH₃)-CH₃ | |
| A-475 | CH₂-C(=CH-CH₃)-CH(CH₃)-CH₃ | |
| A-476 | CH₂-CH(CH=CH₂)-CH(CH₃)-CH₃ | |
| A-477 | CH₂-C(CH₂-CH₃)=C(CH₃)-CH₃ | |
| A-478 | CH₂-CH(CH₂-CH₃)-C(=CH₂)-CH₃ | |
| A-479 | C(=CH-CH₃)-CH₂-CH(CH₃)-CH₃ | |
| A-480 | CH(CH=CH₂)-CH₂-CH(CH₃)-CH₃ | |
| A-481 | C(CH₂-CH₃)=CH-CH(CH₃)-CH₃ | |
| A-482 | CH(CH₂-CH₃)CH=C(CH₃)-CH₃ | |
| A-483 | CH(CH₂-CH₃)CH₂-C(=CH₂)-CH₃ | |
| A-484 | C(=CH-CH₃)CH(CH₃)-CH₂-CH₃ | |
| A-485 | CH(CH=CH₂)CH(CH₃)-CH₂-CH₃ | |
| A-486 | C(CH₂-CH₃)=C(CH₃)-CH₂-CH₃ | |
| A-487 | CH(CH₂-CH₃)-C(=CH₂)-CH₂-CH₃ | |
| A-488 | CH(CH₂-CH₃)-C(CH₃)=CH-CH₃ | |
| A-489 | CH(CH₂-CH₃)-CH(CH₃)-CH=CH₂ | |
| A-490 | C(CH₃)(CH=CH₂)-CH₂-CH₂-CH₃ | |
| A-491 | C(CH₃)(CH₂-CH₃)-CH=CH-CH₃ | |
| A-492 | C(CH₃)(CH₂-CH₃)-CH₂-CH=CH₂ | |
| A-493 | C[=C(CH₃)-CH_{3]}-CH₂-CH₂-CH₃ | |
| A-494 | CH[C(=CH₂)-CH₃]-CH₂-CH₂-CH₃ | |
| A-495 | C(i-C₃H₇)=CH-CH₂-CH₃ | |
| A-496 | CH(i-C₃H₇)-CH=CH-CH₃ | |
| A-497 | CH(i-C₃H₇)-CH₂-CH=CH₂ | |
| A-498 | C(=CH-CH₃)-C(CH₃)₃ | |
| A-499 | CH(CH=CH₂)-C(CH₃)₃ | |
| A-500 | C(CH₃)(CH=CH₂)CH(CH₃)-CH₃ | |
| A-501 | C(CH₃)(CH₂-CH₃)C(=CH₂)-CH₃ | |
| A-502 | 2-CH₃-Cyclohex-1-enyl | |
| A-503 | [2-(=CH₂)]-c-C₆H₉ | |
| A-504 | 2-CH₃-Cyclohex-2-enyl | |
| A-505 | 2-CH₃-Cyclohex-3-enyl | |
| A-506 | 2-CH₃-Cyclohex-4-enyl | |
| A-507 | 2-CH₃-Cyclohex-5-enyl | |
| A-508 | 2-CH₃-Cyclohex-6-enyl | |
| A-509 | 3-CH₃-Cyclohex-1-enyl | |
| A-510 | 3-CH₃-Cyclohex-2-enyl | |
| A-511 | [3-(=CH₂)]-c-C₆H₉ | |
| A-512 | 3-CH₃-Cyclohex-3-enyl | |
| A-513 | 3-CH₃-Cyclohex-4-enyl | |
| A-514 | 3-CH₃-Cyclohex-5-enyl | |
| A-515 | 3-CH₃-Cyclohex-6-enyl | |
| A-516 | 4-CH₃-Cyclohex-1-enyl | |
| A-517 | 4-CH₃-Cyclohex-2-enyl | |
| A-518 | 4-CH₃-Cyclohex-3-enyl | |
| A-519 | [4-(=CH₂)]-c-C₆H₉ | |

Die erfindungsgemäßen Verbindungen der Formel I eignen sich zur Bekämpfung von Schadpilzen und von tierischen Schädlingen aus der Klasse der Insekten, Spinnentiere und Nematoden. Sie können im Pflanzenschutz sowie auf dem Hygiene-, Vorratsschutz- und Veterinärsektor als Fungizide und Schädlingsbekämpfungsmittel eingesetzt werden.

Zu den schädlichen Insekten gehören:
aus der Ordnung der Schmetterlinge *(Lepidoptera) beispielsweise Adoxophyes orana, Agrotis ypsilon, Agrotis segetum, Alabama argillacea, Anticarsia gemmatalis, Argyresthia conjugella, Autographa gamma, Cacoecia murinana, Capua reticulana, Choristoneura fumiferana, Chilo partellus, Choristoneura occidentalis, Cirphis unipuncta, Cnaphalocrocis medinalis, Crocidolomia binotalis, Cydia pomonella, Dendrolimus pini, Diaphania nitidalis, Diatraea grandiosella, Earias insulana, Elasmopalpus lignosellus, Eupoecilia ambiguella, Feltia subterranea, Grapholitha funebrana, Grapholitha molesta, Heliothis armigera, Heliothis virescens, Heliothis zea, Hellula undalis, Hibemia defoliaria, Hyphantria cunea, Hyponomeuta malinellus, Keiferia lycopersicella, Lambdina fiscellaria, Laphygma exigua, Leucoptera scitella, Lithocolletis blancardella, Lobesia botrana, Loxostege sticticalis, Lymantria dispar, Lymantria monacha, Lyonetia clerkella, Manduca sexta, Malacosoma neustria, Mamestra brassicae, Mocis repanda, Operophthera brumata, Orgyia pseudotsugata, Ostrinia nubilalis, Pandemis heparana, Panolis flammea, Pectinophora gossypiella, Phthorimaea operculella, Phyllocnistis citrella, Pieris brassicae, Plathypena scabra, Platynota stultana, Plutella xylostella, Prays citri, Prays oleae, Prodenia sunia, Prodenia ornithogalli, Pseudoplusia includens, Rhyacionia frustrana, Scrobipalpula absoluta, Sesamia inferens, Sparganothis pilleriana, Spodoptera frugiperda, Spodoptera littoralis, Spodoptera litura, Syllepta derogata, Synanthedon myopaeformis, Thaumatopoea pityocampa, Tortrix viridana, Trichoplusia ni, Tryporyza incertulas, Zeiraphera canadensis, ferner Galleria mellonella und Sitotroga cerealella, Ephestia cautella, Tineola bisselliella;*
aus der Ordnung der Käfer *(Coleoptera)* beispielsweise *Agriotes lineatus, Agriotes obscurus, Anthonomus grandis, Anthonomus pomorum, Apion vorax, Atomaria linearis, Blastophagus piniperda, Cassida nebulosa, Cerotoma trifurcata, Ceuthorhynchus assimilis, Ceuthorhynchus napi, Chaetocnema tibialis, Conoderus vespertinus, Crioceris asparagi, Dendroctonus refipennis, Diabrotica longicornis, Diabrotica 12-punctata, Diabrotica virgifera, Epilachna varivestis, Epitrix hirtipennis, Eutinobothrus brasiliensis, Hylobius abietis, Hypera brunneipennis, Hypera postica, Ips typographus, Lema bilineata, Lema melanopus, Leptinotarsa decemlineata, Limonius californicus, Lissorhoptrus oryzophilus, Melanotus communis, Meligethes aeneus, Melolontha hippocastani, Melolontha melolontha, Oulema oryzae, Ortiorrhynchus sulcatus, Otiorrhynchus ovatus, Phaedon cochleariae, Phyllopertha horticola, Phyllophaga sp., Phyllotreta chrysocephala, Phyllotreta nemorum, Phyllotreta striolata, Popillia japonica, Psylliodes napi, Scolytus intricatus, Sitona lineatus, ferner Bruchus rufimanus, Bruchus pisorum, Bruchus lentis, Sitophilus granaria, Lasioderma serricorne, Oryzaephilus surinamensis, Rhyzopertha dominica, Sitophilus oryzae, Tribolium castaneum, Trogoderma granarium, Zabrotes subfasciatus;*
aus der Ordnung der Zweiflügler *(Diptera)* beispielsweise *Anastrepha ludens, Ceratitis capitata, Contarinia sorghicola, Dacus cucurbitae, Dacus oleae, Dasineura brassicae, Delia coarctata, Delia radicum, Hydrellia griseola, Hylemyia platura, Liriomyza sativae, Liriomyza trifolii, Mayetiola destructor, Orseolia oryzae, Oscinella frit, Pegomya hyoscyami, Phorbia antiqua, Phorbia brassicae, Phorbia coarctata, Rhagoletis cerasi, Rhagoletis pomonella, Tipula oleracea, Tipula paludosa, ferner Aedes aegypti, Aedes vexans, Anopheles maculipennis, Chrysomya bezziana, Chrysomya hominivorax, Chrysomya macellaria, Cordylobia anthropophaga, Culex pipiens, Fannia canicularis, Gasterophilus intestinalis, Glossina morsitans, Haematobia irritans, Haplodiplosis e-questris, Hypoderma lineata, Lucilia caprina, Lucilia cuprina, Lucilia sericata, Musca domestica, Muscina stabulans, Oestrus ovis, Tabanus bovinus, Simulium damnosum;*
aus der Ordnung der Thripse *(Thysanoptera)* beispielsweise *Frankliniella fusca, Frankliniella occidentalis, Frankliniella tritici, Haplothrips tritici, Scirtothrips citri, Thrips oryzae, Thrips palmi, Thrips tabaci;*
aus der Ordnung der Hautflügler *(Hymenoptera)* beispielsweise *Athalia rosae, Atta cephalotes, Atta sexdens, Atta texana, Hoplocampa minuta, Hoplocampa testudinea, Iridomyrmes humilis, Iridomyrmex purpureus, Monomorium pharaonis, Solenopsis geminata, Solenopsis invicta, Solenopsis richteri;*
aus der Ordnung der Wanzen *(Heteroptera)* beispielsweise *Acrosternum hilare, Blissus leucopterus, Cyrtopeltis notatus, Dysdercus cingulatus, Dysdercus intermedius, Eurygaster integriceps, Euschistus impictiventris, Leptoglossus phyllopus, Lygus hesperus, Lygus lineolaris, Lygus pratensis, Nezara viridula, Piesma quadrata, Solubea insularis, Thyanta perditor;*
aus der Ordnung der Pflanzensauger *(Homoptera)* beispielsweise *Acyrthosiphon O-nobrychis, Acyrthosiphon pisum, Adelges laricis, Aonidiella aurantii, Aphidula nasturtii, Aphis fabae, Aphis gossypii, Aphis pomi, Aulacorthum solani, Bemisia tabaci, Brachycaudus cardui, Brevicoryne brassicae, Dalbulus maidis, Dreyfusia nordmannianae, Dreyfusia piceae, Dysaphis radicola, Empoasca fabae, Eriosoma lanigerum, Laodelphax striatella, Macrosiphum avenae, Macrosiphum euphorbiae, Macrosiphon rosae, Megoura viciae, Metopolophium dirhodum, Myzus persicae, Myzus cerasi, Nephotettix cincticeps, Nilaparvata lugens, Perkinsiella saccharicida, Phorodon humuli, Planococcus citri, Psylla mali, Psylla piri, Psylla pyricol, Quadraspidiotus pemiciosus, Rhopalosiphum maidis, Saissetia oleae, Schizaphis graminum, Selenaspidus articulatus, Sitobion avenae, Sogatella furcifera, Toxoptera citricida, Trialeurodes abutilonea, Trialeurodes vaporariorum, Viteus vitifolii;*
aus der Ordnung der Termiten *(Isoptera)* beispielsweise *Calotermes flavicollis, Leucotermes flavipes, Macrotermes subhyalinus, Odontotermes formosanus, Reticulitermes lucifugus, Termes natalensis;*
aus der Ordnung der Geradflügler *(Orthoptera)* beispielsweise *Gryllotalpa gryllotalpa, Locusta migratoria, Melanoplus bivittatus, Melanoplus femur-rubrum, Melanoplus mexicanus, Melanoplus sanguinipes, Melanoplus spretus, Nomadacris septemfasciata, Schistocerca americana, Schistocerca peregrina, Stauronotus maroccanus, Schistocerca gregaria, ferner Acheta domestica, Blatta orientalis, Blattella germanica, Periplaneta americana;*
aus der Ordnung der Arachnoidea beispielsweise phytophage Milben wie *Aculops lycopersicae, Aculops pelekassi, Aculus schlechtendali, Brevipalpus phoenicis, Bryobia praetiosa, Eotetranychus carpini, Eutetranychus banksii, Eriophyes sheldoni, Oligonychus pratensis, Panonychus ulmi, Panonychus citri, Phyllocoptruta oleivora, Polyphagotarsonemus latus, Tarsonemus pallidus, Tetranychus cinnabarinus, Tetranychus kanzawai, Tetranchus pacificus, Tetranychus urticae, Zecken wie Amblyomma americanum, Amblyomma variegatum, Argas persicus, Boophilus annulatus, Boophilus decoloratus, Boophilus microplus, Dermacentor silvarum, Hyalomma truncatum, Ixodes ricinus, Ixodes rubicundus, Ornithodorus moubata, Otobius megnini, Rhipicephalus appendiculatus und Rhipicephalus evertsi sowie tierparasitische Milben wie Dermanyssus gallinae, Psoroptes ovis und Sarcoptes scabiei;*
aus der Klasse der Nematoden beispielsweise Wurzelgallennematoden, z.B. *Meloidogyne hapla, Meloidogyne incognita, Meloidogyne javanica, zystenbildende Nematoden, z.B. Globodera pallida, Globodera rostochiensis, Heterodera avenae, Heterodera glycines, Heterodera schachtii, migratorische Endoparasiten und semi-endoparasitische Nematoden, z.B. Heliocotylenchus multicinctus, Hirschmanniella oryzae, Hoplolaimus spp, Pratylenchus brachyurus, Pratylenchus fallax, Pratylenchus penetrans, Pratylenchus vulnus, Radopholus similis, Rotylenchus reniformis, Scutellonema bradys, Tylenchulus semipenetrans, Stock- und Blattnematoden z.B. Anguina tritici, Aphelenchoides besseyi, Ditylenchus angustus, Ditylenchus dipsaci, Virusvektoren, z.B. Longidorus spp, Trichodorus christei, Trichodorus viruliferus, Xiphinema index, Xiphinema mediterraneum.*

Die Verbindungen I können als solche, in Form ihrer Formulierungen oder den daraus bereiteten Anwendungsformen, z.B. in Form von direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Die Verbindungen I eignen sich insbesondere als Fungizide. Sie zeichnen sich aus durch eine hervorragende Wirksamkeit gegen ein breites Spektrum von pflanzenpathogenen Pilzen, insbesondere aus der Klasse der *Ascomyceten, Deuteromyceten, Oomyceten* und *Basidiomyceten.* Sie sind zum Teil systemisch wirksam und können im Pflanzenschutz als Blatt-, Beiz- und Bodenfungizide eingesetzt werden.

Besondere Bedeutung haben sie für die Bekämpfung einer Vielzahl von Pilzen an verschiedenen Kulturpflanzen wie Weizen, Roggen, Gerste, Hafer, Reis, Mais, Gras, Bananen, Baumwolle, Soja, Kaffee, Zuckerrohr, Wein, Obst- und Zierpflanzen und Gemüsepflanzen wie Gurken, Bohnen, Tomaten, Kartoffeln und Kürbisgewächsen, sowie an den Samen dieser Pflanzen.

Speziell eignen sie sich zur Bekämpfung folgender Pflanzenkrankheiten:
*Altemaria-*Arten an Gemüse und Obst,
*Bipolaris-* und *Drechslera-*Arten an Getreide, Reis und Rasen,
*Blumeria graminis* (echter Mehltau) an Getreide,
*Botrytis cinerea* (Grauschimmel) an Erdbeeren, Gemüse, Zierpflanzen und Reben,
*Bremia lactucae* an Salat,
*Erysiphe cichoracearum* und *Sphaerotheca fuliginea* an Kürbisgewächsen,
*Fusarium-* und *Verticillium*-Arten an verschiedenen Pflanzen,
*Mycosphaerella*-Arten an Getreide, Bananen und Erdnüssen,
*Peronospora*-Arten an Kohl und Zwiebelgewächsen,
*Phakospora pachyrhizi* und *P. meibomiae* an Soja,
*Phytophthora infestans* an Kartoffeln und Tomaten,
*Phytophthora capsici* an Paprika,
*Plasmopara viticola* an Reben,
*Podosphaera leucotricha* an Äpfeln,
*Pseudocercosporella* herpotrichoides an Weizen und Gerste,
*Pseudoperonospora*-Arten an Hopfen und Gurken,
*Puccinia-*Arten an Getreide,
*Pyricularia oryzae* an Reis,
*Pythium aphanidermatum* an Rasen,
*Rhizoctonia-*Arten an Baumwolle, Reis und Rasen,
*Septoria tritici* und *Stagonospora nodorum* an Weizen,
*Uncinula necator* an Reben,
*Ustilago*-Arten an Getreide und Zuckerrohr, sowie
*Venturia*-Arten (Schorf) an Äpfeln und Birnen.

Die Verbindungen I eignen sich außerdem zur Bekämpfung von Schadpilzen wie Paecilomyces variotii im Materialschutz (z.B. Holz, Papier, Dispersionen für den Anstrich, Fasern bzw. Gewebe) und im Vorratsschutz.

Darüber hinaus können die Verbindungen der Formel I auch in Kulturen, die durch Züchtung einschließlich gentechnischer Methoden, gegen Insekten- oder Pilzbefall tolerant sind, verwendet werden.

Die Verbindungen I werden angewendet, indem man die Pilze oder die vor Pilzbefall zu schützenden Pflanzen, Saatgüter, Materialien oder den Erdboden mit einer fungizid wirksamen Menge der Wirkstoffe behandelt. Die Anwendung kann sowohl vor als auch nach der Infektion der Materialien, Pflanzen oder Samen durch die Pilze erfolgen.

Die fungiziden Mittel enthalten im allgemeinen zwischen 0,1 und 95, vorzugsweise zwischen 0,5 und 90 Gew.-% Wirkstoff.

Die Aufwandmengen liegen bei der Anwendung im Pflanzenschutz je nach Art des gewünschten Effektes zwischen 0,01 und 2,0 kg Wirkstoff pro ha.

Bei der Saatgutbehandlung werden im allgemeinen Wirkstoffmengen von 0,001 bis 0,1 g, vorzugsweise 0,01 bis 0,05 g je Kilogramm Saatgut benötigt.

Bei der Anwendung im Material- bzw. Vorratsschutz richtet sich die Aufwandmenge an Wirkstoff nach der Art des Einsatzgebietes und des gewünschten Effekts. Übliche Aufwandmengen sind im Materialschutz beispielsweise 0,001 g bis 2 kg, vorzugsweise 0,005 g bis 1 kg Wirkstoff pro Kubikmeter behandelten Materials.

Die Verbindungen 1 können in die üblichen Formulierungen überführt werden, z.B. Lösungen, Emulsionen, Suspensionen, Stäube, Pulver, Pasten und Granulate. Die Anwendungsform richtet sich nach dem jeweiligen Verwendungszweck; sie soll in jedem Fall eine feine und gleichmäßige Verteilung der erfindungsgemäßen Verbindung gewährleisten.

Die Formulierungen werden in bekannter Weise hergestellt, z.B. durch Verstrecken des Wirkstoffs mit Lösungsmitteln und/oder Trägerstoffen, gewünschtenfalls unter Verwendung von Emulgiermitteln und Dispergiermitteln. Als Lösungsmittel / Hilfsstoffe kommen dafür im wesentlichen in Betracht:

Wasser, aromatische Lösungsmittel (z.B. Solvesso Produkte, Xylol), Paraffine (z.B. Erdölfraktionen), Alkohole (z.B. Methanol, Butanol, Pentanol, Benzylalkohol), Ketone (z.B. Cyclohexanon, gamma-Butyrolacton), Pyrrolidone (NMP, NOP), Acetate (Glykoldiacetat), Glykole, Dimethylfettsäureamide, Fettsäuren und Fettsäureester. Grundsätzlich können auch Lösungsmittelgemische verwendet werden,
- Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel wie nichtionogene und anionische Emulgatoren (z.B. Polyoxyethylen-Fettalkohol-Ether, Alkylsulfonate und Arylsulfonate) und Dispergiermittel wie Lignin-Sulfitablaugen und Methylcellulose.

Als oberflächenaktive Stoffe kommen Alkali-, Erdalkali-, Ammoniumsalze von Ligninsulfonsäure, Naphthalinsulfonsäure, Phenolsulfonsäure, Dibutylnaphthalinsulfonsäure, Alkylarylsulfonate, Alkylsulfate, Alkylsulfonate, Fettalkoholsulfate, Fettsäuren und sulfatierte Fettalkoholglykolether zum Einsatz, ferner Kondensationsprodukte von sulfoniertem Naphthalin und Naphthalinderivaten mit Formaldehyd, Kondensationsprodukte des Naphthalins bzw. der Naphtalinsulfonsäure mit Phenol und Formaldehyd, Polyoxyethylenoctylphenolether, ethoxyliertes Isooctylphenol, Octylphenol, Nonylphenol, Alkylphenolpolyglykolether, Tributylphenylpolyglykolether, Tristerylphenylpolyglykolether, Alkylarylpolyetheralkohole, Alkohol- und Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Ligninsulfitablaugen und Methylcellulose in Betracht.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Toluol, Xylol, Paraffin, Tetrahydronaphthalin, alkylierte Naphthaline oder deren Derivate, Methanol, Ethanol, Propanol, Butanol, Cyclohexanol, Cyclohexanon, Isophoron, stark polare Lösungsmittel, z.B. Dimethylsulfoxid, N-Methylpyrrolidon oder Wasser in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden, wie Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Die Formulierungen enthalten im allgemeinen zwischen 0,01 und 95 Gew.%, vorzugsweise zwischen 0,1 und 90 Gew.-% des Wirkstoffs. Die Wirkstoffe werden dabei in einer Reinheit von 90% bis 100%, vorzugsweise 95% bis 100% (nach NMR-Spektrum) eingesetzt.

Beispiele für Formulierungen sind: 1. Produkte zur Verdünnung in Wasser
A) Wasserlösliche Konzentrate (SL)
   10 Gew.-Teile einer erfindungsgemäßen Verbindung werden in Wasser oder einem wasserlöslichen Lösungsmittel gelöst. Alternativ werden Netzmittel oder andere Hilfsmittel zugefügt. Bei der Verdünnung in Wasser löst sich der Wirkstoff.
B) Dispergierbare Konzentrate (DC)
   20 Gew.-Teile einer erfindungsgemäßen Verbindung werden in Cyclohexanon unter Zusatz eines Dispergiermittels z.B. Polyvinylpyrrolidon gelöst. Bei Verdünnung in Wasser ergibt sich eine Dispersion.
C) Emulgierbare Konzentrate (EC)
   15 Gew.-Teile einer erfindungsgemäßen Verbindung werden in Xylol unter Zusatz von Ca-Dodecylbenzolsulfonat und Ricinusölethoxylat (jeweils 5 %) gelöst. Bei der Verdünnung in Wasser ergibt sich eine Emulsion.
D) Emulsionen (EW, EO)
   40 Gew.-Teile einer erfindungsgemäßen Verbindung werden in Xylol unter Zusatz von Ca-Dodecylbenzolsulfonat und Ricinusölethoxylat (jeweils 5 %) gelöst. Diese Mischung wird mittels einer Emulgiermaschine (Ultraturax) in Wasser eingebracht und zu einer homogenen Emulsion gebracht. Bei der Verdünnung in Wasser ergibt sich eine Emulsion.
E) Suspensionen (SC, OD)
   20 Gew.-Teile einer erfindungsgemäßen Verbindung werden unter Zusatz von Dispergier- und Netzmitteln und Wasser oder einem organischen Lösungsmittel in einer Rührwerkskugelmühle zu einer feinen Wirkstoffsuspension zerkleinert. Bei der Verdünnung in Wasser ergibt sich eine stabile Suspension des Wirkstoffs.
F) Wasserdispergierbare und wasserlösliche Granulate (WG, SG)
   50 Gew.-Teile einer erfindungsgemäßen Verbindung werden unter Zusatz von Dispergier- und Netzmitteln fein gemahlen und mittels technischer Geräte (z.B. Extrusion, Sprühturm, Wirbelschicht) als wasserdispergierbare oder wasserlösliche Granulate hergestellt. Bei der Verdünnung in Wasser ergibt sich eine stabile Dispersion oder Lösung des Wirkstoffs.
G) Wasserdispergierbare und wasserlösliche Pulver (WP, SP)
   75 Gew.-Teile einer erfindungsgemäßen Verbindung werden unter Zusatz von Dispergier- und Netzmitteln sowie Kieselsäuregel in einer Rotor-Strator Mühle vermahlen. Bei der Verdünnung in Wasser ergibt sich eine stabile Dispersion oder Lösung des Wirkstoffs.

### 2. Produkte für die Direktapplikation

H) Stäube (DP)
   5 Gew.Teile einer erfindungsgemäßen Verbindung werden fein gemahlen und mit 95 % feinteiligem Kaolin innig vermischt. Man erhält dadurch ein Stäubemittel.
I) Granulate (GR, FG, GG, MG)
   0.5 Gew-Teile einer erfindungsgemäßen Verbindung werden fein gemahlen und mit 95.5 % Trägerstoffe verbunden. Gängige Verfahren sind dabei die Extrusion, die Sprühtrocknung oder die Wirbelschicht. Man erhält dadurch ein Granulat für die Direktapplikation.
J) ULV- Lösungen (UL)
   10 Gew.-Teile einer erfindungsgemäßen Verbindung werden in einem organischen Lösungsmittel z.B. Xylol gelöst. Dadurch erhält man ein Produkt für die Direktapplikation.

Die Wirkstoffe können als solche, in Form ihrer Formulierungen oder den daraus bereiteten Anwendungsformen, z.B. in Form von direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Wässrige Anwendungsformen können aus Emulsionskonzentraten, Pasten oder netzbaren Pulvern (Spritzpulver, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermitttel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser, geeignet sind.

Die Wirkstoffkonzentrationen in den anwendungsfertigen Zubereitungen können in größeren Bereichen variiert werden. Im allgemeinen liegen sie zwischen 0,0001 und 10%, vorzugsweise zwischen 0,01 und 1%.

Die Wirkstoffe können auch mit gutem Erfolg im Ultra-Low-Volume-Verfahren (ULV) verwendet werden, wobei es möglich ist, Formulierungen mit mehr als 95 Gew.-% Wirkstoff oder sogar den Wirkstoff ohne Zusätze auszubringen.

Zu den Wirkstoffen können Öle verschiedenen Typs, Netzmittel, Adjuvants, Herbizide, Fungizide, andere Schädlingsbekämpfungsmittel, Bakterizide, gegebenenfalls auch erst unmittelbar vor der Anwendung (Tankmix), zugesetzt werden. Diese Mittel können zu den erfindungsgemäßen Mitteln im Gewichtsverhältnis 1:10 bis 10:1 zugemischt werden.

Die erfindungsgemäßen Mittel können in der Anwendungsform als Fungizide auch zusammen mit anderen Wirkstoffen vorliegen, der z.B. mit Herbiziden, Insektiziden, Wachstumsregulatoren, Fungiziden oder auch mit Düngemitteln. Beim Vermischen der Verbindungen I. bzw. der sie enthaltenden Mittel in der Anwendungsform als Fungizide mit anderen Fungiziden erhält man in vielen Fällen eine Vergrößerung des fungiziden Wirkungsspektrums.

Die folgende Liste von Fungiziden, mit denen die erfindungsgemäßen Verbindungen gemeinsam angewendet werden können, soll die Kombinationsmöglichkeiten erläutern, nicht aber einschränken:
- Acylalanine wie Benalaxyl, Metalaxyl, Ofurace, Oxadixyl,
- Aminderivate wie Aldimorph, Dodine, Dodemorph, Fenpropimorph, Fenpropidin, Guazatine, Iminoctadine, Spiroxamin, Tridemorph
- Anilinopyrimidine wie Pyrimethanil, Mepanipyrim oder Cyrodinyl,
- Antibiotika wie Cycloheximid, Griseofulvin, Kasugamycin, Natamycin, Polyoxin oder Streptomycin,
- Azole wie Bitertanol, Bromoconazol, Cyproconazol, Difenoconazole, Dinitroconazol, Enilconazol, Epoxiconazol, Fenbuconazol, Fluquiconazol, Flusilazol, Flutriafol, Hexaconazol, Imazalil, Ipconazol, Metconazol, Myclobutanil, Penconazol, Propiconazol, Prochloraz, Prothioconazol, Simeconazol, Tebuconazol, Tetraconazol, Triadimefon, Triadimenol, Triflumizol, Triticonazol,
- Dicarboximide wie Iprodion, Myclozolin, Procymidon, Vinclozolin,
- Dithiocarbamate wie Ferbam, Nabam, Maneb, Mancozeb, Metam, Metiram, Propineb, Polycarbamat, Thiram, Ziram, Zineb,
- Heterocylische Verbindungen wie Anilazin, Benomyl, Boscalid, Carbendazim, Carboxin, Oxycarboxin, Cyazofamid, Dazomet, Dithianon, Famoxadon, Fenamidon, Fenarimol, Fuberidazol, Flutolanil, Furametpyr, Isoprothiolan, Mepronil, Nuarimol, Penthiopyrad, Probenazol, Proquinazid, Pyrifenox, Pyroquilon, Quinoxyfen, Silthiofam, Thiabendazol, Thifluzamid, Thiophanat-methyl, Tiadinil, Tricyclazol, Triforine, 5-Chlor-7-(4-methylpiperidin-1-yl)-6-(2,4,6-trifluorphenyl)-[1,2,4]triazolo[1,5-a]pyrimidin,
- Kupferfungizide wie Bordeaux Brühe, Kupferacetat, Kupferoxychlorid, basisches Kupfersulfat,
- Nitrophenylderivate, wie Binapacryl, Dinocap, Dinobuton, Nitrophthalisopropyl,
- Phenylpyrrole wie Fenpiclonil oder Fludioxonil,
- Schwefel
- Sonstige Fungizide wie Acibenzolar-S-methyl, Benthiavalicarb, Carpropamid, Chlorothalonil, Cyflufenamid, Cymoxanil, Diclomezin, Diclocymet, Diethofencarb, Edifenphos, Ethaboxam, Fenhexamid, Fentin-Acetat, Fenoxanil, Ferimzone, Fluazinam, Fosetyl, Fosetyl-Aluminium, Iprovalicarb, Hexachlorbenzol, Metrafenon, Pencycuron, phosphorige Säure, Propamocarb, Phthalid, Tolclofos-methyl, Quintozene, Zoxamid
- Strobilurine wie Azoxystrobin, Dimoxystrobin, Enestroburin, Fluoxastrobin, Kresoxim-methyl, Metominostrobin, Orysastrobin, Picoxystrobin, Pyraclostrobin oder Trifloxystrobin,
- Sulfensäurederivate wie Captafol, Captan, Dichlofluanid, Folpet, Tolylfluanid
- Zimtsäureamide und Analoge wie Dimethomorph, Flumetover oder Flumorph.

### Synthesebeispiele:

### Herstellung der Ausgangsstoffe

### Beispiel A: 3-(2-Chlor-6-fluorphenyl)-2, 4-dichlor-6-([1, 2, 4]triazol-1-yl)-pyridin (Tabelle B, Nr. V-2)

Aa) 2-Chlor-3-(2-chlor-6-fluorphenyl)-4, 6-dihydroxypyridin
   94,0 g (0,55 Mol) 2-Chlor-6-fluorphenylacetonitril und 160 ml Malonsäuredichlorid wurden 8 Tage bei Raumtemperatur gerührt, wobei die Mischung langsam erstarrte. Zur Aufarbeitung wurde mit Cyclohexan verrührt, abfiltriert und der Rückstand zweimal mit je 25 ml Methylenchlorid gewaschen. Der Rückstand wurde dann unter Eiskühlung in 20%iger Natronlauge gelöst und zweimal mit Methylenchlorid gewaschen. Die wäßrige Phase wurde nun mit konzentrierter Salzsäure unter Eiskühlung angesäuert. Der abfiltrierte Rückstand wurde mit wenig Wasser gewaschen und im Vakuum getrocknet. Ausbeute 150 g.
Ab) 3-(2-Chlor-6-fluorphenyl)-2,4, 6-trichlorpyridin
   40,0 g (0,146 Mol) 2-Chlor-3-(2-chlor-6-fluorphenyl)-4, 6-dihydroxypyridin und 27,9 g (0,292 Mol) Trimethylaminhydrochlorid wurden zu 89,5 g (0,584 Mol) Phosphorylchlorid gegeben und in einem Tantal-Autoklaven 12 h unter Eigendruck auf 120°C erhitzt. Das Reaktionsgemisch wurde mit Toluol aus dem Autoklaven gespült und im Vakuum eingeengt. Dabei blieb ein Rückstand von 100 g zurück. Dieses Rohprodukt wurde durch Säulenchromatographie an Kieselgel mit Cyclohexan/Essigsäureethylester (5:1) gereinigt. Das Produkt wurde zum Schluß mit Pentan ausgerührt. ¹H-NMR (CDCl₃): 7,15 (t); 7,37 (d); 7,44 (m); 7,50 (s).
Ac) 3-(2-Chlor-6-fluorphenyl)-2, 4-dichlor-6-([1,2,4]triazol-1-yl)-pyridin
   3,00 g (9,6 mmol) 3-(2-Chlor-6-fluorphenyl)-2, 4, 6-trichlorpyridin und 0,70 g (9,6 mmol) [1,2, 4]Triazol gelöst in 30 ml Dimethylformamid wurden portionsweise mit 0,42 g (11,0 mmol) 60%igem Natriumhydrid in Paraffin versetzt. Die Mischung wurde 3 d bei Raumtemperatur nachgerührt, auf 50 ml Natriumdihydrogenphosphat-Lösung gegeben, dreimal mit je 50 ml Methyl-tert.-butylether extrahiert, über Natriumsulfat getrocknet und im Vakuum eingeengt. Das Rohprodukt wurde mit Cyclohexan/Essigsäureethyl- ester an Kieselgel chromatographiert. Ausbeute 1,5 g farbloser Feststoff vom Fp. 210°C.

### Beispiel B: 3-(2-Chlor-6-fluorphenyl)-2, 4-dichlor-6-(pyridin-2-yl)-pyridin (Tabelle B, Nr. V-3)

64,8 g (0,26 Mol) 3-(2-Chlor-6-fluorphenyl)-2, 4, 6-trichlorpyridin (s. Beispiel Ab), 83,6 g (0,29 Mol) 2-Tri-n-butylstannylpyridin und 4,8 g (5,2 mmol) Tetrakistriphenylphosphin-Palladium(0) gelöst in 600 ml Dimethylformamid und 400 ml 1,4-Dioxan wurden 8 h unter Rückfluß gekocht und über Nacht bei Raumtemperatur nachgerührt. Nach dem Entfernen der flüchtigen Bestandteile im Vakuum wurden 400 ml Wasser zugegeben, fünfmal mit je 150 ml Methyl-tert.-butylether extrahiert, die vereinigten Extrakte einmal mit 100 ml gesättigter Natriumchlorid-Lösung gewaschen, über Natriumsulfat getrocknet und im Vakuum eingeengt. Das Rohprodukt wurde mit Cyclohexan/Essigsäureethylester an Kieselgel chromatographiert und mit n-Pentan ausgerührt. Ausbeute 36,0 g farbloser Feststoff vom Fp. 45°C.

### Beispiel C: 3-(2-Chlor-6-fluorphenyl)-2, 4-dichlor-6-(pyrimidin-2-yl)-pyridin (Tabelle B, Nr. V-6)

14,6 g (0,047 Mol) 3-(2-Chlor-6-fluorphenyl)-2, 4, 6-trichlorpyridin (s. Beispiel Ab), 20,0 g (0,051 Mol) 2-Tri-n-butylstannylpyrimidin und 1,1 g (0,94 mmol) Tetrakistriphenylphosphin-Palladium(0) gelöst in 70 ml Dimethylformamid und 50 ml 1,4-Dioxan wurden 10 h bei 110°C gehalten. Nach dem Entfernen der flüchtigen Bestandteile im Vakuum wurde mit Cyclohexan/Essigsäureethylester an Kieselgel aufgereinigt. Ausbeute 2,0 g gelbe Kristalle (Fp. 147°C).

### Beispiel D: 5-Brom-3-(2-chlor-6-fluorphenyl)-2, 4, 6-trichlorpyridin (Tabelle B, Nr. V-8)

2,0 g (6,4 mmol) 3-(2-Chlor-6-fluorphenyl)-2, 4, 6-trichlorpyridin (s. Beispiel Ab), gelöst in 25 ml Tetrahydrofuran wurden bei Raumtemperatur mit 2,0 g (22,4 mmol) N, N-Dimethylaminoethanol versetzt und auf -78°C abgekühlt. Dann wurden 26 ml (41,9 mmol) n-Butyllithium (1,6 M in Hexan) zugetropft und 1,5 h bei -75°C bis -50°C nachgerührt. Dann wurden 4,0 g (15,4 mmol) 1, 2-Dibromtetrafluorethan zugetropft, 1 h bei -50°C bis -70°C nachgerührt, auf Raumtemperatur aufgetaut, in 70 ml 10%ige Salzsäure gegeben, dreimal mit je 80 ml Methyl-tert.-butylether extrahiert, über Natriumsulfat getrocknet, im Vakuum eingeengt und mit Hexan ausgerührt. Ausbeute 1,6 g. ¹H-NMR (CDCl₃) δ = 7,15 (m); 7,40 (m).

### Beispiel E: 5-Brom-3-(2-chlor-6-fluorphenyl)-2, 4-dichlor-6-methylthiopyridin

1,6 g (4,1 mmol) 5-Brom-3-(2-chlor-6-fluorphenyl)-2, 4, 6-trichlorpyridin (Beispiel D) und 300 mg (4,1 mmol) Natriummethylthiolat gelöst in 30 ml Tetrahydrofuran wurden 5 h unter Rückfluß gekocht. Die Mischung wurde in 50 ml Wasser gegeben, dreimal mit je 50 ml Diethylether extrahiert, über Natriumsulfat getrocknet, im Vakuum eingeengt und mit Cyclohexan/Essigsäureethylester an Kieselgel chromatographiert. Ausbeute 1,13 g Öl. ¹H-NMR (COCl₃) δ = 2,60 (s); 7,15 (m); 7,40 (m).

### Beispiel F: 5-Brom-3-(2-chlor-6-fluorphenyl)-2, 4-dichlor-6-methylsulfonylpyridin

1,1 g (2,7 mmol) 5-Brom-3-(2-chlor-6-fluorphenyl)-2, 4-dichlor-6-methylthiopyridin (Beispiel E) gelöst in 10 ml Eisessig wurden bei Raumtemperatur mit 50 mg (0,1 mmol) Natriumwolframat versetzt und dann 0,78 g (6,9 mmol) 30%iges Wasserstoffperoxid zugetropft. Die Mischung wurde über Nacht bei Raumtemperatur nachgerührt, in 50 ml Eiswasser gegeben, dreimal mit je 50 ml Methyl-tert.-butylether extrahiert, einmal mit 20 ml Natriumcarbonat-Lösung gewaschen, über Natriumsulfat getrocknet und im Vakuum eingeengt. Ausbeute 1,07 g. ¹H-NMR (CDCl₃) δ = 3,50 (s); 7,17 (m); 7,40 (m).

### Beispiel G: 5-Brom-3-(2-chlor-6-fluorphenyl)-6-cyano-2, 4-dichlor-pyridin

1,65 g (3,8 mmol) 5-Brom-3-(2-chlor-6-fluorphenyl)-2, 4-dichlor-6-methylsulfonylpyridin (Beispiel F), gelöst in 50 ml Acetonitril wurden bei Raumtemperatur mit einer Spatelspitze (ca. 10 mg) Kronenether (18-Krone-6) und 1 ml Dimethylsulfoxid versetzt und auf 60°C erwärmt. Nun wurden 500 mg (7,6 mmol) Kaliumcyanid portionsweise innerhalb von 6 h bei dieser Temperatur zugegeben. Die Mischung wurde über Nacht bei Raumtemperatur nachgerührt und direkt mit Cyclohexan/Essigsäureethylester an Kieselgel chromatographiert. Ausbeute 0,57 g Feststoff, Fp. 90°C. ¹H-NMR (CDCl₃) δ = 7,18 (m); 7,40 (m).

### Beispiel H: 3-Brom-5-(2-chlor-6-fluorphenyl)-4, 6-dichlor-2-pyridincarboxamidoxim (Tabelle B, V-14)

300 mg (0,79 mmol) 5-Brom-3-(2-chlor-6-fluorphenyl)-6-cyano-2, 4-dichlor-pyridin (Beispiel G), 70 mg (1,0 mmol) Hydroxylaminhydrochlorid und 50 mg (0,63 mmol) Natriumhydrogencarbonat gelöst in 10 ml Ethanol und 2 ml Wasser wurden bei Raumtemperatur über Nacht nachgerührt und dann nochmals 70 mg (1,0 mmol) Hydroxylaminhydrochlorid und 50 mg (0,63 mmol) Natriumhydrogencarbonat zugegeben und wiederum über Nacht bei Raumtemperatur nachgerührt. Die Mischung wurde im Vakuum eingeengt, 30 ml Wasser zugegeben, dreimal mit je 50 ml Methyl-tert.-butylether extrahiert, über Natriumsulfat getrocknet und im Vakuum eingeengt. Ausbeute 340 mg, Fp. 132°C. ¹H-NMR (CDCl₃) δ = 5,40 (verbr.); 7,18 (m); 7,40 (m); 7,60 (verbr.).

Analog zu den Beispielen A bis H konnten die Zwischenprodukte V der Tabelle B hergestellt werden. Hierzu wurde 3-(2-Chlor-6-fluorphenyl)-2, 4, 6-trichlorpyridin (s. Beispiel Ab) mit den entsprechenden Nukleophilen umgesetzt.

**Tabelle B: Zwischenprodukte V**

| Nr | Formel | Phys. Daten |
|---|---|---|
| V-1 | 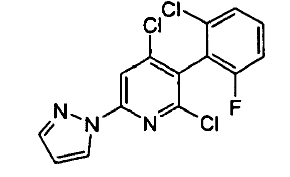 | Fp. 55 °C |
| V-2 | 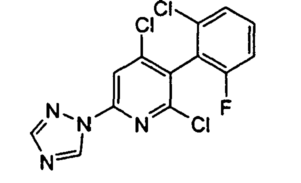 | Fp. 210 °C |
| V-3 | 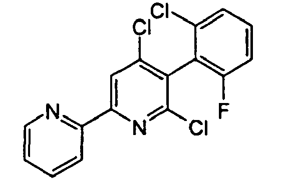 | Fp. 45 °C |
| V-4 | 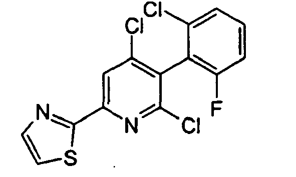 | ¹H-NMR (CDCl₃) δ = 7,15-7,50 (m); 7,55 (6); 7,97 (d); 8,30 (s). |
| V-5 | 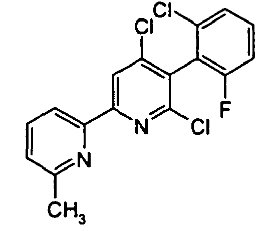 | Fp. 152 °C |
| V-6 | 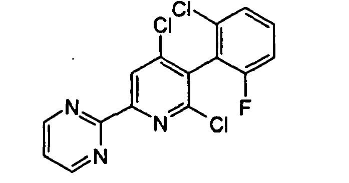 | Fp. 147 °C |
| V-7 | 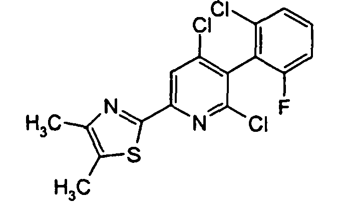 | Fp. 153 °C |
| V-8 | 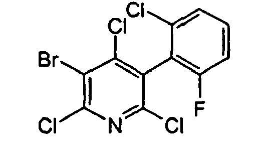 | Fp. 118-120 °C |
| V-9 | 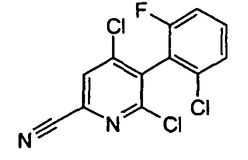 | Fp. 56 °C |
| V-10 | 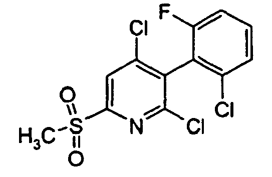 | Fp. 94 °C |
| V-11 | 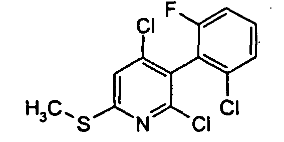 | Fp. 105 °C |
| V-12 | 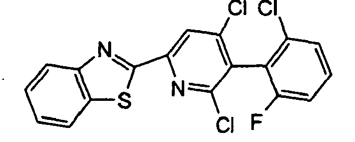 | Fp. 199 °C |
| V-13 | 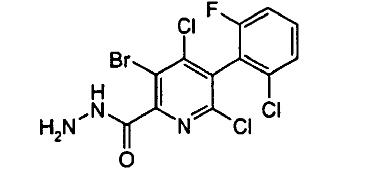 | Fp. 153-156 °C |
| V-14 | 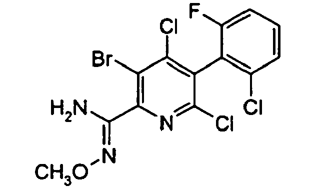 | Fp. 132 °C |

### Wirkstoffsynthesen

### Beispiel 1: 2-Chlor-3-(2-chlor-6-fluorphenyl)-4-(2-propylamino)-6-([1, 2, 4]triazol-1-yl)-pyridin und 4-Chlor-3-(2-chlor-6-fluorphenyl)-2-(2-propylamino)-6-([1, 2, 4]triazol-1-yl)-pyridin (Tabelle C, Nr. I-13, I-14)

700 mg (2,0 mmol) 3-(2-Chlor-6-fluorphenyl)-2, 4-dichlor-6-([1, 2, 4]triazolyl)-pyridin (Beispiel A) und 15 g Diisopropylamin wurden 12 h im Edelstahlautoklaven auf 160°C erhitzt. Die flüchtigen Anteile wurden im Vakuum entfernt und der Rückstand mit Cyc-Iohexan/Essigsäureethylester an Kieselgel chromatographiert. Dabei konnten 240 mg 2-Chlor-3-(2-chlor-6-fluorphenyl)-4-(2-propylamino)-6-([1, 2, 4]triazolyl)-pyridin (Fp. 152°C) und 310 mg 4-Chlor-3-(2-chlor-6-fluorphenyl)-2-(2-propylamino)-6-([1, 2, 4]triazolyl)-pyridin (Fp. 152°C) isoliert werden.

### Beispiel 2: 2-Chlor-3-(2-chlor-6-fluorphenyl)-4-(2-butylamino)-6-(pyridin-2-yl)-pyridin und 4-Chlor-3-(2-chlor-6-fluorphenyl)-2-(2-butylamino)-6-(pyridin-2-yl)-pyridin (Tabelle B, Nr. I-43, I-44)

2,0 g (5,7 mmol) 3-(2-Chlor-6-fluorphenyl)-2, 4-dichlor-6-(pyridin-2-yl)-pyridin (Beispiel B) und 15 g racemisches 2-Butylamin wurden 12 h im Edelstahlautoklaven auf 180°C erhitzt. Die flüchtigen Anteile wurden im Vakuum entfernt und der Rückstand mit Cyclohexan/Essigsäureethylester an Kieselgel chromatographiert. Dabei konnten 450 mg 4-Chlor-3-(2-chlor-6-fluorphenyl)-2-(2-butylamino)-6-(pyridin-2-yl)-pyridin (¹H-NMR (CDCl₃) δ = 0,90 (m); 1,20 (m); 1,55 (m); 3,80 (verbr.); 4,27 (m); 7,15-7,45 (m); 7,80 (t); 7,90 (s); 8,42 (d); 8,67 (m)) und 370 mg 2-Chlor-3-(2-chlor-6-fluorphenyl)-4-(2-butylamino)-6-(pyridin-2-yl)-pyridin (¹H-NMR (CDCl₃) δ = 0,90 (m); 1,20 (m); 1,50 (m); 3,67 (verbr.); 3,77 (m); 7,15-7,45 (m); 7,75 (s); 7,82 (t); 8,42 (d); 8,67 (m)) isoliert werden. Die Verbindungen liegen als Rotameren-Gemische vor.

### Beispiel 3: 2-Chlor-3-(2-chlor-6-fluorphenyl)-4-(4-methylpiperidinyl)-6-(pyrimidin-2-yl)-pyridin und 4-Chlor-3-(2-chlor-6-fluorphenyl)-2-(4-methylpiperidinyl)-6-(pyrimidin-2-yl)-pyridin (Tabelle B, Nr. I-53, I-54)

500 mg (1,4 mmol) 3-(2-Chlor-6-fluorphenyl)-2, 4-dichlor-6-(pyrimidin-2-yl)-pyridin (Beispiel C) und 15 g 4-Methylpiperidin wurden 12 h im Edelstahlautoklaven auf 140°C erhitzt. Die flüchtigen Anteile wurden im Vakuum entfernt und der Rückstand mit Cyclohexan/Essigsäureethylester an Kieselgel chromatographiert. Dabei konnten 220 mg 4-Chlor-3-(2-chlor-6-fluorphenyl)-2-(4-methylpiperidinyl)-6-(pyrimidin-2-yl)-pyridin (Fp. 129°C) und 60 mg 2-Chlor-3-(2-chlor-6-fluorphenyl)-4-(4-methylpiperidinyl)-6-(pyrimidin-2-yl)-pyridin (Fp. 129°C) isoliert werden.

**Tabelle C, Wirkstoffe**

| **Nr.** | **Formel** | **Phys. Daten** |
|---|---|---|
| I-1 | 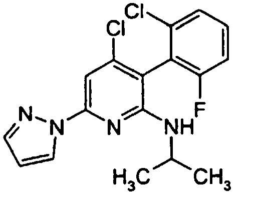 | ¹H-NMR (CDCl₃) δ = 1,20 (t); 3,87 (d); 4,30 (m); 6,45 (m); 7,20 (t); 7,40 (m); 7,73 (m); 8,52 (m). |
| I-2 | 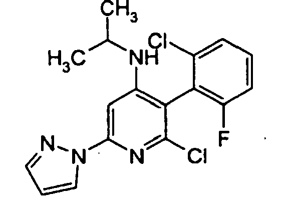 | ¹H-NMR (CDCl₃) δ = 1,22 (m); 3,75 (m); 3,88 (m); 6,45 (m); 7,15-7,40 (m); 7,75 (m); 8,55 (m). |
| I-3 | 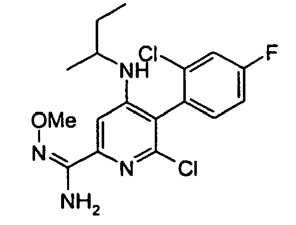 | |
| I-4 | 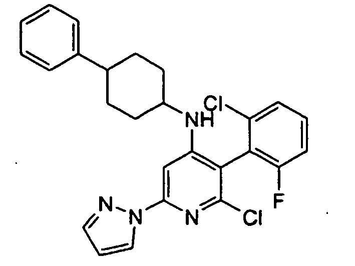 | Fp. 170 °C |
| I-5 | 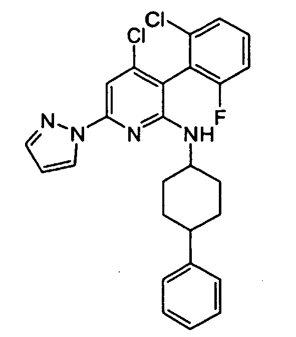 | Fp. 78 °C |
| I-6 | 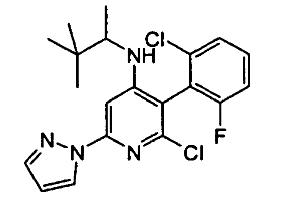 | ¹H-NMR (CDCl₃) δ = 0,85 (s); 1,05 (d); 3,50 (m); 3,88 (d); 6,42 (t); 7,20 (m); 7,40 (m); 7,75 (d); 8,55 (d) [Rotamerengemisch]. |
| I-7 | 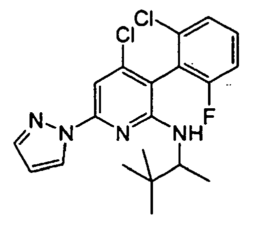 | ¹H-NMR (CDCl₃) δ = 0,85 und 0,87 (s); 1,05 und 1,06 (d); 3,90 (d); 4,20 (m); 6,47 (m); 7,20 (m); 7,40 (m); 7,73 (m); 8,50 (m) [Rotamerengemisch]. |
| I-8 | 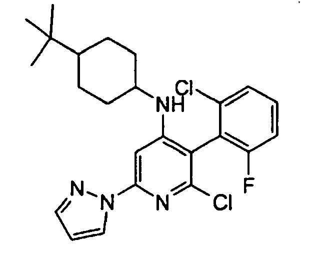 | ¹H-NMR (CDCl₃) δ = 0,75-2,25 (m); 3,22 (m); 3,78 (d); 6,45 (m); 7,18 (m); 7,40 (m); 7,74 (m); 8,55 (m) |
| I-9 | 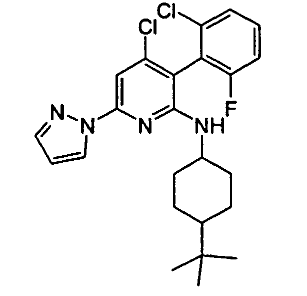 | Fp. 126 °C |
| I-10 | 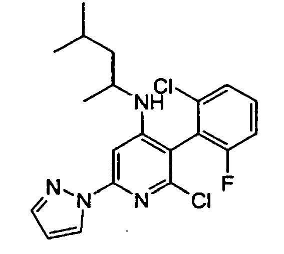 | ¹H-NMR (CDCl₃) δ = 0,85 und 0,87 (d); 1,16 und 1,18 (d); 1,30 (m); 1,60 (m); 3,75 (m); 6,45 (m); 7,20 (m); 7,40 (m); 7,75 (m); 8,55 (m) [Rotamerengemisch]. |
| I-11 | 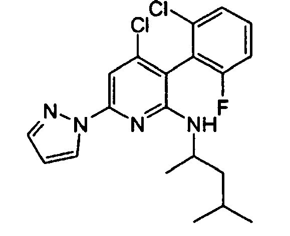 | ¹H-NMR (CDCl₃) δ = 0,88 (m); 1,13 (m); 1,30 (m); 1,60 (m); 3,80 (m); 4,33 (m); 6,45 (m); 7,20 (m); 7,40 (m); 7,73 (m); 8,55 (m) [Rotamerengemisch]. |
| I-12 | 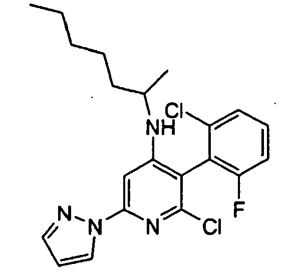 | ¹H-NMR (CDCl₃) δ = 0,88 (m); 1,16 (m); 1,25 (m); 1,40 (m); 1,63 (m); 3,70 (m); 6,45 (m); 7,20 (m); 7,40 (m); 7,73 (m); 8,55 (m) [Rotamerengemisch]. |
| I-13 | 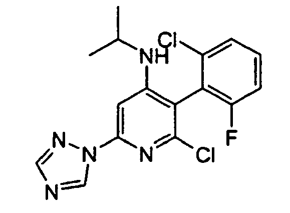 | Fp. 152 °C |
| I-14 | 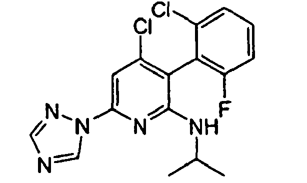 | Fp. 152 °C |
| I-15 | 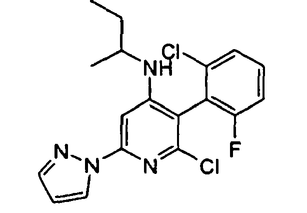 | Fp. 108 °C |
| I-16 | 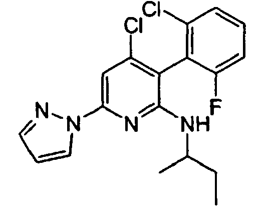 | Fp. 114 °C |
| I-17 | 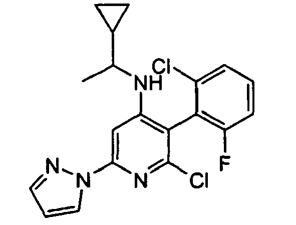 | ¹H-NMR (CDCl₃) δ = 0,25 (m); 0,47 (m); 0,80 (m); 1,25 (m); 3,23 (m); 3,95 (m); 6,45 (m); 7,20 (m); 7,40 (m); 7,73 (m); 8,55 (m) [Rotamerengemisch]. |
| I-18 | 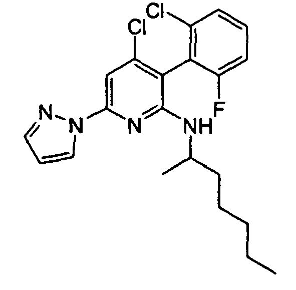 | ¹H-NMR (CDCl₃) δ = 0,88 (m); 1,15 (m); 1,30 (m); 1,50 (m); 3,85 (m); 4,20 (m); 6,45 (m); 7,20 (m); 7,40 (m); 7,73 (m); 8,55 (m) [Rotamerengemisch]. |
| I-19 | 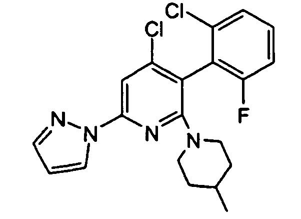 | Fp. 120 °C |
| I-20 | 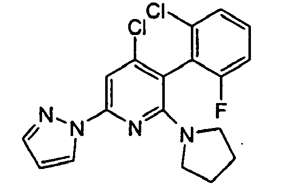 | Fp. 158 °C |
| I-21 | 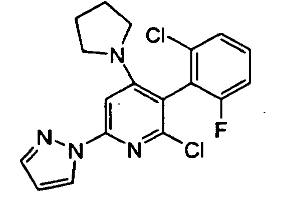 | Fp. 161 °C |
| I-22 | 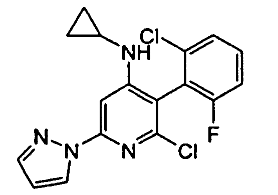 | Fp. 164 °C |
| I-23 | 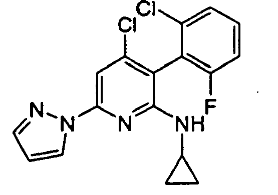 | Fp. 103 °C |
| I-24 | 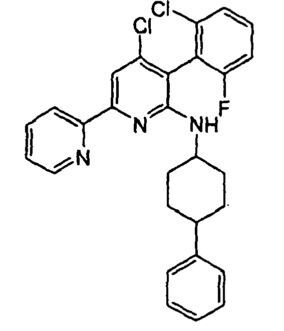 | Fp. 162 °C |
| I-25 | 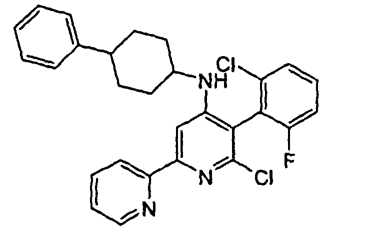 | Fp. 181 °C |
| I-26 | 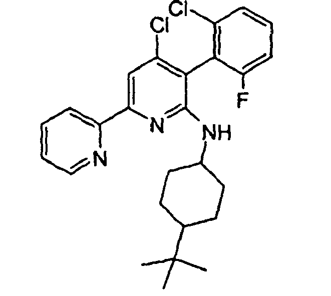 | ¹H-NMR (CDCl₃) δ = 0,85 (s); 0,95-2,25 (m); 3,80 (d); 4,00 (m); 7,10 (t); 7,37 (m); 7,85 (t); 7,90 (s); 8,43 (d); 8,63 (m). |
| I-27 | 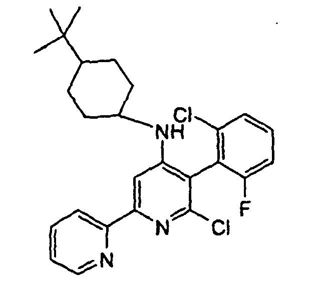 | Fp. 163 °C |
| I-28 | 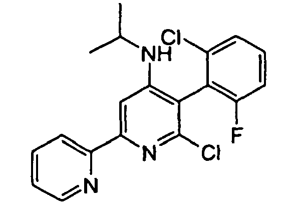 | ¹H-NMR (CDCl₃) δ = 1,20 (m); 3,72 (d); 3,96 (m); 7,13 (t); 7,33 (m); 7,40 (m); 7,75 (s); 7,80 (t); 8,45 (d); 8,70 (m). |
| I-29 | 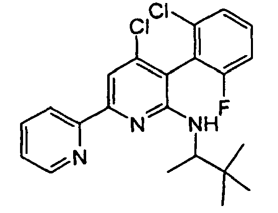 | ¹H-NMR (CDCl₃) δ = 0,90 (s); 1,10 (m); 3,85 (d); 4,30 (m); 7,20 (m); 7,33 (m); 7,43 (m); 7,83 (m); 7,90 (s); 8,45 (d); 8,70 (m). |
| I-30 | 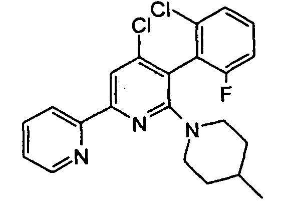 | ¹H-NMR (CDCl₃) δ = 0,88 (d); 0,95 (m); 1,50 (m); 2,80 (m); 7,10 (m); 7,33 (m); 7,80 (t); 8,10 (s); 8,45 (d); 8,70 (m). |
| I-31 | 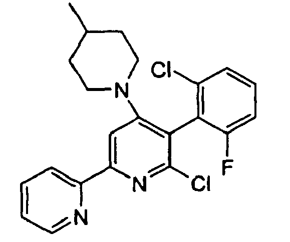 | Fp. 119 °C |
| 1-32 | 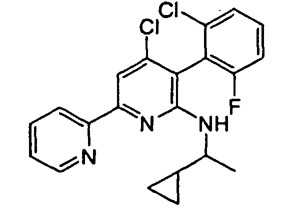 | |
| I-33 | 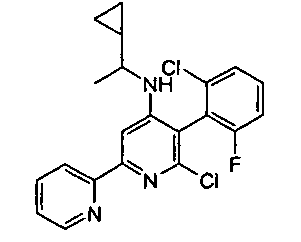 | ¹H-NMR (CDCl₃) δ = 0,25 (m); 0,45 (m); 0,80 (m); 1,22 (m); 3,30 (m); 3,90 (m); 7,13-7,45 (m); 7,70 (s); 7,80 (t); 8,45 (d); 8,70 (m) (Rotamerengemisch). |
| I-34 | 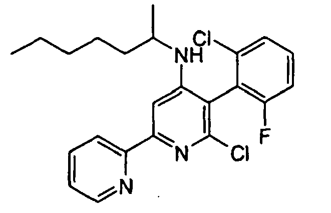 | ¹H-NMR (CDCl₃) δ = 0,82 (m); 1,10-1,50 (m); 3,70 (m); 3,80 (m); 7,13-7,45 (m); 7,75 (s); 7,80 (t); 8,45 (d); 8,67 (m). |
| I-35 | 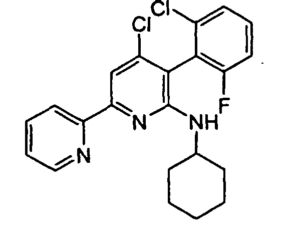 | ¹H-NMR (CDCl₃) δ = 1,00-1,75 (m); 2,10 (m); 3,90 (d); 4,15 (m); 7,13-7,45 (m); 7,80 (t); 7,87 (s); 8,40 (d); 8,70 (m). |
| I-36 | 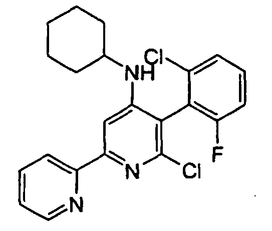 | Fp. 83 °C |
| I-37 | 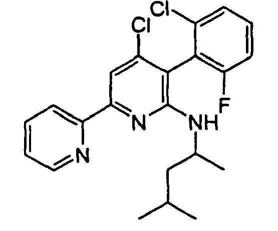 | ¹H-NMR (CDCl₃) δ = 0,90 (m); 1,18 (m); 1,25 (m); 1,40 (m); 1,67 (m); 3,75 (m); 4,45 (m); 7,15-7,45 (m); 7,80 (t); 7,90 (s); 8,42 (d); 8,67 (m). |
| I-38 | 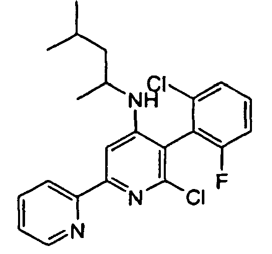 | ¹H-NMR (CDCl₃) δ = 0,90 (m); 1,18 (m); 1,25-1,40 (m); 1,62 (m); 3,63 (m); 3,85 (m); 7,15-7,45 (m); 7,76 (s); 7,80 (t); 8,42 (d); 8,67 (m). |
| I-39 | 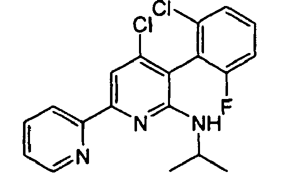 | Fp. 67 °C |
| I-40 | 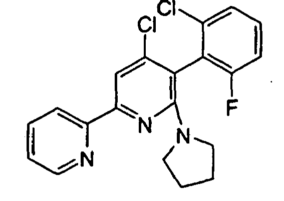 | Fp. 155°C |
| I-41 | 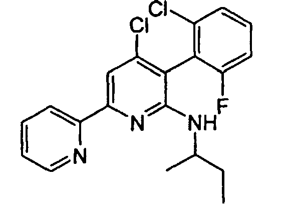 | ¹H-NMR (CDCl₃) δ = 0,90 (m); 1,20 (m); 1,55 (m); 3,80 (verbr.); 4,27 (m); 7,15-7,45 (m); 7,80 (t); 7,90 (s); 8,42 (d); 8,67 (m). |
| I-42 | 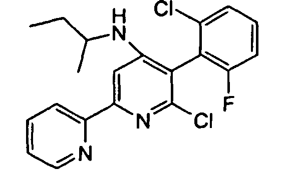 | ¹H-NMR (CDCl₃) δ = 0,90 (m); 1,20 (m); 1,50 (m); 3,67 (verbr.); 3,77 (m); 7,15-7,45 (m); 7,75 (s); 7,82 (t); 8,42 (d); 8,67 (m). |
| I-43 | 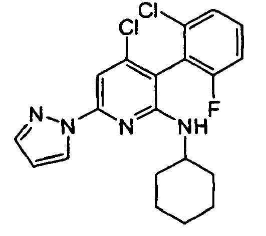 | Fp. 152 °C |
| I-44 | 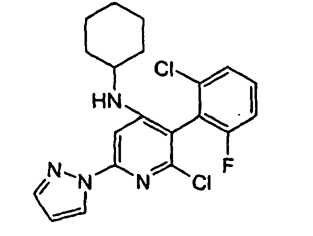 | Fp. 112 °C |
| I-45 | 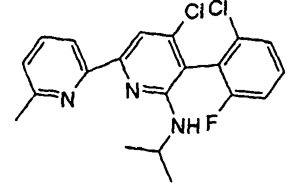 | Fp. 131 °C |
| I-46 | 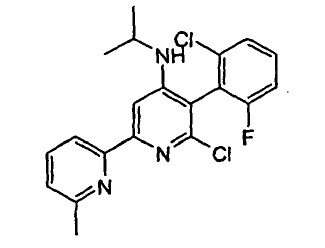 | ¹H-NMR (CDCl₃) δ = 1,22 (t); 2,67 (s); 3,63 (m); 3,75 (m); 7,15-7,45 (m); 7,70 (t); 7,77 (s); 8,20 (d). |
| I-47 | 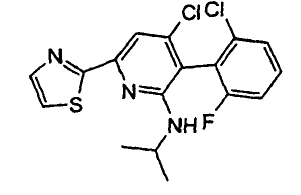 | ¹H-NMR (CDCl₃) δ = 1,22 (t); 3,90 (d); 4,33 (m); 7,20 (t); 7,40 (m); 7,63 (s); 7,90 (d). |
| I-48 | 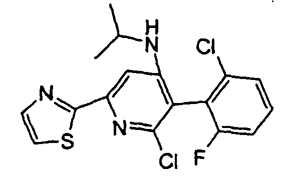 | Fp. 78°C |
| I-49 | 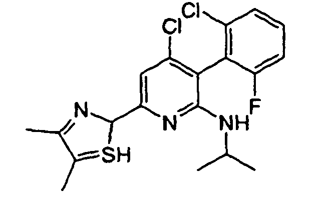 | Fp. 118°C |
| I-50 | 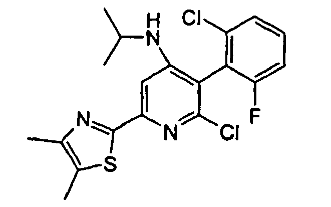 | Fp. 183 °C |
| I-51 | 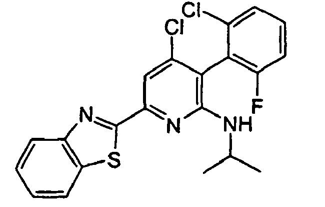 | Fp. 119 °C |
| I-52 | 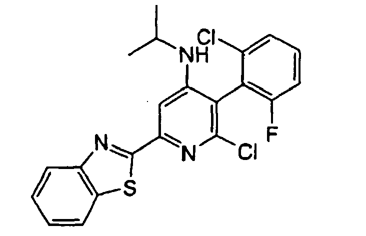 | Fp. 154 °C |
| I-53 | 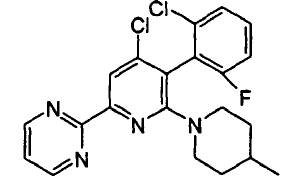 | Fp. 129 °C |
| I-54 | 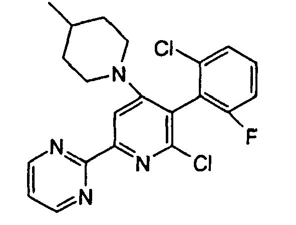 | Fp. 129 °C |
| I-55 | 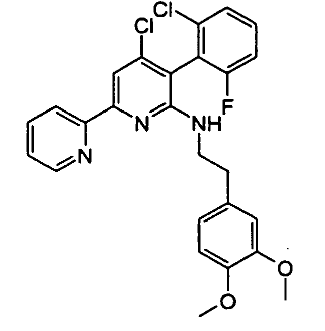 | ¹H-NMR (CDCl₃) δ = 2,90 (t); 3,77 (m); 3,86 (s); 3,88 (s); 4,13 (m); 6,70 (m); 7,10 (t); 7,35 (m); 7,80 (t); 7,90 (s); 8,47 (d); 8,73 (m). |
| I-56 | 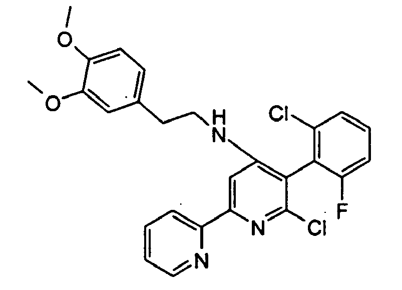 | Fp. 78 °C |
| I-57 | 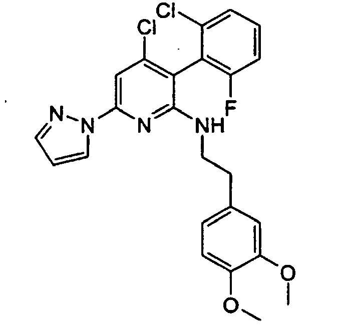 | Fp. 59 °C |
| I-58 | 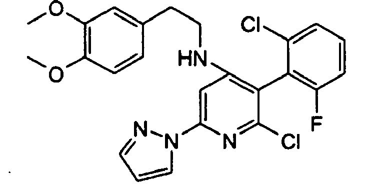 | Fp. 201 °C |

### Beispiele für die Wirkung gegen Schadpilze

Die fungizide Wirkung der Verbindungen der Formel I ließ sich durch die folgenden Versuche zeigen:

Die Wirkstoffe wurden getrennt als Stammlösung formuliert mit 0,25 Gew.-% Wirkstoff in Aceton oder DMSO. Dieser Lösung wurde 1 Gew.-% Emulgator Uniperol® EL (Netzmittel mit Emulgier- und Dispergierwirkung auf Basis ethoxylierter Alkylphenole) zugesetzt. Die Stammlösungen der Wirkstoffe wurden entsprechend der angegebenen Konzentration mit Wasser verdünnt.

### Anwendungsbeispiele

### Wirksamkeit gegen Mehltau an Gurkenblättem verursacht durch Sphaerotheca fuliginea bei protektiver Anwendung

Blätter von in Töpfen gewachsenen Gurkenkeimlingen wurden im Keimblattstadium mit wässriger Suspension in einer Wirkstoffkonzentration von 250 ppm bis zur Tropfnässe besprüht. 20 Stunden nach dem Antrocknen des Spritzbelages wurden die Pflanzen mit einer wässrigen Sporensuspension des Gurkenmehltaus *(Sphaerotheca fuliginea)* inokuliert. Anschließend wurden die Pflanzen im Gewächshaus bei Temperaturen zwischen 20 und 24° C und 60 bis 80 % relativer Luftfeuchtigkeit für 7 Tage kultiviert. Dann wurde das Ausmaß der Mehltauentwicklung visuell in %-Befall der Keimblattfläche ermittelt.

In diesem Versuch zeigten die mit den Verbindungen I-10, I-17, I-30, I-32, I-33, I-42 bis I-45, I-51, I-52, I-54 bis I-57 oder I-58 behandelten Pflanzen einen Befall von 15% und weniger während die unbehandelten Pflanzen zu 60% befallen waren.

### Beispiele zur Wirkung gegen tierische Schädlinge

Die Wirkung der Verbindungen der allgemeinen Formel l gegen tierische Schädlinge ließ sich durch folgende Versuche zeigen:
Die Wirkstoffe wurden
a) als 0,1 %-ige Lösung in Aceton oder
b) als 10 %-ige Emulsion in einem Gemisch aus 70 Gew.-% Cyclohexanon, 20 Gew.-% NekanilR LN (LutensolR AP6, Netzmittel mit Emulgier- und Dispergierwirkung auf der Basis ethoxylierter Alkylphenole) und 10 Gew.-% EmulphorR EL (EmulanR EL, Emulgator auf der Basis ethoxylierter Fettalkohole)
aufbereitet und entsprechend der gewünschten Konzentration mit Aceton im Fall von a) bzw. mit Wasser im Fall von b) verdünnt.
Nach Abschluß der Versuche wurde die jeweils niedrigste Konzentration ermittelt, bei der die Verbindungen im Vergleich zu unbehandelten Kontrollversuchen noch eine 80 - 100 %-ige Hemmung bzw. Mortalität hervorriefen (Wirkschwelle bzw. Minimalkonzentration).

## Patentansprüche

1. 2-Substituierte Pyridine der Formel I, 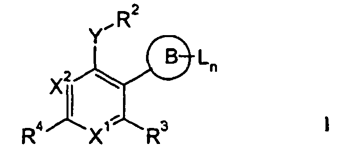 in der die Indices und die Substituenten die folgende Bedeutung haben:
X¹, X² jeweils einer der beiden Ringglieder bedeutet N, der andere C-H oder C-Halogen;
Y bedeutet eine Gruppe -CH-R¹-, -N-R¹-, -O- oder -S-;
R¹, R² bedeuten unabhängig voneinander C₁-C₈-Alkyl, C₂-C₈-Alkenyl, C₂-C₈-Alkinyl, C₃-C₆-Cycloalkyl, C₃-C₆-Halogencycloalkyl, wobei R¹ und R² ihrerseits partiell oder vollständig halogeniert sein oder eine bis vier Gruppen R^{v} tragen können:
R^{v} bedeutet Cyano, C₁-C₆-Alkyl, C₃-C₆-Cycloalkyl, C₄-C₆-Cycloalkenyl, Hydroxy, C₁-C₆-Alkoxy, C₂-C₈-Alkenyloxy, C₂-C₈-Alkinyloxy, C₃-C₆-Cycloalkyloxy, C₄-C₆-Cycloalkenyloxy, C₁-C₆-Alkylthio, -C(=O)-A, -C(=O)-O-A, -C(=O)-N(A')A, C(A')(=N-OA), N(A')A, N(A')-C(=O)-A, N(A")-C(=O)-N(A')A, S(=O)ₘ-A, S(=O)ₘ-O-A oder S(=O)ₘ-N(A')A oder Phenyl, wobei der Phenylteil ein bis drei Reste ausgewählt aus der Gruppe: Halogen, C₁-C₆-Alkyl, C₂-C₆-Alkenyl, C₂-C₆-Alkinyl, C₃-C₆-Cycloalkyl, C₁-C₆-Halogenalkyl, C₁-C₆-Alkoxy, Cyano, Nitro, -C(=O)-A, -C(=O)-O-A, -C(=O)-N(A')A, C(A')(=N-OA), N(A')A tragen kann;
R¹ kann zusätzlich Wasserstoff bedeuten;
R¹ und R² können auch zusammen mit dem Stickstoff- oder Kohlenstoffatom, an das sie gebunden sind, einen gesättigten oder ungesättigten fünf- oder sechsgliedrigen Ring bilden, der durch eine Ether -(-O-), Carbonyl-(C=O)-, Thio -(-S-), Sulfoxyl -(-S[=O]-) oder Sulfenyl -(-SO₂-) oder eine weitere Amino -(-N(R^{a})- Gruppe, wobei R^{a} Wasserstoff oder C₁-C₆-Alkyl bedeutet, unterbrochen sein und/oder einen oder mehrere Substituenten aus der Gruppe Halogen, C₁-C₆-Alky), C₁-C₆-Halogenalkyl und Oxy-C₁-C₃-alkylenoxy enthalten kann;
R³ bedeutet Halogen, Cyano, C₁-C₄-Alkyl, C₂-C₄-Alkenyl, C₂-C₄-Alkinyl, C₃-C₆-Cycloalkyl, C₁-C₄-Alkoxy, C₃-C₄-Alkenyloxy, C₃-C₄-Alkinyloxy, C₁-C₆-Alkylthio, Di-(C₁-C₆-alkyl)amino oder C₁-C₆-Alkylamino, wobei die Alkyl, Alkenyl und Alkinylreste von R³ durch Halogen, Cyano, Nitro, C₁-C₂-Alkoxy oder C₁-C₄-Alkoxycarbonyl substituiert sein können;
R⁴ bedeutet ein fünf- oder sechsgliedriger gesättigter, partiell ungesättigter oder aromatischer mono- oder bicyclischer Heterocyclus, enthaltend ein bis vier Heteroatome aus der Gruppe O, N oder S, der seinerseits partiell oder vollständig halogeniert sein oder eine bis vier Gruppen R^{u} tragen kann:
R^{u} bedeutet Cyano, C₁-C₈-Alkyl, C₂-C₈-Alkenyl, C₂-C₈-Alkinyl, C₁-C₆-Alkoxy, C₃-C₆-Cycloalkyl, C₂-C₈-Alkenyloxy, C₂-C₈-Alkinyloxy, C₄-C₆-Cycloalkenyl, C₃-C₆-Cycloalkyloxy, C₄-C₆-Cycloalkenyloxy, -C(=O)-A, -C(=O)-O-A, -C(=O)-N(A')A, C(A')(=N-OA), N(A')A, N(A')-C(=O)-A, N(A")-C(=O)-N(A')A, S(=O)ₘ-A, S(=O)ₘ-O-A oder S(=O)ₘ-N(A')A, wobei m, A, A', A" die vorgenannte Bedeutung haben;
weiterhin kann R⁴ bedeuten:
Cyano, C(=Z)OR^{a}, C(=Z)NR^{z}R^{b}, C(=Z)NR^{a}-NR^{z}R^{b}, C(=Z)R^{a}, CR^{a}R^{b}-OR^{z}, CR^{a}R^{b}-NR^{z}R^{c},
ON(=CR^{a}R^{b}), O-C(=Z)R^{a},
NR^{a}R^{b'}, NR^{a}(C(=Z)R^{b}), NR^{a}(C(=Z)OR^{b}), NR^{a}(C(=Z)-NR^{z}R^{b}), NR^{a}(N=CR^{c}R^{b}), NR^{a}-NR^{z}R^{b}, NR^{z}-OR^{a}, wobei
Z O, S, NR^{a}, NOR^{a} oder N-NR^{z}R^{c} bedeutet;
R^{a},R^{b},R^{c} unabhängig voneinander für Wasserstoff, C₁-C₆-Alkyl, C₂-C₆-Alkenyl, C₂-C₆-Alkinyl, C₃-C₆-Cycloalkyl oder C₄-C₆-Cycloalkenyl stehen;
R^{b'} bis auf Wasserstoff die gleichen Bedeutungen wie R^{b} hat;
R^{z} die gleichen Bedeutungen wie R^{a} hat und zusätzlich -CO-R^{a} bedeuten kann;
wobei die aliphatischen oder alicyclischen Gruppen der Restedefinitionen von R^{a},R^{b},R^{c} oder R^{z} ihrerseits partiell oder vollständig halogeniert sein oder eine bis vier Gruppen R^{w} tragen können:
R^{w} bedeutet Halogen, Cyano, C₁-C₈-Alkyl, C₂-C₁₀-Alkenyl, C₂-C₁₀-Alkinyl, C₁-C₆-Alkoxy, C₂-C₁₀-Alkenyloxy, C₂-C₁₀-Alkinyloxy, C₃-C₆-Cycloalkyl, C₃-C₆-Cycloalkenyl, C₃-C₆-Cycloalkoxy, C₃-C₆-Cycloalkenyloxy, und wobei zwei der Reste R^{a}, R^{b}, R^{c} oder R^{z} zusammen mit den Atomen an die sie gebunden sind einen fünf- bis sechsgliedrigen gesättigten, partiell ungesättigten oder aromatischen Heterocyclus enthaltend ein bis vier Heteroatome aus der Gruppe O, N oder S, bilden können;
Ⓑ steht für fünf- oder sechsgliedriges Hetaryl enthaltend 1 bis 3 Heteroatome ausgewählt aus der Gruppe O, N oder S oder Phenyl;
n ist eine ganze Zahl von 1 bis 5;
L bedeutet Halogen, Cyano, Cyanato (OCN), C₁-C₈-Alkyl, C₂-C₈-Alkenyl, C₂-C₈-Alkinyl, C₁-C₆-Alkoxy, C₂-C₈-Alkenyloxy, C₂-C₈-Alkinyloxy, C₃-C₆-Cycloalkyl, C₄-C₆-Cycloalkenyl, C₃-C₆-Cycloalkyloxy, C₄-C₆-Cycloalkenyloxy, Nitro, -C(=O)-A, -C(=O)-O-A, -C(=O)-N(A')A, -C(=S)-N(A')A, C(A')(=N-OA), N(A')A, N(A')-C(=O)-A, N(A")-C(=O)-N(A')A, S(=O)ₘ-A, S(=O)ₘ-O-A oder S(=O)ₘ-N(A')A,
m ist 0, 1 oder 2;
A, A', A" sind unabhängig voneinander Wasserstoff. C₁-C₆-Alkyl, C₂-C₆-Alkenyl, C₂-C₆-Alkinyl, C₃-C₈-Cycloalkyl, C₃-C₈-Cycloalkenyl, Phenyl, wobei die organischen Reste partiell oder vollständig halogeniert sein können oder durch Nitro, Cyanato, Cyano oder C₁-C₄-Alkoxy substituiert sein können; oder A und A' stehen zusammen mit den Atomen, an die sie gebunden sind, für einen fünf- bis sechsgliedrigen gesättigten, partiell ungesättigten oder aromatischen Heterocyclus, enthaltend ein bis vier Heteroatome aus der Gruppe O, N oder S;
wobei die aliphatischen Gruppen der Restedefinitionen von L ihrerseits partiell oder vollständig halogeniert sein oder eine bis vier Gruppen R^{L} tragen können:
R^{L} bedeutet Cyano, C₁-C₆-Alkoxy, C₃-C₆-Cycloalkyl, C₂-C₈-Alkenyloxy, C₂-C₈-Alkinyloxy, C₄-C₆-Cycloalkenyl, C₃-C₆-Cycloalkyloxy, C₄-C₆-Cycloalkenyloxy, -C(=O)-A, -C(=O)-O-A, -C(=O)-N(A')A, C(A')(=N-OA), N(A')A, N(A')-C(=O)-A, N(A")-C(=O)-N(A')A, S(=O)ₘ-A, S(=O)ₘ-O-A oder S(=O)ₘ-N(A')A.

2. 2-Substituierte Pyridine nach Anspruch 1,
worin B = Phenyl bedeutet und die der Formel l' entsprechen: 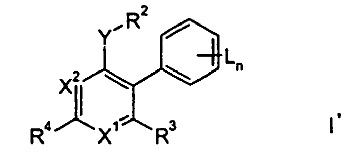 wobei
Y eine Gruppe -CH-R¹-, -N-R¹- oder -O- bedeutet;
R¹,R² unabhängig voneinander C₁-C₆-Alkyl, C₂-C₆-Alkenyl, C₂-C₆-Alkinyl, C₁-C₆-Halogenalkyl, C₂-C₆-Halogenalkenyl oder C₂-C₆-Halogenalkinyl bedeuten;
R¹ zusätzlich Wasserstoff bedeuten kann;
R¹ und R² auch zusammen mit dem Stickstoffatom, an das sie gebunden sind, einen gesättigten oder ungesättigten fünf- oder sechsgliedrigen Ring bilden können, der durch eine Ether -(-O-) oder eine weitere Amino-(-N(R^{a})-Gruppe, wobei R^{a} Wasserstoff oder C₁-C₆-Alkyl bedeutet, unterbrochen sein und/oder einen oder mehrere Substituenten aus der Gruppe Halogen, C₁-C₆-Alkyl, C₁-C₆-Halogenalkyl und Oxy-C₁-C₃-alkylenoxy enthalten kann;
R³ Halogen, Cyano, C₁-C₄-Alkyl, C₁-C₄-Alkoxy oder C₁-C₄-Haloalkyl bedeutet;
R⁴ Pyrrol, Pyrazol, Imidazol, 1,2,3-Triazol, 1,2,4-Triazol, Tetrazol, Oxazol, Isoxazol, 1,3,4-Oxadiazol, Furan, Thiophen, Thiazol, Isothiazol, Pyridin, Pyrimidin, Pyrazin, Pyridazin, 1,2,3-Triazin, 1,2,4-Triazin, 1-Pyridin(1,2,-dihydro)-2-on oder 1-Pyrrolidon bedeutet, wobei der Heterocyclus über C oder N an den Pyrimidinring gebunden sein kann und bis zu drei Substituenten R^{u} tragen kann,
R^{u} Halogen, Cyano, C₁-C₈-Alkyl, C₁-C₆-Alkoxy, -C(=O)-A, -C(=O)-O-A, -C(=O)-N(A')A, C(A')(=N-OA), N(A')A, N(A')-C(=O)-A,
oder
Cyano, C(=O)NR^{z}R^{b}, C(=NOR^{a})NR^{z}R^{b}, C(=NOR^{b})R^{a}, C(=N-NR^{z}R^{b})R^{a} oder CR^{a}R^{b}-NR^{z}R^{c}, ON(=CR^{a}R^{b}), NR^{a}(C(=O)R^{b}), NR^{a}(C(=O)OR^{b}), N-R^{a}(N=CR^{c}R^{b}) oder NR^{z}-OR^{a} bedeutet;
n eine ganze Zahl von 1 bis 3 ist, wobei mindestens ein Substituent L in ortho-Stellung am Phenylring sitzt;
L Halogen, Cyano, Methyl, Methoxy, -C(=O)-O-A, -C(=O)-N(A')A, -C(=S)-N(A')A, C(A')(=N-OA), N(A')A, N(A')-C(=O)-A bedeutet,
A,A' unabhängig voneinander Wasserstoff, C₁-C₆-Alkyl, C₂-C₆-Alkenyl, C₂-C₆-Alkinyl, Phenyl bedeuten, wobei die organischen Reste partiell oder vollständig halogeniert sein können oder durch C₁-C₄-Alkoxy substituiert sein können, oder A und A' zusammen mit den Atomen an die sie gebunden sind für einen fünf- bis sechsgliedrigen gesättigten Heterocyclus, enthaltend ein oder zwei Heteroatome aus der Gruppe O, N oder S stehen;
wobei die aliphatischen Gruppen der Restedefinitionen von L ihrerseits partiell oder vollständig halogeniert sein können.

3. 2-Substituierte Pyridine nach Anspruch 1, in der
R⁴ 1-Pyrazol, 1-[1,2,4]Triazol, 2-Pyridin, 2-Pyrimidin, 3-Pyridazin, 1-Pyridin(1,2,-dihydro)-2-on oder 1-Pyrrolidon bedeutet.

4. 2-Substituierte Pyridine nach Anspruch 1, in der
R⁴ C(=Z)OR^{a}, C(=Z)NR^{z}R^{b} oder C(=Z)R^{a} und
Z O, NR^{a} oder NOR^{a} bedeutet.

5. 2-Substituierte Pyridine nach Anspruch 1, wobei
Y eine Gruppe -CH-R¹- oder -N-R¹-, wobei R¹ Wasserstoff bedeutet, und
R² in α-Stellung verzweigtes C₃-C₈-Alkyl, C₃-C₈-Alkenyl, C₃-C₈-Alkinyl oder C₃-C₆-Halogenalkyl bedeuten.

6. 2-Substituierte Pyridine nach einem der Ansprüche 1 bis 4, worin B durch Lₙ substituiertes Phenyl bedeutet und dargestellt ist durch 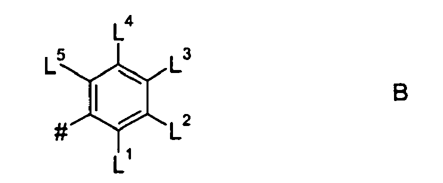 worin # die Verknüpfungsstelle mit dem Pyridin-Gerüst ist und
L¹ Fluor, Chlor, CH₃ oder CF₃;
L²,L⁴ unabhängig voneinander Wasserstoff, CH₃ oder Fluor;
L³ Wasserstoff, Fluor, Chlor, Brom, Nitro, Cyano, CH₃, SCH₃, OCH₃, SO₂CH₃, CO-NH₂, CO-NHCH₃, CO-NHC₂H₅, CO-N(CH₃)₂, CS-NH₂, CS-NHCH₃, CS-N(CH₃)₂, NH-C(=O)CH₃, N(CH₃)-C(=O)CH₃ oder COOCH₃ und
L⁵ Wasserstoff, Fluor, Chlor oder CH₃ bedeuten.

7. Verfahren zur Herstellung der Verbindungen I* und I**, 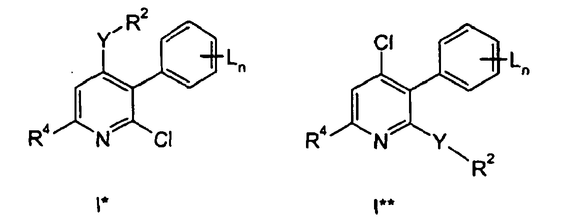 wobei die Substituenten Y, R², R⁴ und Lₙ die in Anspruch 2 angegebene Bedeutung haben, indem Phenylacetonitril II 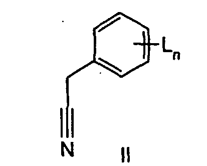 und Malonylchlorid zum Dihydroxypyridin III 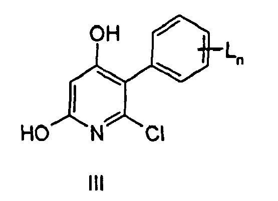 umgesetzt wird, welches mit Phosphoroxychlorid zum Trichlorpyridin IV 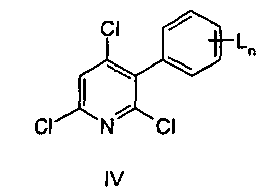 zur Reaktion gebracht wird und mit R⁴W, wobei W Wasserstoff oder einen metallorganischen Rest bedeutet, zum Zwischenprodukt V 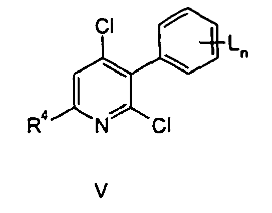 umgesetzt wird, welches schließlich mit R²YW', wobei W' Wasserstoff oder einen metallorganischen Rest bedeutet, zur Reaktion gebracht wird.

8. Pestizides Mittel, enthaltend einen festen oder flüssigen Trägerstoff und eine Verbindung der Formel I gemäß Anspruch 1.

9. Verfahren zur Bekämpfung von pflanzenpathogenen Schadpilzen, **dadurch gekennzeichnet, dass** man die Pilze oder die vor Pilzbefall zu schützenden Materialien, Pflanzen, den Boden oder Saatgüter mit einer wirksamen Menge einer Verbindung der Formel I gemäß Anspruch 1 behandelt.

10. Verfahren zur Bekämpfung von tierischen Schädlingen in der Landwirtschaft, **dadurch gekennzeichnet, dass** man die Schädlinge oder die von ihnen zu schützenden Materialien, Pflanzen, den Boden oder Saatgüter mit einer wirksamen Menge einer Verbindung der Formel I gemäß Anspruch 1 behandelt.

11. Verbindungen der Formel V, 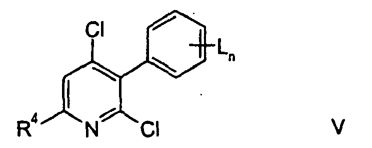 wobei Lₙ die in Anspruch 2 angegebene Bedeutung besitzt und R⁴ neben der in Anspruch 2 gegebenen Definition die Bedeutungen C₁-C₆-Alkylthio oder C₁-C₆-Alkylsulfenyl hat.

## Claims

1. A 2-substituted pyridine of the formula I 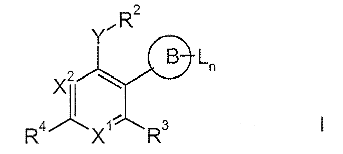 in which the indices and the substituents are as defined below:
X¹, X² in each case, one of the two ring members is N, the other is C-H or C-halogen;
Y is a group -CH-R¹- -N-R¹-, -O- or -S-;
R¹, R² independently of one another are C₁-C₈-alkyl, C₂-C₈-alkenyl, C₂-C₈-alkynyl, C₃-C₆-cycloalkyl, C₃-C₆-halocycloalkyl, where R¹ and R² for their part may be partially or fully halogenated or may carry one to four groups R^{v}:
R^{v} is cyano, C₁-C₆-alkyl, C₃-C₆-cycloalkyl, C₄-C₆-cycloalkenyl, hydroxyl, C₁-C₆-alkoxy, C₂-C₈-alkenyloxy, C₂-C₈-alkynyloxy, C₃-C₆-cycloalkyloxy, C₄-C₆-cycloalkenyloxy, C₁-C₆-alkylthio, -C(=O)-A, -C(=O)-O-A, -C(=O)-N(A')A, C(A')(=N-OA), N(A')A, N(A')-C(=O)-A, N(A")-C(=O)-N(A')A, S(=O)ₘ-A, S(=O)ₘ-O-A or S(=O)ₘ-N(A')A or phenyl, where the phenyl moiety may carry one to three radicals selected from the group consisting of: halogen, C₁-C₆-alkyl, C₂-C₆-alkenyl, C₂-C₆-alkynyl, C₃-C₆-cycloalkyl, C₁-C₆-haloalkyl, C₁-C₆-alkoxy, cyano, nitro, -C(=O)-A, -C(=O)-O-A, -C(=O)-N(A')A, C(A')(=N-OA), N(A')A;
R¹ may additionally be hydrogen;
R¹ and R² may also, together with the nitrogen or carbon atom to which they are attached, form a saturated or unsaturated five- or six-membered ring which may be interrupted by an ether (-O-), carbonyl (C[=O]-), thio (-S-), sulfoxyl (-S[=O]-) or sulfenyl (-SO₂-) group or by a further amino -(-N(R^{a})- group, where R^{a} is hydrogen or C₁-C₆-alkyl, and/or may comprise one or more substituents from the group consisting of halogen, C₁-C₆-alkyl, C₁-C₆-haloalkyl and oxy-C₁-C₃-alkyleneoxy;
R³ is halogen, cyano, C₁-C₄-alkyl, C₂-C₄-alkenyl, C₂-C₄-alkynyl, C₃-C₆-cycloalkyl, C₁-C₄-alkoxy, C₃-C₄-alkenyloxy, C₃-C₄-alkynyloxy, C₁-C₆-alkylthio, di-(C₁-C₆-alkyl)amino or C₁-C₆-alkylamino, where the alkyl, alkenyl and alkynyl radicals of R³ may be substituted by halogen, cyano, nitro, C₁-C₂-alkoxy or C₁-C₄-alkoxycarbonyl ;
R⁴ is a five- or six-membered saturated, partially unsaturated or aromatic mono- or bicyclic heterocycle which comprises one to four heteroatoms from the group consisting of O, N and S which for its part may be partially or fully halogenated or may carry one to four groups R^{u}:
R^{u} is cyano, C₁-C₈-alkyl, C₂-C₈-alkenyl, C₂-C₈-alkynyl, C₁-C₆-alkoxy, C₃-C₆-cycloalkyl, C₂-C₈-alkenyloxy, C₂-C₈-alkynyloxy, C₄-C₆-cycloalkenyl, C₃-C₆-cycloalkyloxy, C₄-C₆-cycloalkenyloxy, -C(=O)-A, -C(=O)-O-A, -C(=O)-N(A')A, C(A')(=N-OA), N(A')A, N(A')-C(=O)-A, N(A")-C(=O)-N(A')A, S(=O)ₘ-A, S(=O)ₘ-O-A or S(=O)ₘ-N(A')A, where m, A, A', A" are as defined above;
R⁴ may furthermore be:
cyano, C(=Z)OR^{a}, C(=Z)NR^{z}R^{b}, C(=Z)NR^{a}-NR^{z}R^{b}, C(=Z)R^{a}, CR^{a}R^{b}OR^{z}, CR^{a}R^{b}-NR^{z}R^{c},
ON(=CR^{a}R^{b}), O-C(=Z)R^{a},
NR^{a}Rb^{b'}, NR^{a}(C(=Z)R^{b}), NR^{a}(C(=Z)OR^{b}), NR^{a}(C(=Z)-NR^{z}R^{b}), NR^{a}(N=CR^{c}R^{b}), NR^{a}-NR^{z}R^{b}, NR^{z}-OR, where
Z is O, S, NR^{a}, NOR^{a} or N-NR^{z}R^{c};
R^{a},R^{b},R^{c} independently of one another are hydrogen, C₁-C₆-alkyl, C₂-C₆-alkenyl, C₂-C₆-alkynyl, C₃-C₆-cycloalkyl or C₄-C₆-cycloalkenyl;
R^{b'} has the same meanings as R^{b}, except for hydrogen;
R^{z} has the same meanings as R^{a} and may additionally be -CO-R^{a};
where the aliphatic or alicyclic groups of the radical definitions of R^{a},R^{b},R^{c} or R^{z} for their part may be partially or fully halogenated or may carry one to four groups R^{w}:
R^{w} is halogen, cyano, C₁-C₈-alkyl, C₂-C₁₀-alkenyl, C₂-C₁₀-alkynyl, C₁-C₆-alkoxy, C₂-C₁₀-alkenyloxy, C₂-C₁₀-alkynyloxy, C₃-C₆-cycloalkyl, C₃-C₆-cycloalkenyl, C₃-C₆-cycloalkoxy, C₃-C₆-cycloalkenyloxy, and where two of the radicals R^{a}, R^{b}, R^{c} or R^{z} together with the atoms, to which they are attached, may form a five- or six-membered saturated, partially unsaturated or aromatic heterocycle which comprises one to four heteroatoms from the group consisting of O, N and S;
Ⓑ is a five- or six-membered hetaryl which comprises 1 to 3 heteroatoms selected from the group consisting of O, N and S or is phenyl;
n is an integer from 1 to 5;
L is halogen, cyano, cyanato (OCN), C₁-C₈-alkyl, C₂-C₈-alkenyl, C₂-C₈-alkynyl, C₁-C₆-alkoxy, C₂-C₈-alkenyloxy, C₂-C₈-alkynyloxy, C₃-C₆-cycloalkyl, C₄-C₆-cycloalkenyl, C₃-C₆-cycloalkyloxy, C₄-C₆-cycloalkenyloxy, nitro, -C(=O)-A, -C(=O)-O-A, -C(=O)-N(A')A, -C(=S)-N(A')A, C(A')(=N-OA), N(A')A, N(A')-C(=O)-A, N(A")-C(=O)-N(A')A, S(=O)ₘ-A, S(=O)ₘ-O-A or S(=O)ₘ-N(A')A,
m is 0, 1 or 2;
A, A', A" independently of one another are hydrogen, C₁-C₆-alkyl, C₂-C₆-alkenyl, C₂-C₆-alkynyl, C₃-C₈-cycloalkyl, C₃-C₈-cycloalkenyl, phenyl, where the organic radicals may be partially or fully halogenated or may be substituted by nitro, cyanato, cyano or C₁-C₄-alkoxy; or A and A' together with the atoms to which they are attached are a five- or six-membered saturated, partially unsaturated or aromatic heterocycle which comprises one to four heteroatoms from the group consisting of O, N and S;
where the aliphatic groups of the radical definitions of L for their part may be partially or fully halogenated or may carry one to four groups R^{L}:
R^{L} is cyano, C₁-C₆-alkoxy, C₃-C₆-cycloalkyl, C₂-C₈-alkenyloxy, C₂-C₈-alkynyloxy, C₄-C₆-cycloalkenyl, C₃-C₆-cycloalkyloxy, C₄-C₆-cycloalkenyloxy, -C(=O)-A, -C(=O)-O-A, -C(=O)-N(A')A, C(A')(=N-OA), N(A')A, N(A')-C(=O)-A, N(A")-C(=O)-N(A')A, S(=O)ₘ-A, S(=O)ₘ-O-A or S(=O)ₘ-N(A')A.

2. The 2-substituted pyridine according to claim 1,
in which B = phenyl and which corresponds to the formula I': 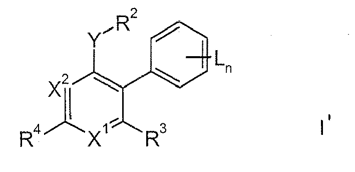 where
Y is a group -CH-R¹-, -N-R¹- or -O-;
R¹,R² independently of one another are C₁-C₆-alkyl, C₂-C₆-alkenyl, C₂-C₆-alkynyl, C₁-C₆-haloalkyl, C₂-C₆-haloalkenyl or C₂-C₆-haloalkynyl;
R¹ may additionally be hydrogen;
R¹ and R² may also, together with the nitrogen atom to which they are attached, form a saturated or unsaturated five- or six-membered ring which may be interrupted by an ether (-O-) or by a further amino -(-N(R^{a}))- group, where R^{a} is hydrogen or C₁-C₆-alkyl, and/or may comprise one or more substituents from the group consisting of halogen, C₁-C₆-alkyl, C₁-C₆-haloalkyl and oxy-C₁-C₃-alkyleneoxy;
R³ is halogen, cyano, C₁-C₄-alkyl, C₁-C₄-alkoxy or C₁-C₄-haloalkyl;
R⁴ is pyrrole, pyrazole, imidazole, 1,2,3-triazole, 1,2,4-triazole, tetrazole, oxazole, isoxazole, 1,3,4-oxadiazole, furan, thiophene, thiazole, isothiazole, pyridine, pyrimidine, pyrazine, pyridazine, 1,2,3-triazine, 1,2,4-triazine, 1-pyridin(1,2,-dihydro)-2-one or 1-pyrrolidone, where the heterocycle may be attached via C or N to the pyrimidine ring and may carry up to three substituents R^{u}.
R^{u} is halogen, cyano, C₁-C₈-alkyl, C₁-C₆-alkoxy, -C(=O)-A, -C(=O)-O-A, -C(=O)-N(A')A, C(A')(=N-OA), N(A')A, N(A')-C(=O)-A,
or
cyano, C(=O)NR^{z}R^{b}, C(=NOR^{a})NR^{z}R^{b}, C(=NOR^{b})R^{a}, C(=N-NR^{z}R^{b})R^{a} or CR^{a}R^{b}-NR^{z}R^{c}, ON(=CR^{a}R^{b}), NR^{a}(C(=O)R^{b}), NR^{a}(C(=O)OR^{b}), NR^{a}(N=CR^{c}R^{b}) or NR^{z}-OR^{a}.
n is an integer from 1 to 3 where at least one substituent L is located in the ortho-position on the phenyl ring;
L is halogen, cyano, methyl, methoxy, -C(=O)-O-A, -C(=O)-N(A')A, -C(=S)-N(A')A, C(A')(=N-OA), N(A')A, N(A')-C(=O)-A,
A,A' independently of one another are hydrogen, C₁-C₆-alkyl, C₂-C₆-alkenyl, C₂-C₆-alkynyl, phenyl, where the organic radicals may be partially or fully halogenated or may be substituted by C₁-C₄-alkoxy; or A and A' together with the atoms to which they are attached are a five- or six-membered saturated heterocycle which comprises one or two heteroatoms from the group consisting of O, N and S;
where the aliphatic groups of the radical definitions of L for their part may be partially or fully halogenated.

3. The 2-substituted pyridine according to claim 1 in which
R⁴ is 1-pyrazole, 1-[1,2,4]triazole, 2-pyridine, 2-pyrimidine, 3-pyridazine, 1-pyridin(1,2,-dihydro)-2-one or 1-pyrrolidone.

4. The 2-substituted pyridine according to claim 1 in which
R⁴ is C(=Z)OR^{a}, C(=Z)NR^{z}R^{b} or C(=Z)R^{a} and
Z is O, NR^{a} or NOR^{a}.

5. The 2-substituted pyridine according to claim 1 in which
Y is a group -CH-R¹ or -N-R¹-, where R¹ is hydrogen and
R² is C₃-C₈-alkyl, C₃-C₈-alkenyl, C₃-C₈-alkynyl or C₃-C₆-haloalkyl branched in the α-position.

6. The 2-substituted pyridine according to any of claims 1 to 4 in which B is Lₙ-substituted phenyl and is represented by 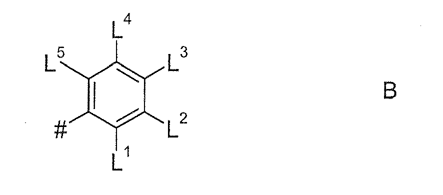 where # is the point of attachment to the pyridine skeleton and
L¹ is fluorine, chlorine, CH₃ or CF₃;
L², L⁴ independently of one another are hydrogen, CH₃ or fluorine;
L³ is hydrogen, fluorine, chlorine, bromine, nitro, cyano, CH₃, SCH₃, OCH₃, SO₂CH₃, CO-NH₂, CO-NHCH₃, CO-NHC₂H₅, CO-N(CH₃)₂, CS-NH₂, CS-NHCH₃, CS-N(CH₃)₂, NH-C(=O)CH₃, N(CH₃)-C(=O)CH₃ or COOCH₃ and
L⁵ is hydrogen, fluorine, chlorine or CH₃.

7. A process for preparing the compounds I* and I**, 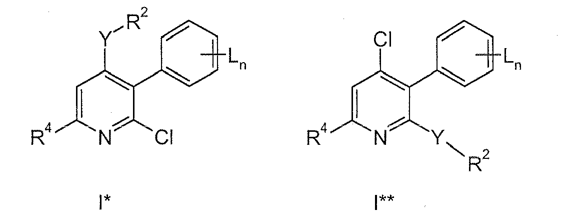 where the substituents Y, R², R⁴ and Lₙ are as defined in claim 2, by reacting phenylacetonitrile II 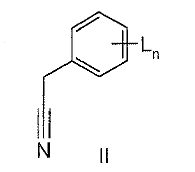 and malonyl chloride to give the dihydroxypyridine III 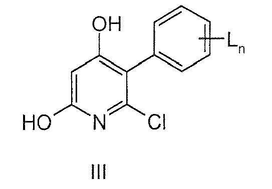 which is reacted with phosphorus oxychloride to give the trichloropyridine IV 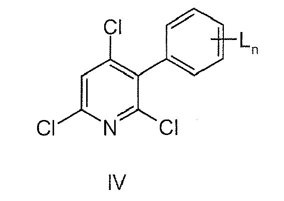 and reacted with R⁴W, where W is hydrogen or an organometallic radical, to give the intermediate V 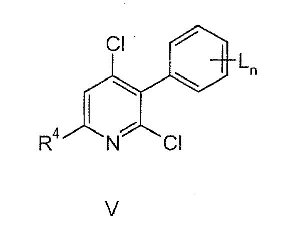 which is finally reacted with R²YW', where W' is hydrogen or an organometallic radical.

8. A pesticidal composition which comprises a solid or liquid carrier and a compound of the formula I according to claim 1.

9. A method for controlling phytopathogenic harmful fungi which comprises treating the fungi or the materials, plants, the soil or the seeds to be protected against fungal attack with an effective amount of a compound of the formula I according to claim 1.

10. A method for controlling animal pests in agriculture which comprises treating the pests or the materials, plants, the soil or the seeds to be protected against them with an effective amount of a compound of the formula I according to claim 1.

11. A compound of the formula V 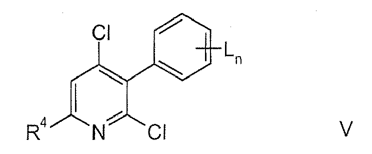 where Lₙ is as defined in claim 2 and R⁴ has, in addition to the definition given in claim 2, the meanings C₁-C₆-alkylthio or C₁-C₆-alkylsulfenyl.

## Revendications

1. Pyridines 2-substituées de formule I, 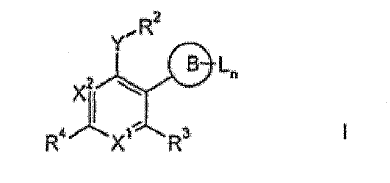 dans laquelle les indices et les substituants ont la signification suivante :
l'un des deux éléments de cycle X¹, X² désigne N, l'autre C-H ou C-halogène ;
Y désigne un groupe -CH-R¹-, -N-R¹-, -O- ou -S- ;
R¹, R² désignent, indépendamment l'un de l'autre, un radical C₁-C₈-alkyle, C₂-C₈-alcényle, C₂-C₈-alcinyle, C₃-C₆-cycloalkyle, C₃-C₆-halogénocycloalkyle, R¹ et R² pouvant, pour leur part, être halogénés totalement ou partiellement ou porter un à quatre groupes R^{v} :
R^{v} désigne un radical cyano, C₁-C₆-alkyle, C₃-C₆-cycloalkyle, C₄-C₆-cycloalcényle, hydroxy, C₁-C₆-alcoxy, C₂-C₈-alcényloxy, C₂-C₈-alcinyloxy, C₃-C₆-cycloalkyloxy, C₄-C₆-cycloalcényloxy, C₁-C₆-alkylthio, -C(=O)-A, -C(=O)-O-A, -C(=O)-N(A')A, C(A') (=N-OA), N(A')A, N(A')-C(=O)-A, N(A")-C(=O)-N(A')A, S(=O)ₘ-A, S(=O)ₘ-O-A ou S(=O)ₘ-N(A')A ou un radical phényle dans lequel la partie phényle contient un à trois radicaux sélectionnés dans le groupe : halogène, C₁-C₆-alkyle, C₂-C₈-alcényle, C₂-C₆-alcinyle, C₃-C₆-cycloalkyle, C₁-C₆-halogénoalkyle, C₁-C₆-alcoxy, cyano, nitro, -C(=O)-A, -C(=O)-O-A, -C(=O)-N(A')A, C(A')(=N-OA), N(A')A ;
R¹ peut par ailleurs désigner l'hydrogène ;
R¹ et R² peuvent aussi former avec l'atome d'azote ou de carbone auquel ils sont liés un cycle pentavalent- ou hexavalent saturé ou insaturé, qui peut être interrompu par un groupe éther-(-O-), carbonyl-(C=O)-, thio-(-S-), sulfoxyl-(-S[=O]-) ou sulfényle -(-SO₂-) ou un autre amino-(-N(R^{a})-, R^{a} désignant l'hydrogène ou un radical C₁-C₆-alkyle et/ou peut contenir un ou plusieurs substituants du groupe halogène, C₁-C₆-alkyle, C₁-C₆-halogénoalkyle et oxy-C₂-C₃-alkylénoxy ;
R³ désigne un halogène, un groupe cyano, C₁-C₄-alkyle, C₂-C₄-alcényle, C₂-C₄-alcynyle, C₃-C₆-cycloalkyle, C₁-C₄-alcoxy, C₃-C₄-alcényloxy, C₃-C₄-alcynyloxy, C₁-C₆-alkylthio, di-(C₁-C₆-alkyl, amino ou C₁-C₆-alkylamino, les radicaux alkyle, alcényle et alcinyle de R³ pouvant être substitués par un halogène, un radical cyano, nitro, C₁-C₂-alcoxy ou C₁-C₄-alcoxycarbonyle ;
R⁴ désigne un hétérocycle monocyclique ou bicyclique, saturé, partiellement insaturé ou aromatique pentavalent ou hexavalent, contenant un à quatre hétéroatomes du groupe O, N ou S qui, pour sa part, peut être partiellement ou complètement halogéné ou peut porter un à quatre groupes R^{u} :
R^{u} désigne un radical cyano, C₁-C₈-alkyle, C₂-C₈-alcényle, C₂-C₈-alcinyle, C₁-C₆-alcoxy, C₃-C₆-cycloalkyle, C₂-C₈-alcényloxy, C₂-C₈-alcinyloxy, C₄-C₆-cycloalcényle, C₃-C₆-cycloalkyloxy, C₄-C₆-cycloalcényloxy, -C(=O)-A, -C(=O)-O-A, -C(=O)-N(A')A, C(A')(=N-OA), N(A')A, N(A')-C(=O)-A, N(A")-C(=O)-N(A')A, S(=O)ₘ-A, S(=O)ₘ-O-A ou S(=O)ₘ-N(A')A, m, A, A', A" ayant la signification précitée ;
Par ailleurs, R⁴ peut désigner :
un groupe cyano, C(=Z)OR^{a}, C(=Z)NR^{z}R^{b}, C(=Z)NR^{a}-NR^{z}R^{b}, C(=Z)R^{a}, CR^{a}R^{b}-OR^{z}, CR^{a}R^{b}-NR^{z}R^{c}, ON(=CR^{a}R^{b}), OC(=Z,R^{a}, NR^{a}R^{b'}, NR^{a}(C(=Z)R^{b}), NR^{a}(C(=Z)OR^{b}), NR^{a}(C(=Z)-NR^{z}R^{b}), NR^{a}(N=CR^{c}R^{b}), NR^{a}-NR^{z}R^{b}, NR^{z}-OR^{a}, dans lesquels
Z désigne O, S, NR^{a}, NOR^{a} ou N-NR^{z}R^{c} ;
R^{a}, R^{b}, R^{c} désignent, indépendamment les uns des autres, l'hydrogène, un groupe C₁-C₆-alkyle, C₂-C₆-alcényle, C₂-C₆-alcynyle, C₃-C₆-cycloalkyle ou C₄-C₆-cycloalcényle,
R^{b'} a les mêmes significations que R^{b}, hormis l'hydrogène ;
R^{z} a les mêmes significations que R^{a} et peut par ailleurs désigner -CO-R^{a} ;
les groupes aliphatiques ou alicycliques des définitions des radicaux de R^{a}, R^{b}, R^{c} ou R^{z} pouvant, pour leur part, être halogénés partiellement ou complètement et porter un à quatre groupes R^{w} :
R^{w} désigne un radical halogène, cyano, C₁-C₈-alkyle, C₂-C₁₀-alcényle, C₂-C₁₀-alcinyle, C₁-C₆-alcoxy, C₂-C₁₀-alcényloxy, C₂-C₁₀-alcinyloxy, C₃-C₆-cycloalkyle, C₃-C₆-cycloalcényle, C₃-C₆-cycloalcoxy, C₃-C₆-cycloalcényloxy, et deux des radicaux R^{a}, R^{b}, R^{c} ou R^{z} pouvant former avec les atomes auxquels ils sont liés un hétérocycle saturé, partiellement insaturé ou aromatique, pentavalent à hexavalent contenant un à quatre hétéroatomes du groupe O, N ou S ;
Ⓑ désigne un hétaryle pentavalent ou hexavalent contenant 1 à 3 hétéroatomes sélectionnés dans le groupe O, N ou S ou phényle ;
n désigne un nombre entier de 1 à 5 ;
L désigne un halogène, un radical cyano, cyanato (OCN), C₁-C₈-alkyle, C₂-C₈-alcényle, C₂-C₈-alcinyle, C₁-C₆-alcoxy, C₂-C₈-alcényloxy, C₂-C₈-alcinyloxy, C₃-C₆-cycloalkyle, C₄-C₆-cycloalcényle, C₃-C₆-cycloalkyloxy, C₄-C₆-cycloalcényloxy, nitro, -C(=O)-A, -C(=O)-O-A, -C(=O)-N(A')A, -C(=S)-N(A')A, C(A')(=N-OA), N(A')A, N(A')-C(=O)-A, N(A")-C(=O)-N(A')A, S(=O)ₘ-A, S(=O)ₘ-O-A ou S(=O)ₘ-N(A')A ;
m désigne 0, 1 ou 2 ;
A, A', A" désignent, indépendamment les uns des autres, l'hydrogène, un radical C₁-C₆-alkyle, C₂-C₆-alcényle, C₂-C₆-alcynyle, C₃-C₈-cycloalkyle, C₃-C₈-cycloalcényle, phényle, les radicaux organiques pouvant être partiellement ou totalement halogénés ou substitués par un radical nitro, cyanato, cyano ou C₁-C₄-alcoxy ; ou A et A' désignent avec les atomes auxquels il sont liés un hétérocycle saturé, partiellement insaturé ou aromatique, pentavalent ou hexavalent contenant un à quatre hétéroatomes du groupe O, N ou S ;
les groupes aliphatiques des définitions des radicaux de L pouvant pour leur part être partiellement ou totalement halogénés ou porter un à quatre groupes R^{L} :
R^{L} désigne un radical cyano, C₁-C₆-alcoxy, C₃-C₆-cycloalkyle, C₂-C₈-alcényloxy, C₂-C₈-alcinyloxy, C₄-C₆-cycloalcényle, C₃-C₆-cycloalkyloxy, C₄-C₆-cycloalcényloxy, -C(=O)-A, -C(=O)-O-A, -C(=O)-N(A')A, C(A')(=N-OA), N(A')A, N(A')-C(=O)-A, N(A")-C(=O)-N(A')A, S(=O)ₘ-A, S(=O)ₘ-O-A ou S(=O)ₘ-N(A')A.

2. Pyridines 2-substituées selon la revendication 1, dans laquelle B = phényle et qui correspondent à la formule I', 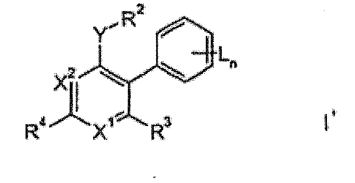 dans laquelle :
Y désigne un groupe -CH-R¹-, -N-R¹- ou -O- ;
R¹, R² désignent, indépendamment l'un de l'autre, un radical C₁₋C₆-alkyle, C₂-C₆-alcényle, C₂-C₆-alcinyle, C₁-C₆-halogénoalkyle, C₂-C₆-halogénoalcényle ou C₂-C₆-halogénoalcynyle ;
R¹ peut par ailleurs désigner l'hydrogène ;
R¹ et R² peuvent aussi former avec l'atome d'azote auquel ils sont liés un cycle pentavalent ou hexavalent saturé ou insaturé, qui peut être interrompu par un groupe éther-(-O-) ou un autre amino -(-N(R^{a})-, R^{a} désignant l'hydrogène ou un radical C₁-C₆-alkyle et/ou peut contenir un ou plusieurs substituants du groupe halogène, C₁-C₆-alkyle, C₁-C₆-halogénoalkyle et oxy-C₁-C₃-alkylénoxy ;
R³ désigne un halogène, un groupe cyano, C₁-C₄-alkyle, C₁-C₄-alcoxy ou C₁-C₄-halogénoalkyle ;
R⁴ désigne un groupe pyrrole, pyrazole, imidazole, 1,2,3-triazole, 1,2,4-triazole, tétrazole, oxazole, isoxazole, 1,3,4-oxadiazole, furane, thiophène, thiazole, isothiazole, pyridine, pyrimidine, pyrazine, pyridazine, 1,2,3-triazine, 1,2,4-triazine, 1-pyridin(1,2-dihydro)-2-one ou 1-pyrrolidone, l'hétérocycle pouvant être lié par l'intermédiaire de C ou N au cycle pyrimidine et pouvant porter jusqu'à trois substituants R^{u} ;
R^{u} désigne un halogène, un radical cyano, C₁-C₈-alkyle, C₁-C₆-alcoxy, -C(=O)-A, -C(=O)-O-A, -C(=O)-N(A')A, C(A') (=N-OA), N(A')A, N(A')-C(=O)-A,
ou
un groupe cyano, C(=O)NR^{z}R^{b}, C(=NOR^{a})NR^{z}R^{b}, C(=NOR^{b})R^{a}, C(=N-NR^{z}R^{b})R^{a} ou CR^{a}R^{b}-NR^{z}R^{c}. ON (=CR^{a}R^{b}), NR^{a}(C(=O)R^{b}), NR^{a}(C(=O)OR^{b}), N-R^{a}(N=CR^{c}R^{b}) ou NR^{z}-OR^{a}, dans lesquels
n désigne un nombre entier de 1 à 3, au moins un substituant L se trouvant en position ortho sur le cycle phényle ;
L désigne un halogène, un radical cyano, méthyle, méthoxy, -C(=O)-O-A, -C(=O)-N(A')A, -C(=S)-N(A')A, C(A')(=N-OA), N(A')A, N(A')-C(=O)-A;
A, A' désignent, indépendamment l'un de l'autre, l'hydrogène, un radical C₁-C₆-alkyle, C₂-C₆-alcényle, C₂-C₆-alcynyle, phényle, les radicaux organiques pouvant être partiellement ou totalement halogénés ou substitués par un radical C₁-C₄-alcoxy ; ou A et A' désignent avec les atomes auxquels ils sont liés un hétérocycle saturé, pentavalent ou hexavalent contenant un ou deux hétéroatomes du groupe O, N ou S ;
les groupes aliphatiques des définitions des radicaux de L pouvant pour leur part être partiellement ou totalement halogénés.

3. Pyridines 2-substituées selon la revendication 1, dans laquelle
R⁴ désigne un radical 1-pyrazole, 1-[1,2,4]triazole, 2-pyridine, 2-pyrimidine, 3-pyridazine, 1-pyridin(1,2-dihydro)-2-one ou 1-pyrrolidone.

4. Pyridines 2-substituées selon la revendication 1, dans laquelle
R⁴ désigne C(=Z)OR^{a}, C(=Z)NR^{z}R^{b} ou C(=Z)R^{a} et
Z désigne O, NR^{a} ou NOR^{a}.

5. Pyridines 2-substituées selon la revendication 1, dans laquelle
Y désigne un groupe -CH-R¹- ou -N-R¹-, R¹ désignant l'hydrogène et
R² désigne un radical C₃-C₈-alkyle, C₃-C₈-alcényle, C₃-C₈-alcinyle ou C₃-C₆-halogénoalkyle ramifié en position α.

6. Pyridines 2-substituées selon l'une des revendications 1 à 4, dans laquelle B désigne un radical phényle substitué par Lₙ et est représenté par 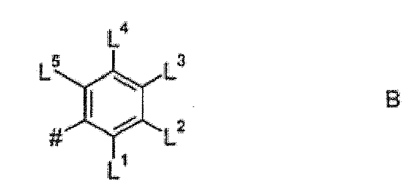 dans laquelle # désigne le site de liaison avec la structure pyridine et
L¹ désigne le fluor, le chlore, un radical CH₃ ou CF₃ ;
L², L⁴ désignent indépendamment l'un de l'autre l'hydrogène, CH₃ ou le fluor ;
L³ désigne l' hydrogène, le fluor, le chlore, le brome, un radical nitro, cyano, CH₃, SCH₃, OCH₃, SO₂CH₃, CO-NH₂, CO-NHCH₃, CO-NHC₂H₅, CO-N(CH₃)₂, CS-NH₂, CS-NHCH₃, CS-N(CH₃)₂, NH-C(=O)CH₃, N(CH₃)-C(=O)CH₃ ou COOCH₃ et
L⁵ désigne l'hydrogène, le fluor, le chlore ou CH₃.

7. Procédé de préparation de composés I* et I** 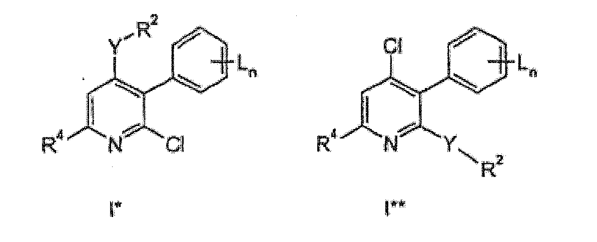 dans lesquels les substituants Y, R², R⁴ et Lₙ ont la signification mentionnée dans la revendication 2, en mettant réaction du phénylacétonitrile II 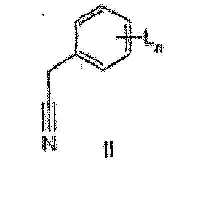 et du chlorure de malonyle pour obtenir de la dihydroxypyridine III 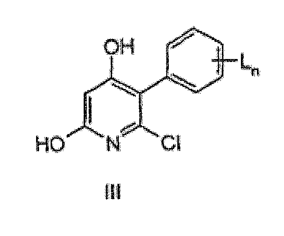 qui est mise en réaction avec de l'oxychlorure de phosphore pour obtenir de la trichloropyridine IV 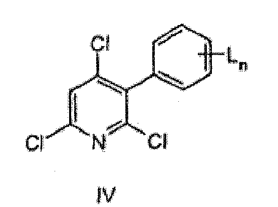 et est mis en réaction avec R⁴W, dans laquelle W désigne l'hydrogène ou un radical organométallique pour obtenir le produit intermédiaire V 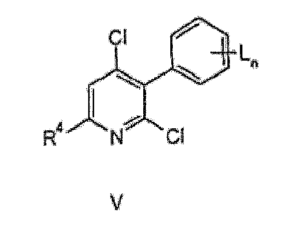 qui est finalement mis en réaction avec R²YW', W' désignant l'hydrogène ou un radical organométallique.

8. Pesticide contenant un excipient solide ou liquide et un composé de formule I selon la revendication 1.

9. Procédé de lutte contre les champignons nuisibles phytopathogènes, **caractérisé en ce qu'**on traite les champignons ou les matières, plantes, sols ou semences à protéger d'une infestation par ces champignons avec une quantité efficace d'un composé de formule I suivant la revendication 1.

10. Procédé de lutte contre les animaux nuisibles dans l'agriculture, **caractérisé en ce qu'**on traite les animaux nuisibles ou les matières, plantes, sols ou semences à protéger d'une infestation par ceux-ci avec une quantité efficace d'un composé de formule I suivant la revendication 1.

11. Composés de formule V 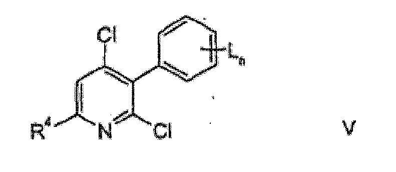 dans laquelle Lₙ possède la signification mentionnée dans la revendication 2 et R⁴ peut désigner, hors la définition mentionnée dans la revendication 2, un radical C₁-C₆-alkylthio ou C₁-C₆-alkylsulfényle.
